(19) Europäisches Patentamt
European Patent Office
Office européen des brevets

(11) EP 4 603 485 A1

(12) **EUROPEAN PATENT APPLICATION**
published in accordance with Art. 153(4) EPC

(43) Date of publication:
**20.08.2025 Bulletin 2025/34**

(21) Application number: **23877356.8**

(22) Date of filing: **13.10.2023**

(51) International Patent Classification (IPC):
***C07D 401/04*** (2006.01) ***A01M 1/20*** (2006.01)
***A01N 43/42*** (2006.01) ***A01N 43/54*** (2006.01)
***A01N 43/653*** (2006.01) ***A01N 43/90*** (2006.01)
***A01N 47/02*** (2006.01) ***A01N 53/12*** (2006.01)
***A01N 53/14*** (2006.01) ***A01P 7/02*** (2006.01)
***A01P 7/04*** (2006.01) ***A61K 31/4725*** (2006.01)
***A61P 33/14*** (2006.01)

(52) Cooperative Patent Classification (CPC):
**A01M 1/20; A01N 43/42; A01N 43/54; A01N 43/653; A01N 43/90; A01N 47/02; A01N 53/00; A01P 7/02; A01P 7/04; A61K 31/4725; A61P 33/14; C07D 401/04**

(86) International application number:
**PCT/JP2023/037149**

(87) International publication number:
**WO 2024/080355 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.10.2022 JP 2022165233**

(71) Applicant: **Sumitomo Chemical Company, Limited**
**Tokyo 103-6020 (JP)**

(72) Inventors:
- **NISHIBU, Saki**
  **Takarazuka-shi, Hyogo 665-8555 (JP)**
- **SATO, Natsumi**
  **Chuo-ku, Osaka-shi, Osaka 5418550 (JP)**
- **MINEGISHI, Hidemitsu**
  **Takarazuka-shi, Hyogo 665-8555 (JP)**
- **SATO YANAGIHARA, Saki**
  **Osaka-shi, Osaka 541-8550 (JP)**

(74) Representative: **Vossius & Partner Patentanwälte Rechtsanwälte mbB**
**Siebertstrasse 3**
**81675 München (DE)**

# (54) SULFONAMIDE COMPOSITION AND HARMFUL-ARTHROPOD CONTROL COMPOSITION CONTAINING SAME

(57) The present invention provides a compound represented by formula (I) or an N oxide thereof that exhibits an excellent control effect on harmful arthropod , in which, in the formula, Q represented by the following formula, in which, in the formula, # represents a binding site to the sulfur atom, and • represents a binding site to Het and represents a group represented by formula Q1 or the like; $R^{2a}$ and $R^{2b}$ are identical to or different from each other, and each represents a C1-C6 alkyl group optionally substituted with one or more halogen atoms or the like; $R^{3a}$, $R^{3b}$, and $R^{3d}$ are identical to or different from each other, and each represents a hydrogen atom or the like; Het represents a group represented by formula Het 5 or the like; $A^2$ represents $NR^{5a}$ or $CR^{4a}R^{4d}$; $A^3$ represents $CR^{4b}R^{4e}$; $W^1$ represents an oxygen atom or a sulfur atom; when Het is a group represented by formula Het 5, $B^1$, $B^2$, and $B^3$ represent, as a combination, a combination in which $B^1$ is $CR^{6e}$, $B^2$ is a nitrogen atom or $CR^{6b}$, and $B^3$ is a nitrogen atom or $CR^{6d}$ or the like; $R^{4a}$, $R^{4b}$, $R^{6a}$, $R^{6b}$, and $R^{6c}$ are identical to or different from each other, and each represents a hydrogen atom or the like; $R^{4d}$ and $R^{4e}$ are identical to or different from each other, and each represents a hydrogen atom or the like; $R^{6e}$ represents a C1-C6 chain hydrocarbon group substituted with one or more substituents selected from a group consisting of a cyano group and a halogen atom or the like; and $R^{5a}$ represents a hydrogen atom or the like.

EP 4 603 485 A1

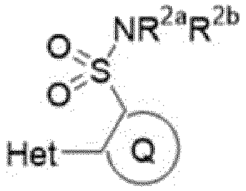 (I)

,

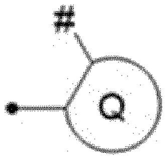

,

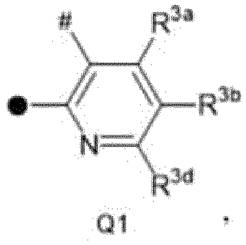

Q1

,

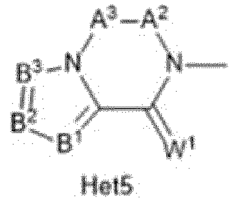

Het5

## Description

TECHNICAL FIELD

This patent application claims the benefit of priority from the Paris Convention based on Japanese Patent Application No. 2022-165233 (filed on October 14, 2022), the entire content of which is incorporated herein by reference.
The present invention relates to sulfonamide compounds and compositions for controlling harmful arthropods comprising the same.

BACKGROUND ART

Various compounds have been studied so far for the purpose of controlling harmful arthropods. For example, Patent Document 1 describes that a certain compound has a pest control effect.

PRIOR ART DOCUMENT

PATENT DOCUMENT

Patent Document 1: WO 2020/158889 A

SUMMARY OF THE INVENTION

PROBLEMS TO BE SOLVED BY THE INVENTION

An object of the present invention is to provide a compound having an excellent control effect on harmful arthropods.

MEANS FOR SOLVING THE PROBLEMS

As a result of investigations to find a compound having an excellent control effect on harmful arthropods, the present inventor has found that a compound represented by the following formula (I) has an excellent control effect on harmful arthropods.
That is, the present invention is as follows.

[1] A compound represented by a formula (I) (hereinafter, referred to as Present compound N) or an N oxide thereof (hereinafter, the compound represented by the formula (I) or the N oxide thereof is referred to as Present compound):

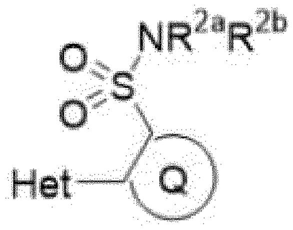

(I)

wherein, in the formula,

Q represented by the following formula

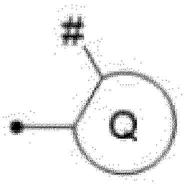

represents a group represented by formula Q1, a group represented by formula Q2, a group represented by

formula Q3, a group represented by formula Q4, a group represented by formula Q5, a group represented by formula Q6, or a group represented by formula Q7, where # represents a binding site to the sulfur atom, and • represents a binding site to Het:

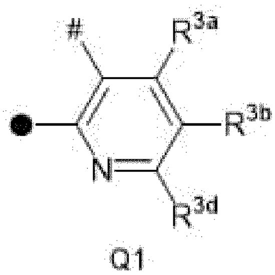
Q1

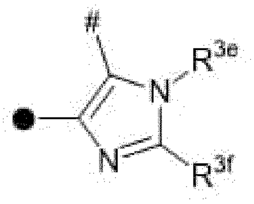
Q2

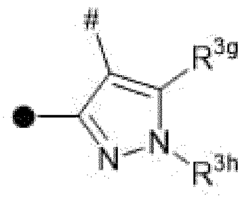
Q3

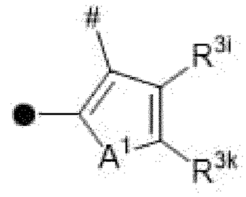
Q4

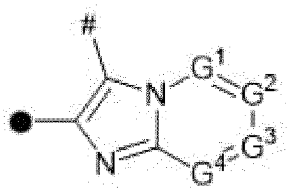
Q5

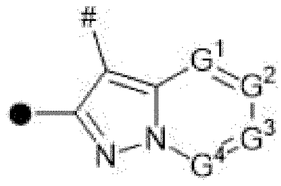
Q6

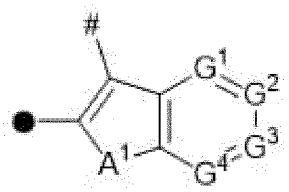
Q7

;

$A^1$ represents $NR^5$, an oxygen atom, or a sulfur atom;

the combination of $G^1$, $G^2$, $G^3$, and $G^4$ represents:

a combination in which $G^1$ is a nitrogen atom or $CR^{3a}$, $G^2$ is $CR^{3b}$, $G^3$ is $CR^{3d}$, and $G^4$ is $CR^{3c}$;

a combination in which $G^1$ is $CR^{3a}$, $G^2$ is a nitrogen atom, $G^3$ is $CR^{3d}$, and $G^4$ is $CR^{3c}$;

a combination in which $G^1$ is $CR^{3a}$, $G^2$ is $CR^{3b}$, $G^3$ is a nitrogen atom, and $G^4$ is $CR^{3c}$; or

a combination in which $G^1$ is $CR^{3a}$, $G^2$ is $CR^{3b}$, $G^3$ is $CR^{3d}$, and $G^4$ is a nitrogen atom;

$R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom; or

$R^{2a}$ and $R^{2b}$ may be combined with the nitrogen atom to which they are attached to form an aziridinyl group, an azetidinyl group, a pyrrolidinyl group, or a piperidyl group;

$R^{3a}$, $R^{3b}$, $R^{3c}$, $R^{3d}$, $R^{3f}$, $R^{3g}$, $R^{3i}$, and $R^{3k}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5- or 6-membered aromatic heterocyclic group optionally substituted with one or more substituents selected from Group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N=CHNR^{31}R^{32}$, $N=S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}=NOR^{17}$, $NR^{11}CR^{24}=NOR^{17}$, $S(O)_qR^{23}$, a cyano group, a nitro group, a hydrogen atom, or a halogen atom;

$R^{3e}$ and $R^{3h}$ are identical to or different from each other, and each represents a C1-C6 alkyl group optionally substituted with one or more halogen atoms, or a phenyl group optionally substituted with one or more substituents selected from Group H;

when Q is a group represented by formula Q1, two adjacent substituents among $R^{3a}$, $R^{3b}$, and $R^{3d}$ may be combined with the two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring {the benzene ring, the pyrrole ring, the furan ring, the thiophene ring, the pyrazole ring, the imidazole ring, the oxazole ring, the isoxazole ring, the thiazole ring, the isothiazole ring, the pyridine ring, the pyridazine ring, the pyrimidine ring, and the pyrazine ring may be substituted with one or more substituents selected from Group H}, or a triazole ring optionally substituted with one or more substituents selected from Group I;

p represents 0 or 1;

q represents 0, 1, or 2;

$R^{30}$ represents a C1-C6 chain hydrocarbon group optionally substituted with one or more halogen atoms, a halogen atom, $OR^{35}$, $NR^{36}R^{37}$, or a hydrogen atom;

$R^{17}$ represents a C1-C6 chain hydrocarbon group optionally substituted with one or more halogen atoms, a phenyl group optionally substituted with one or more substituents selected from Group D, or a hydrogen atom;

$R^{12}$ represents a C1-C6 chain hydrocarbon group optionally substituted with one or more substituents selected from Group F, a C3-C7 cycloalkyl group optionally substituted with one or more substituents selected from Group J, a C3-C7 cycloalkenyl group optionally substituted with one or more substituents selected from Group J, a phenyl group optionally substituted with one or more substituents selected from Group D, a 6-membered aromatic heterocyclic group optionally substituted with one or more substituents selected from Group D, a hydrogen atom, or $S(O)_2R^{23}$;

$R^{23}$ represents a C1-C6 chain hydrocarbon group optionally substituted with one or more halogen atoms, or a phenyl group optionally substituted with one or more substituents selected from Group D;

$R^{11a}$ and $R^{12a}$ are combined with the nitrogen atom to which they are attached to form a 3- to 7-membered non-aromatic heterocyclic group optionally substituted with one or more substituents selected from Group E;

$R^{13}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, a phenyl group optionally substituted with one or more substituents selected from Group D, a 5- or 6-membered aromatic heterocyclic group optionally substituted with one or more substituents selected from Group D, or a hydrogen atom;

$R^{14}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, or a phenyl C1-C3 alkyl group {a phenyl moiety in the phenyl C1-C3 alkyl group may be substituted with one or more substituents selected from Group D};

$R^{15}$ and $R^{16}$ are identical to or different from each other, and each represents a C1-C6 alkyl group optionally substituted with one or more halogen atoms;

$R^{31}$ represents a C1-C6 alkyl group optionally substituted with one or more halogen atoms, or a hydrogen atom;

$R^{32}$ represents a C1-C6 chain hydrocarbon group optionally substituted with one or more substituents selected from Group F, a C3-C7 cycloalkyl group optionally substituted with one or more substituents selected from Group J, or a hydrogen atom;

Het represents a group represented by formula Het 5, a group represented by formula Het 6, or a group represented by formula Het 7:

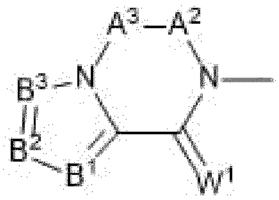
Het5

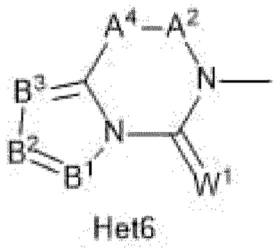
Het6

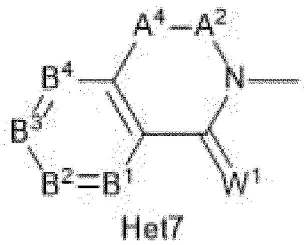
Het7

;

$A^2$ represents $NR^{5a}$ or $CR^{4a}R^{4d}$;

$A^3$ represents $CR^{4b}R^{4e}$;

$A^4$ represents $NR^{5b}$ or $CR^{4c}R^{4f}$;

$W^1$ represents an oxygen atom or a sulfur atom;

when Het is a group represented by formula Het 5,

the combination of $B^1$, $B^2$, and $B^3$ represents:

a combination in which $B^1$ is $CR^{6e}$, $B^2$ is a nitrogen atom or $CR^{6b}$, and $B^3$ is a nitrogen atom or $CR^{6c}$;

a combination in which $B^1$ is a nitrogen atom or $CR^{6a}$, $B^2$ is $CR^{6e}$, and $B^3$ is a nitrogen atom or $CR^{6c}$; or

a combination in which $B^1$ is a nitrogen atom or $CR^{6a}$, $B^2$ is a nitrogen atom or $CR^{6b}$, and $B^3$ is $CR^{6e}$;

when Het is a group represented by formula Het 6,

the combination of $A^2$ and $A^4$ represents:

a combination in which $A^2$ is $CR^{4a}R^{4d}$, and $A^4$ is $NR^{5b}$ or $CR^{4c}R^{4f}$; or
a combination in which $A^2$ is $NR^{5a}$, and $A^4$ is $CR^{4c}R^{4f}$;
the combination of $B^1$, $B^2$, and $B^3$ represents:

a combination in which $B^1$ is $CR^{6e}$, $B^2$ is a nitrogen atom or $CR^{6b}$, and $B^3$ is a nitrogen atom or $CR^{6c}$;
a combination in which $B^1$ is a nitrogen atom or $CR^{6a}$, $B^2$ is $CR^{6e}$, and $B^3$ is a nitrogen atom or $CR^{6c}$; or
a combination in which $B^1$ is a nitrogen atom or $CR^{6a}$, $B^2$ is a nitrogen atom or $CR^{6b}$, and $B^3$ is $CR^{6e}$;
when Het is a group represented by formula Het 7,
the combination of $A^2$ and $A^4$ represents:

a combination in which $A^2$ is $CR^{4a}R^{4d}$, and $A^4$ is $NR^{5b}$ or $CR^{4c}R^{4f}$; or
a combination in which $A^2$ is $NR^{5a}$, and $A^4$ is $CR^{4c}R^{4f}$;
the combination of $B^1$, $B^2$, $B^3$, and $B^4$ represents:

a combination in which $B^1$ is a nitrogen atom or $CR^{6a}$, $B^2$ is $CR^{6e}$, $B^3$ is a nitrogen atom or $CR^{6c}$, and $B^4$ is a nitrogen atom or $CR^{6d}$;
a combination in which $B^1$ is a nitrogen atom or $CR^{6a}$, $B^2$ is a nitrogen atom or $CR^{6b}$, $B^3$ is $CR^{6e}$, and $B^4$ is a nitrogen atom or $CR^{6d}$;
a combination in which $B^1$ is a nitrogen atom or $CR^{6a}$, $B^2$ is a nitrogen atom or $CR^{6b}$, $B^3$ is $CR^{6c}$, and $B^4$ is $CR^{6e}$;
a combination in which $B^1$ is a nitrogen atom or $CR^{6a}$, $B^2$ is $CR^{6b}$, $B^3$ is a nitrogen atom, and $B^4$ is $CR^{6e}$; or
a combination in which $B^1$ is $CR^{6a}$, $B^2$ is a nitrogen atom, $B^3$ is a nitrogen atom, and $B^4$ is $CR^{6e}$;
$R^{4a}$, $R^{4b}$, $R^{4c}$, $R^{6a}$, $R^{6b}$, $R^{6c}$, and $R^{6d}$ are identical to or different from each other, and each represents a C1-C6 chain hydrocarbon group optionally substituted with one or more halogen atoms, a nitro group, $OR^{18}$, $NR^{18}R^{19}$, a cyano group, an amino group, a halogen atom, or a hydrogen atom;
$R^{4d}$, $R^{4e}$, and $R^{4f}$ are identical to or different from each other, and each represents a C1-C6 chain hydrocarbon group optionally substituted with one or more halogen atoms, a cyano group, a halogen atom, or a hydrogen atom;
$R^{6e}$ represents a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $S(O)_mR^7$, $OR^7$, a halogen atom, or $OS(O)_2R^7$;
$R^5$ represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom;
$R^{5a}$ and $R^{5b}$ are identical to or different from each other, and each represents a C1-C6 chain hydrocarbon group optionally substituted with one or more substituents selected from Group K, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom;
$R^7$ represents a C1-C6 chain hydrocarbon group substituted with one or more halogen atoms;
m represents 0, 1, or 2;
$R^{18}$ and $R^{35}$ are identical to or different from each other, and each represents a C1-C6 chain hydrocarbon group optionally substituted with one or more halogen atoms; and
$R^{11}$, $R^{19}$, $R^{24}$, $R^{36}$, and $R^{37}$ are identical to or different from each other, and each

represents a C1-C6 chain hydrocarbon group optionally substituted with one or more halogen atoms, or a hydrogen atom;

Group B: a group consisting of a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, a C3-C6 cycloalkyl group optionally substituted with one or more halogen atoms, a cyano group, a hydroxy group, and a halogen atom;

Group C: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, and a halogen atom;

Group D: a group consisting of a C1-C6 chain hydrocarbon group optionally substituted with one or more halogen atoms, a hydroxy group, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a sulfanyl group, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom,

wherein $R^{21}$ and $R^{22}$ are identical to or different from each other, and each represents a C1-C6 alkyl group optionally substituted with one or more halogen atoms;

Group E: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a halogen atom, an oxo group, a hydroxy group, a cyano group, and a nitro group;

Group F: a group consisting of a C1-C6 alkoxy group optionally substituted with one or more halogen atoms, a phenyl group optionally substituted with one or more substituents selected from Group D, a 5- or 6-membered aromatic heterocyclic group optionally substituted with one or more substituents selected from Group D, a C3-C7 cycloalkyl group optionally substituted with one or more halogen atoms, a 3- to 7-membered non-aromatic heterocyclic group optionally substituted with one or more substituents selected from Group C, an amino group, $NHR^{21}$, $NR^{21}R^{22}$, a halogen atom, and a cyano group;

Group H: a group consisting of a C1-C6 alkyl group optionally substituted with one or more halogen atoms, $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5- or 6-membered aromatic heterocyclic group, wherein $R^9$ represents a C1-C6 alkyl group optionally substituted with one or more halogen atoms, or a C3-C6 cycloalkyl group optionally substituted with one or more halogen atoms, and

$R^{10}$ represents a C1-C6 alkyl group optionally substituted with one or more halogen atoms, a C3-C6 cycloalkyl group optionally substituted with one or more halogen atoms, or a hydrogen atom;

Group I: a group consisting of a C2-C6 chain hydrocarbon group optionally substituted with one or more halogen atoms, a C3-C6 cycloalkyl group optionally substituted with one or more halogen atoms, a phenyl group optionally substituted with one or more substituents selected from Group D, a 5- or 6-membered aromatic heterocyclic group optionally substituted with one or more substituents selected from Group D, a C2-C6 alkylcarbonyl group optionally substituted with one or more halogen atoms, a C2-C6 alkoxycarbonyl group optionally substituted with one or more halogen atoms, an

aminocarbonyl group, a (C1-C6 alkyl) aminocarbonyl group optionally substituted with one or more halogen atoms, a methyl group, and a di (C1-C4 alkyl) aminocarbonyl group optionally substituted with one or more halogen atoms;
Group J: a group consisting of a C1-C6 alkyl group optionally substituted with one or more halogen atoms, a halogen atom, and a cyano group;
Group K: a group consisting of a C1-C6 alkoxy group optionally substituted with one or more halogen atoms, a phenyl group optionally substituted with one or more substituents selected from Group D, a 5- or 6-membered aromatic heterocyclic group optionally substituted with one or more substituents selected from Group D, a C3-C7 cycloalkyl group optionally substituted with one or more halogen atoms, and a 3- to 7-membered non-aromatic heterocyclic group optionally substituted with one or more substituents selected from the group C.

[2] The compound or the N oxide thereof according to [1], wherein Q is a group represented by formula Q2, a group represented by formula Q3, or a group represented by formula Q4.
[3] The compound or the N oxide thereof according to [1], wherein Q is a group represented by formula Q1 or a group represented by formula Q5.
[4] The compound or the N oxide thereof according to [1], wherein Q is a group represented by formula Q5.
[5] The compound or the N oxide of the compound according to [1], wherein

Q is a group represented by formula Q5;
$G^1$ is $CR^{3a}$;
$G^2$ is $CR^{3b}$;
$G^3$ is $CR^{3d}$; and
$G^4$ is $CR^{3c}$.

[6] The compound or the N oxide thereof according to [1], wherein

Q is a group represented by formula Q5;
$G^1$ is $CR^{3a}$;
$G^2$ is $CR^{3b}$;
$G^3$ is $CR^{3d}$;
$G^4$ is $CR^{3c}$;
$R^{3a}$ is a hydrogen atom;
$R^{3b}$ is a C1-C6 chain hydrocarbon group optionally substituted with one or more substituents selected from Group B, a hydrogen atom, or a halogen atom;
$R^{3c}$ is a hydrogen atom; and
$R^{3d}$ is a C1-C6 chain hydrocarbon group optionally substituted with one or more substituents selected from Group B, a hydrogen atom, or a halogen atom.

[7] The compound or the N oxide thereof according to any one of [1] to [6], wherein Het is a group represented by formula Het 7;

$A^2$ is $CR^{4a}R^{4d}$;
$A^4$ is $NR^{5b}$; and
$W^1$ is an oxygen atom.

[8] A composition for controlling a harmful arthropod comprising the compound or an N-oxide thereof according to any one of [1] to [7]; and an inert carrier.
[9] A composition comprising one or more ingredients selected from the group consisting of Group (a), Group (b), Group (c), and Group (d), and the compound or an N-oxide thereof according to any one of [1] to [7]:

Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

[10] A method for controlling a harmful arthropod which comprises applying an effective amount of the compound or an N-oxide thereof according to any one of [1] to [7], or an effective amount of the composition according to [9], to a harmful arthropod or a habitat where a harmful arthropod lives.

[11] A seed or a vegetative reproductive organ holding an effective amount of the compound or the N oxide thereof according to any one of [1] to [7], or an effective amount of the composition according to [9].

EFFECT OF THE INVENTION

According to the present invention, harmful arthropods can be controlled.

MODE FOR CARRYING OUT THE INVENTION

The substituents in the present invention will be described.

The halogen atom means a fluorine atom, a chlorine atom, a bromine atom, or an iodine atom.

When a substituent is substituted with two or more halogen atoms or substituents, the halogen atoms or substituents may be identical to or different from each other.

In the present specification, the notation of "CX-CY" means that the number of carbon atoms is X to Y. For example, the notation "C1-C6" means that the number of carbon atoms is 1 to 6.

The chain hydrocarbon group represents an alkyl group, an alkenyl group, or an alkynyl group.

Examples of the alkyl group include a methyl group, an ethyl group, a propyl group, an isopropyl group, a 1,1-dimethylpropyl group, a 1,2-dimethylpropyl group, a 1-ethylpropyl group, a butyl group, a sec-butyl group, a tert-butyl group, a pentyl group, and a hexyl group.

Examples of the alkenyl group include a vinyl group, a 1-propenyl group, a 2-propenyl group, a 1-methyl-1-propenyl group, a 1-methyl-2-propenyl group, a 1,2-dimethyl-1-propenyl group, a 1-ethyl-2-propenyl group, a 3-butenyl group, a 4-pentenyl group, and a 5-hexenyl group.

Examples of the alkynyl group include an ethynyl group, a 1-propynyl group, a 2-propynyl group, a 1-methyl-2-propynyl group, a 1,1-dimethyl-2-propynyl group, a 1-ethyl-2-propynyl group, a 2-butynyl group, a 4-pentynyl group, and a 5-hexynyl group.

Examples of the alkoxy group include a methoxy group, an ethoxy group, a propoxy group, an isopropoxy group, a butoxy group, a tert-butoxy group, a pentyloxy group, and a hexyloxy group.

Examples of the alkenyloxy group include a 2-propenyloxy group, a 2-butenyloxy group, and a 5-hexenyloxy group.

Examples of the alkynyloxy group include a 2-propynyloxy group, a 2-butynyloxy group, and a 5-hexynyloxy group.

Examples of the cycloalkyl group include a cyclopropyl group, a cyclobutyl group, a cyclopentyl group, a cyclohexyl group, and a cycloheptyl group.

Examples of the cycloalkenyl group include a cyclopropenyl group, a cyclobutenyl group, a cyclopentenyl group, a cyclohexenyl group, and a cycloheptenyl group.

Examples of the 3-7 membered non-aromatic heterocyclic group include an aziridinyl group, an oxiranyl group, a thiiranyl group, an azetidinyl group, an oxetanyl group, a thietanyl group, a pyrrolidinyl group, a tetrahydrofuranyl group, a tetrahydrothienyl group, a piperidyl group, a pyranyl group, a dihydropyranyl group, a tetrahydropyranyl group, a tetrahydrothiopyranyl group, an azepanyl group, an oxepanyl group, a thiepanyl group, a pyrazolinyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolyl group, a dioxolanyl group, a dioxanyl group, a morpholinyl group, a thiomorpholinyl group, and a piperazinyl group.

Examples of the 5- or 6-membered aromatic heterocyclic group include a pyrrolyl group, a furyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a pyridyl group, a pyridazinyl group, a pyrimidinyl group, a pyrazinyl group, a triazinyl group, and a tetrazinyl group.

Examples of the 6 membered aromatic heterocyclic group include a pyridyl group, a pyridazinyl group, a pyrimidinyl group, a pyrazinyl group, a triazinyl group, and a tetrazinyl group.

The (C3-C6 cycloalkyl) C1-C3 alkyl group optionally substituted with one or more halogen atoms represents a group in which the (C3-C6 cycloalkyl) and/or the (C1-C3 alkyl) is optionally substituted with one or more halogen atoms. Examples thereof include a cyclopropylmethyl group, a (2-fluorocyclopropyl) methyl group, a cyclopropyl (fluoro) methyl group, and a (2-fluorocyclopropyl) (fluoro) methyl group.

The (C3-C7 cycloalkyl) C1-C6 alkyl group optionally substituted with one or more halogen atoms represents a group in which the (C3-C7 cycloalkyl) and/or the (C1-C6 alkyl) is optionally substituted with one or more halogen atoms. Examples thereof include a (2,2-difluorocyclopropyl) methyl group, a 2-cyclopropyl-1,1,2,2-tetrafluoroethyl group, a

2-(2,2-difluorocyclopropyl)-1,1,2,2-tetrafluoroethyl group, a (2,2-difluorocyclopropyl) propyl group, a (2,2-difluorocyclopropyl) butyl group, a (2,2-difluorocyclopropyl) pentyl group, and a (2,2-difluorocyclopropyl) hexyl group.

Examples of the phenyl C1-C3 alkyl group {wherein the phenyl moiety in the phenyl C1-C3 alkyl group may be substituted with one or more substituents selected from Group D} include a benzyl group, a 2-fluorobenzyl group, a 4-(difluoromethyl) benzyl group, a 4-fluorobenzyl group, a 4-(trifluoromethyl) benzyl group, and a 2-[4-(trifluoromethyl) phenyl] ethyl group.

The alkylsulfanyl group, the alkylsulfinyl group, and the alkylsulfonyl group represent an alkyl group having a moiety represented by $S(O)_z$.

Examples of the alkylsulfanyl group in which z is 0 include a methylsulfanyl group, an ethylsulfanyl group, a propylsulfanyl group, and an isopropylsulfanyl group.

Examples of the alkylsulfinyl group in which z is 1 include a methylsulfinyl group, an ethylsulfinyl group, a propylsulfinyl group, and an isopropylsulfinyl group.

Examples of the alkylsulfonyl group in which z is 2 include a methylsulfonyl group, an ethylsulfonyl group, a propylsulfonyl group, and an isopropylsulfonyl group.

Examples of the alkylcarbonyl group include an acetyl group, a propanoyl group, a 2-methylpropanoyl group, and a hexanoyl group.

Examples of the alkoxycarbonyl group include a methoxycarbonyl group, an ethoxycarbonyl group, an isopropoxycarbonyl group, and a pentyloxycarbonyl group.

Examples of the (C1-C6 alkyl) aminocarbonyl group include a methylaminocarbonyl group, an ethylaminocarbonyl group, a propylaminocarbonyl group, an isopropylaminocarbonyl group, a butylaminocarbonyl group, a pentylaminocarbonyl group, and a hexylaminocarbonyl group.

As the di (C1-C4 alkyl) aminocarbonyl group, the two (C1-C4 alkyl) moieties may be identical to or different from each other. Examples thereof include a dimethylaminocarbonyl group, a diethylaminocarbonyl group, a dipropylaminocarbonyl group, a dibutylaminocarbonyl group, an ethylmethylaminocarbonyl group, and an ethylisopropylaminocarbonyl group.

Examples of the N oxide of the compound represented by formula (I) include a compound represented by the following formula:

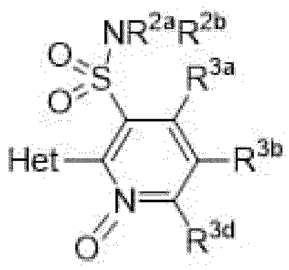

in which, in the formula, the symbols represent the same meaning as described above.

In the present specification, the term "Present compound" is referred to, the Present compound includes the Present compound N unless otherwise specified.

The Present compound may have one or more stereoisomers. Stereoisomers include enantiomers, diastereomers, geometric isomers, and the like. The Present compound includes each stereoisomer and a stereoisomer mixture in any ratio.

The Present compound may form an acid addition salt. Examples of an acid to from the acid addition salt include inorganic acids such as hydrogen chloride, phosphoric acid, and sulfuric acid, and organic acids such as acetic acid, trifluoroacetic acid, benzoic acid, and p-toluenesulfonic acid. The acid addition salt is obtained by mixing the Present compound with the acid.

Aspects of the Present compound N include the following compounds.

[Aspect 1] The Present compound N, wherein Q is a group represented by formula Q2, a group represented by formula Q3, or a group represented by formula Q4.

[Aspect 2] The Present compound N, wherein Q is a group represented by formula Q1 or a group represented by formula Q5.

[Aspect 3] The Present compound N, wherein Q is a group represented by formula Q5.

[Aspect 4] The Present compound N, wherein Q is a group represented by formula Q5;

$G^1$ is $CR^{3a}$;

$G^2$ is $CR^{3b}$;
$G^3$ is $CR^{34}$; and
$G^4$ is $CR^{3c}$.

[Aspect 5] The Present compound N, wherein Q is a group represented by formula Q5;

$G^1$ is $CR^{3a}$;
$G^2$ is $CR^{3b}$;
$G^3$ is $CR^{3d}$;
$G^4$ is $CR^{3c}$;
$R^{3a}$ is a hydrogen atom;
$R^{3b}$ is a C1-C6 chain hydrocarbon group optionally substituted with one or more substituents selected from Group B, a hydrogen atom, or a halogen atom;
$R^{3c}$ is a hydrogen atom; and
$R^{3d}$ is a C1-C6 chain hydrocarbon group optionally substituted with one or more substituents selected from Group B, a hydrogen atom, or a halogen atom.

[Aspect 6] The Present compound N, wherein Q is a group represented by formula Q5;

$G^1$ is $CR^{3a}$;
$G^2$ is $CR^{3b}$;
$G^3$ is $CR^{3d}$;
$G^4$ is $CR^{3c}$;
$R^{3a}$ is a hydrogen atom;
$R^{3b}$ is a C1-C6 chain hydrocarbon group optionally substituted with one or more substituents selected from Group B, or a halogen atom;
$R^{3c}$ is a hydrogen atom; and
$R^{3d}$ is a hydrogen atom.

[Aspect 7] The Present compound N, wherein Q is a group represented by formula Q5;

$G^1$ is $CR^{3a}$;
$G^2$ is $CR^{3b}$;
$G^3$ is $CR^{3d}$;
$G^4$ is $CR^{3c}$;
$R^{3a}$, $R^{3c}$, and $R^{3d}$ are hydrogen atoms; and
$R^{3b}$ is a C1-C6 chain hydrocarbon group optionally substituted with one or more substituents selected from Group B.

[Aspect 8] The Present compound N, wherein Het is a group represented by formula Het 7;

$A^2$ is $CR^{4a}R^{4d}$;
$A^4$ is $NR^{5b}$; and
$W^1$ is an oxygen atom.

[Aspect 9] The Present compound N, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 10] The Present compound N, wherein Het is a group represented by formula Het 5;

$A^2$ is $CR^{4a}R^{4d}$; and
$A^3$ is $CR^{4b}R^{4e}$.

[Aspect 11] The Present compound N, wherein Het is a group represented by formula Het 7;

$A^2$ is $CR^{4a}R^{4d}$;
$A^4$ is $NR^{5b}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 12] The Present compound N, wherein Het is a group represented by formula Het 6;

$A^2$ is $CR^{4a}R^{4d}$, and
$W^1$ is an oxygen atom.

[Aspect 13] The Present compound N, wherein Het is a group represented by formula Het 5;

$A^2$ is $CR^{4a}R^{4d}$;
$A^3$ is $CR^{4b}R^{4e}$; and
$W^1$ is an oxygen atom.

[Aspect 14] The Present compound N, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 15] The Present compound N, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 16] The Present compound N, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 17] The Present compound N, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom.

[Aspect 18] The Present compound N, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 19] The Present compound N, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 20] The compound according to the Aspect 1, wherein Het is a group represented by formula Het 7;

$A^2$ is $CR^{4a}R^{4d}$;
$A^4$ is $NR^{5b}$; and
$W^1$ is an oxygen atom.

[Aspect 21] The compound according to the Aspect 2, wherein Het is a group represented by formula Het 7;

$A^2$ is $CR^{4a}R^{4d}$;
$A^4$ is $NR^{5b}$; and
$W^1$ is an oxygen atom.

[Aspect 22] The compound according to the Aspect 3, wherein Het is a group represented by formula Het 7;

$A^2$ is $CR^{4a}R^{4d}$;
$A^4$ is $NR^{5b}$; and
$W^1$ is an oxygen atom.

[Aspect 23] The compound according to the Aspect 4, wherein Het is a group represented by formula Het 7;

$A^2$ is $CR^{4a}R^{4d}$;
$A^4$ is $NR^{5b}$; and

$W^1$ is an oxygen atom.

[Aspect 24] The compound according to the Aspect 5, wherein Het is a group represented by formula Het 7;

$A^2$ is $CR^{4a}R^{4d}$;
$A^4$ is $NR^{5b}$; and
$W^1$ is an oxygen atom.

[Aspect 25] The compound according to the Aspect 6, wherein Het is a group represented by formula Het 7;

$A^2$ is $CR^{4a}R^{4d}$;
$A^4$ is $NR^{5b}$; and
$W^1$ is an oxygen atom.

[Aspect 26] The compound according to the Aspect 7, wherein Het is a group represented by formula Het 7;

$A^2$ is $CR^{4a}R^{4d}$;
$A^4$ is $NR^{5b}$; and
$W^1$ is an oxygen atom.

[Aspect 27] The compound according to the Aspect 1, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 28] The compound according to the Aspect 2, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 29] The compound according to the Aspect 3, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 30] The compound according to the Aspect 4, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 31] The compound according to the Aspect 5, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 32] The compound according to the Aspect 6, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 33] The compound according to the Aspect 7, wherein Het is a group represented by formula Het 6;

$A^2$ is $CR^{4a}R^{4d}$;
$A^4$ is $CR^{4c}R^{4f}$; and
$W^1$ is an oxygen atom.

[Aspect 34] The compound according to the Aspect 1, wherein Het is a group represented by formula Het 5;

$A^2$ is $CR^{4a}R^{4d}$; and
$A^3$ is $CR^{4b}R^{4e}$.

[Aspect 35] The compound according to the Aspect 2, wherein Het is a group represented by formula Het 5;

$A^2$ is $CR^{4a}R^{4d}$; and
$A^3$ is $CR^{4b}R^{4e}$.

[Aspect 36] The compound according to the Aspect 3, wherein Het is a group represented by formula Het 5;

$A^2$ is $CR^{4a}R^{4d}$; and
$A^3$ is $CR^{4b}R^{4e}$.

[Aspect 37] The compound according to the Aspect 4, wherein Het is a group represented by formula Het 5;

$A^2$ is $CR^{4a}R^{4d}$; and
$A^3$ is $CR^{4b}R^{4e}$.

[Aspect 38] The compound according to the Aspect 5, wherein Het is a group represented by formula Het 5;

$A^2$ is $CR^{4a}R^{4d}$; and
$A^3$ is $CR^{4b}R^{4e}$.

[Aspect 39] The compound according to the Aspect 6, wherein Het is a group represented by formula Het 5;

$A^2$ is $CR^{4a}R^{4d}$; and
$A^3$ is $CR^{4b}R^{4e}$.

[Aspect 40] The compound according to the Aspect 7, wherein Het is a group represented by formula Het 5;

$A^2$ is $CR^{4a}R^{4d}$; and
$A^3$ is $CR^{4b}R^{4e}$.

[Aspect 41] The compound according to the Aspect 1, wherein Het is a group represented by formula Het 7;

$A^2$ is $CR^{4a}R^{4d}$;
$A^4$ is $NR^{5b}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 42] The compound according to the Aspect 2, wherein Het is a group represented by formula Het 7;

$A^2$ is $CR^{4a}R^{4d}$;
$A^4$ is $NR^{5b}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 43] The compound according to the Aspect 3, wherein Het is a group represented by formula Het 7;

$A^2$ is $CR^{4a}R^{4d}$;
$A^4$ is $NR^{5b}$;
$W^1$ is an oxygen atom, and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 44] The compound according to the Aspect 4, wherein Het is a group represented by formula Het 7;

$A^2$ is $CR^{4a}R^{4d}$;
$A^4$ is $NR^{5b}$;
$W^1$ is an oxygen atom, and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 45] The compound according to the Aspect 5, wherein Het is a group represented by formula Het 7;

$A^2$ is $CR^{4a}R^{4d}$;
$A^4$ is $NR^{5b}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 46] The compound according to the Aspect 6, wherein Het is a group represented by formula Het 7;

$A^2$ is $CR^{4a}R^{4d}$;
$A^4$ is $NR^{5b}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 47] The compound according to the Aspect 7, wherein Het is a group represented by formula Het 7;

$A^2$ is $CR^{4a}R^{4d}$;
$A^4$ is $NR^{5b}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is $CR^{6d}$; or
a combination in which $B^1$ is $CR^{6a}$, $B^2$ is $CR^{6b}$, $B^3$ is $CR^{6e}$, and $B^4$ is $CR^{6d}$.

[Aspect 48] The compound according to the Aspect 1, wherein Het is a group represented by formula Het 6, $A^2$ is $CR^{4a}R^{4d}$, $A^4$ is $NR^{5b}$ or $CR^{4c}R^{4f}$, and $W^1$ is an oxygen atom.
[Aspect 49] The compound according to the Aspect 2, Het is a group represented by formula Het 6, $A^2$ is $CR^{4a}R^{4d}$, $A^4$ is $NR^{5b}$ or $CR^{4c}R^{4f}$, and $W^1$ is an oxygen atom.
[Aspect 50] The compound according to the Aspect 3, wherein Het is a group represented by formula Het 6;

$A^2$ is $CR^{4a}R^{4d}$;
$A^4$ is $NR^{5b}$ or $CR^{4c}R^{4f}$; and

$W^1$ is an oxygen atom.

[Aspect 51] The compound according to the Aspect 4, wherein Het is a group represented by formula Het 6;

$A^2$ is $CR^{4a}R^{4d}$;
$A^4$ is $NR^{5b}$ or $CR^{4c}R^{4f}$; and
$W^1$ is an oxygen atom.

[Aspect 52] The compound according to the Aspect 5, wherein Het is a group represented by formula Het 6;

$A^2$ is $CR^{4a}R^{4d}$;
$A^4$ is $NR^{5b}$ or $CR^{4c}R^{4f}$; and
$W^1$ is an oxygen atom.

[Aspect 53] The compound according to the Aspect 6, wherein Het is a group represented by formula Het 6;

$A^2$ is $CR^{4a}R^{4d}$;
$A^4$ is $NR^{5b}$ or $CR^{4c}R^{4f}$; and
$W^1$ is an oxygen atom.

[Aspect 54] The compound according to the Aspect 7, wherein Het is a group represented by formula Het 6;

$A^2$ is $CR^{4a}R^{4d}$;
$A^4$ is $NR^{5b}$ or $CR^{4c}R^{4f}$; and
$W^1$ is an oxygen atom.

[Aspect 55] The compound according to the Aspect 1, wherein Het is a group represented by formula Het 5;

$A^2$ is $CR^{4a}R^{4d}$;
$A^3$ is $CR^{4b}R^{4e}$; and
$W^1$ is an oxygen atom.

[Aspect 56] The compound according to the Aspect 2, wherein Het is a group represented by formula Het 5;

$A^2$ is $CR^{4a}R^{4d}$;
$A^3$ is $CR^{4b}R^{4e}$; and
$W^1$ is an oxygen atom.

[Aspect 57] The compound according to the Aspect 3, wherein Het is a group represented by formula Het 5;

$A^2$ is $CR^{4a}R^{4d}$;
$A^3$ is $CR^{4b}R^{4e}$; and
$W^1$ is an oxygen atom.

[Aspect 58] The compound according to the Aspect 4, wherein Het is a group represented by formula Het 5;

$A^2$ is $CR^{4a}R^{4d}$;
$A^3$ is $CR^{4b}R^{4e}$; and
$W^1$ is an oxygen atom.

[Aspect 59] The compound according to the Aspect 5, wherein Het is a group represented by formula Het 5;

$A^2$ is $CR^{4a}R^{4d}$;
$A^3$ is $CR^{4b}R^{4e}$, and
$W^1$ is an oxygen atom.

[Aspect 60] The compound according to the Aspect 6, wherein Het is a group represented by formula Het 5;

$A^2$ is $CR^{4a}R^{4d}$;
$A^3$ is $CR^{4b}R^{4e}$; and
$W^1$ is an oxygen atom.

[Aspect 61] The compound according to the Aspect 7, wherein Het is a group represented by formula Het 5;

$A^2$ is $CR^{4a}R^{4d}$;
$A^3$ is $CR^{4b}R^{4e}$; and
$W^1$ is an oxygen atom.

[Aspect 62] The compound according to the Aspect 1, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 63] The compound according to the Aspect 2, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 64] The compound according to the Aspect 3, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 65] The compound according to the Aspect 4, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 66] The compound according to the Aspect 5, wherein Het is a group represented by formula Het 7;

$A^2$ is $CR^{4a}R^{4d}$;
$A^4$ is $NR^{5b}$;

$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 67] The compound according to the Aspect 6, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 68] The compound according to the Aspect 7, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$; and
$R^{6a}$, $R^{6c}$, and $R^{6d}$ are hydrogen atoms.

[Aspect 69] The compound according to the Aspect 1, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 70] The compound according to the Aspect 2, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 71] The compound according to the Aspect 3, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 72] The compound according to the Aspect 4, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 73] The compound according to the Aspect 5, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 74] The compound according to the Aspect 6, wherein Het is a group represented by formula Het 7;

$A^2$ is $CR^{4a}R^{4d}$;
$A^4$ is $NR^{5b}$ ;
$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 75] The compound according to the Aspect 7, wherein Het is a group represented by formula Het 7;

$A^2$ is $CR^{4a}R^{4d}$;
$A^4$ is $NR^{5b}$;

$W^1$ is an oxygen atom;
$B^1$ is $CR^{6a}$;
$B^2$ is $CR^{6e}$;
$B^3$ is $CR^{6c}$;
$B^4$ is $CR^{6d}$;
$R^{4a}$, $R^{4d}$, $R^{6a}$, $R^{6c}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 76] The compound according to the Aspect 1, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 77] The compound according to the Aspect 2, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 78] The compound according to the Aspect 3, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 79] The compound according to the Aspect 4, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;

$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 80] The compound according to the Aspect 5, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6a}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 81] The compound according to the Aspect 6, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 82] The compound according to the Aspect 7, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 83] The compound according to the Aspect 1, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;

$B^4$ is $CR^{6a}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom.

[Aspect 84] The compound according to the Aspect 2, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms and
$R^{6e}$ is a C1-C6 chain hydrocarbon group substituted with one or more substituents selected from a group consisting of a cyano group and a halogen atom.

[Aspect 85] The compound according to the Aspect 3, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom.

[Aspect 86] The compound according to the Aspect 4, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6a}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom.

[Aspect 87] The compound according to the Aspect 5, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6a}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom.

[Aspect 88] The compound according to the Aspect 6, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom.

[Aspect 89] The compound according to the Aspect 7, wherein Het is a group represented by formula Het 7;

$A^2$ is $CR^{4a}R^{4d}$;
$A^4$ is $NR^{5b}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom.

[Aspect 90] The compound according to the Aspect 1, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 91] The compound according to the Aspect 2, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 92] The compound according to the Aspect 3, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 93] The compound according to the Aspect 4, wherein Het is a group represented by formula Het 5;

$A^2$ is $CR^{4a}R^{4d}$;
$A^3$ is $CR^{4b}R^{4e}$;

$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 94] The compound according to the Aspect 5, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 95] The compound according to the Aspect 6, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 96] The compound according to the Aspect 7, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom; and
$R^{4a}$, $R^{4b}$, $R^{4d}$, $R^{4e}$, and $R^{6b}$ are hydrogen atoms.

[Aspect 97] The compound according to the Aspect 1, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 98] The compound according to the Aspect 2, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and

$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 99] The compound according to the Aspect 3, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 100] The compound according to the Aspect 4, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 101] The compound according to the Aspect 5, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 102] The compound according to the Aspect 6, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 103] The compound according to the Aspect 7, wherein Het is a group represented by formula Het 5;

$A^2$ is $CR^{4a}R^{4d}$;
$A^3$ is $CR^{4b}R^{4e}$;
$W^1$ is an oxygen atom;
$B^1$ is $CR^{6e}$;
$B^2$ is $CR^{6b}$;
$B^3$ is a nitrogen atom;
$R^{4a}$, $R^{4b}$, $R^{4d}$, $R^{4e}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect 104] Any one of the compound according to Aspects 1 to 103 and the Present compound N, wherein $R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$ are combined with the nitrogen atom to which they are attached to form a pyrrolidinyl group or a piperidyl group.
[Aspect 105] Any one of the compound according to Aspects 1 to 103 and the Present compound N, wherein $R^{2a}$ and $R^{2b}$ are identical to or different from each other, and each represents a C1-C6 alkyl group optionally substituted with one or more halogen atoms, or a hydrogen atom, or $R^{2a}$ and $R^{2b}$ are combined with the nitrogen atom to which they are attached to form a pyrrolidinyl group or a piperidyl group.
[Aspect 106] Any one of the compound according to Aspects 1 to 103 and the Present compound N, wherein $R^{2a}$ and $R^{2b}$ are identical to or different from each other, and each represents a C1-C6 alkyl group optionally substituted with one or more halogen atoms.
[Aspect A1] The Present compound N, wherein Q is a group represented by formula Q1.
[Aspect A2] The Present compound N, wherein Q is a group represented by formula Q1;

$R^{3a}$ is a hydrogen atom;
$R^{3b}$ is a C1-C6 chain hydrocarbon group optionally substituted with one or more halogen atoms, a C3-C7 cycloalkyl group optionally substituted with one or more halogen atoms, a phenyl group optionally substituted with one or more halogen atoms, a 5- or 6-membered aromatic heterocyclic group optionally substituted with one or more halogen atoms, $OR^{12}$, a hydrogen atom, or a halogen atom;
$R^{12}$ is a C1-C6 chain hydrocarbon group optionally substituted with one or more halogen atoms; and
$R^{3d}$ is a hydrogen atom.

[Aspect A3] The Present compound N, wherein Q is a group represented by formula Q1;

$R^{3a}$ is a hydrogen atom;
$R^{3b}$ is a hydrogen atom; and
$R^{3d}$ is a hydrogen atom.

[Aspect A4] The Present compound N, wherein Het is a group represented by formula Het 7;

$A^2$ is $CR^{4a}R^{4d}$;
$A^4$ is $CR^{4c}R^{4f}$; and
$W^1$ is an oxygen atom.

[Aspect A5] The Present compound N, wherein Het is a group represented by formula Het 7;

$A^2$ is $CR^{4a}R^{4d}$;
$A^4$ is $CR^{4c}R^{4f}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is a nitrogen atom;
a combination in which $B^1$ is $CR^{6a}$, $B^2$ is $CR^{6b}$, $B^3$ is $CR^{6e}$, and $B^4$ is a nitrogen atom; or
a combination in which $B^1$ is $CR^{6a}$, $B^2$ is $CR^{6b}$, $B^3$ is $CR^{6e}$, and $B^4$ is $CR^{6d}$.

[Aspect A6] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom; and
$R^{6a}$ and $R^{6c}$ are hydrogen atoms.

[Aspect A7] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A8] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a halogen atom.

[Aspect A9] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom; and
$R^{6a}$ and $R^{6b}$ are hydrogen atoms.

[Aspect A10] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more

substituents selected from a group consisting of a cyano group and a halogen atom, $OR^7$, or a halogen atom.

[Aspect A11] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and
$R^{6e}$ is a halogen atom.

[Aspect A12] In the Present compound N, Het is a group represented by formula Het 7, $A^2$ is $CR^{4a}R^{4d}$, $A^4$ is $CR^{4c}R^{4f}$, $W^1$ is an oxygen atom, $B^1$ is $CR^{6a}$, $B^2$ is $CR^{6b}$, $B^3$ is $CR^{6e}$, $B^4$ is $CR^{6d}$, and $R^{6a}$, $R^{6b}$, and $R^{6d}$ are hydrogen atoms.
[Aspect A13] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{4c}$, $R^{4f}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A14] The Present compound N, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{4c}$, $R^{4f}$, $R^{6a}$, $R^{6b}$, and $R^{6d}$ are hydrogen atoms; and
$R^{6e}$ is a halogen atom.

[Aspect A15] The compound according to the Aspect A1, wherein Het is a group represented by formula Het 7;

$A^2$ is $CR^{4a}R^{4d}$;
$A^4$ is $CR^{4c}R^{4f}$ and
$W^1$ is an oxygen atom.

[Aspect A16] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is a nitrogen atom;
a combination in which $B^1$ is $CR^{6a}$, $B^2$ is $CR^{6b}$, $B^3$ is $CR^{6e}$, and $B^4$ is a nitrogen atom; or
a combination in which $B^1$ is $CR^{6a}$, $B^2$ is $CR^{6b}$, $B^3$ is $CR^{6e}$, and $B^4$ is $CR^{6a}$.

[Aspect A17] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom; and
$R^{6a}$ and $R^{6c}$ are hydrogen atoms.

[Aspect A18] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A19] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a halogen atom.

[Aspect A20] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom; and
$R^{6a}$ and $R^{6b}$ are hydrogen atoms.

[Aspect A21] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and

$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A22] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and
$R^{6e}$ is a halogen atom.

[Aspect A23] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$; and
$R^{6a}$, $R^{6b}$, and $R^{6d}$ are hydrogen atoms.

[Aspect A24] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{4c}$, $R^{4f}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A25] The compound according to the Aspect A1, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{4c}$, $R^{4f}$, $R^{6a}$, $R^{6b}$, and $R^{6d}$ are hydrogen atoms; and
$R^{6e}$ is a halogen atom.

[Aspect A26] The compound according to the Aspect A2, wherein Het is a group represented by formula Het 7;

$A^2$ is $CR^{4a}R^{4d}$;
$A^4$ is $CR^{4c}R^{4f}$; and
$W^1$ is an oxygen atom.

[Aspect A27] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is a nitrogen atom;
a combination in which $B^1$ is $CR^{6a}$, $B^2$ is $CR^{6b}$, $B^3$ is $CR^{6e}$, and $B^4$ is a nitrogen atom; or
a combination in which $B^1$ is $CR^{6a}$, $B^2$ is $CR^{6b}$, $B^3$ is $CR^{6e}$, and $B^4$ is $CR^{6d}$.

[Aspect A28] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom; and
$R^{6a}$ and $R^{6c}$ are hydrogen atoms.

[Aspect A29] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A30] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a halogen atom.

[Aspect A31] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom; and
$R^{6a}$ and $R^{6b}$ are hydrogen atoms.

[Aspect A32] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A33] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and
$R^{6e}$ is a halogen atom.

[Aspect A34] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$; and
$R^{6a}$, $R^{6b}$, and $R^{6d}$ are hydrogen atoms.

[Aspect A35] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{4c}$, $R^{4f}$, $R^{6a}$, $R^{6b}$, and $R^{6d}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A36] The compound according to the Aspect A2, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;

$B^3$ is $CR^{6e}$;
$B^4$ is $CR^{6d}$
$R^{4a}$, $R^{4d}$, $R^{4c}$, $R^{4f}$, $R^{6a}$, $R^{6b}$, and $R^{6d}$ are hydrogen atoms; and
$R^{6e}$ is a halogen atom.

[Aspect A37] The compound according to the Aspect A3, wherein Het is a group represented by formula Het 7;

$A^2$ is $CR^{4a}R^{4d}$;
$A^4$ is $CR^{4c}R^{4f}$; and
$W^1$ is an oxygen atom.

[Aspect A38] The compound according to the Aspect A3, wherein Het is a group represented by formula Het 7;

$A^2$ is $CR^{4a}R^{4d}$;
$A^4$ is $CR^{4}CR^{4f}$;
$W^1$ is an oxygen atom; and
the combination of $B^1$, $B^2$, $B^3$, and $B^4$ represents:

a combination in which $B^1$ is $CR^{6a}$, $B^2$ is $CR^{6e}$, $B^3$ is $CR^{6c}$, and $B^4$ is a nitrogen atom;
a combination in which $B^1$ is $CR^{6a}$, $B^2$ is $CR^{6b}$, $B^3$ is $CR^{6e}$, and $B^4$ is a nitrogen atom; or
a combination in which $B^1$ is $CR^{6a}$, $B^2$ is $CR^{6b}$, $B^3$ is $CR^{6e}$, and $B^4$ is $CR^{6a}$.

[Aspect A39] The compound according to the Aspect A3, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is $CR^{6c}$;
$B^4$ is a nitrogen atom; and
$R^{6a}$ and $R^{6c}$ are hydrogen atoms.

[Aspect A40] The compound according to the Aspect A3, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is CR6c;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A41] The compound according to the Aspect A3, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6e}$;
$B^3$ is CR6c;
$B^4$ is a nitrogen atom;
$R^{4a}$, $R^{4d}$, $R^{4c}$, $R^{4f}$, $R^{6a}$, and $R^{6c}$ are hydrogen atoms; and
$R^{6e}$ is a halogen atom.

[Aspect A42] The compound according to the Aspect A3, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom; and
$R^{6a}$ and $R^{6b}$ are hydrogen atoms.

[Aspect A43] The compound according to the Aspect A3, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and
$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A44] The compound according to the Aspect A3, wherein Het is a group represented by formula Het 7;

$A^2$ is $CR^{4a}R^{4d}$;
$A^4$ is $CR^4CR^{4f}$;
$W^1$ is an oxygen atom;
$B^1$ is $CR^{6a}$;
$B^2$ is $CR^{6b}$;
$B^3$ is $CR^{6e}$;
$B^4$ is a nitrogen atom;
$R^{4a}$, $R^{4d}$, $R^{4c}$, $R^{4f}$, $R^{6a}$, and $R^{6b}$ are hydrogen atoms; and
$R^{6e}$ is a halogen atom.

[Aspect A45] The compound according to the Aspect A3, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$; and
$R^{6a}$, $R^{6b}$, and $R^{6d}$ are hydrogen atoms.

[Aspect A46] The compound according to the Aspect A3, wherein Het is a group represented by formula Het 7;

$A^2$ is $CR^{4a}R^{4d}$;
$A^4$ is $CR^4CR^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$
$R^{4a}$, $R^{4d}$, $R^{4c}$, $R^{4f}$, $R^{6a}$, $R^{6b}$, and $R^{6d}$ are hydrogen atoms; and

$R^{6e}$ is a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $OR^7$, or a halogen atom.

[Aspect A47] The compound according to the Aspect A3, wherein Het is a group represented by formula Het 7;

$A^2$ is $CR^{4a}R^{4d}$;
$A^4$ is $CR^{4c}R^{4f}$;
$W^1$ is an oxygen atom;
$B^1$ is $CR^{6a}$;
$B^2$ is $CR^{6b}$;
$B^3$ is $CR^{6e}$;
$B^4$ is $CR^{6d}$;
$R^{4a}$, $R^{4d}$, $R^{4c}$, $R^{4f}$, $R^{6a}$, $R^{6b}$, and $R^{6d}$ are hydrogen atoms; and
$R^{6e}$ is a halogen atom.

[Aspect A48] Any one of the compound according to Aspects A1 to A47, wherein $R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$ are combined with the nitrogen atom to which they are attached to form a pyrrolidinyl group or a piperidyl group.
[Aspect A49] Any one of the compound according to Aspects A1 to A47, wherein $R^{2a}$ and $R^{2b}$ are identical to or different from each other, and each represents a C1-C6 alkyl group optionally substituted with one or more halogen atoms, or a hydrogen atom, or $R^{2a}$ and $R^{2b}$ are combined with the nitrogen atom to which they are attached to form a pyrrolidinyl group or a piperidyl group.
[Aspect A50] Any one of the compound according to Aspects A1 to A47, wherein $R^{2a}$ and $R^{2b}$ are identical to or different from each other, and each represents a C1-C6 alkyl group optionally substituted with one or more halogen atoms.

Next, the production method of the Present compound (including the Present compound N) will be described.

Production Method 1

The compound represented by formula (I) (Present compound N) can be produced by reacting a compound represented by formula (M-1) (hereinafter, referred to as compound (M-1)) with a compound represented by formula (M-2) (hereinafter, referred to as compound (M-2)):

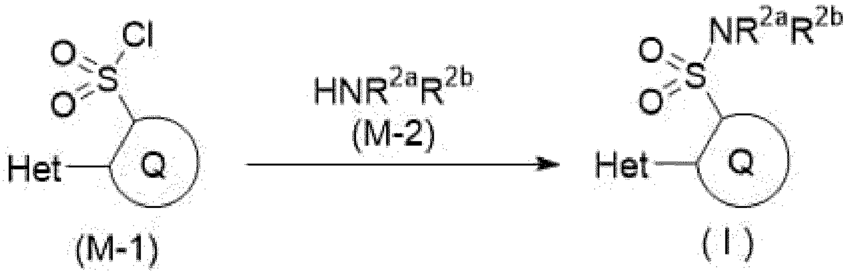


in which, in the formula, symbols represent the same meaning as described above.
The reaction is usually carried out in a solvent. Examples of the solvent include ethers such as tetrahydrofuran (hereinafter, referred to as THF), 1,4-dioxane, 1,2-dimethoxyethane (hereinafter, referred to as DME), methyl tert-butyl ether (hereinafter, referred to as MTBE), and diethyl ether (hereinafter, collectively referred to as ethers); halogenated hydrocarbons such as dichloromethane and chloroform (hereinafter, collectively referred to as halogenated hydrocarbons); aromatic hydrocarbons such as toluene and xylene (hereinafter, collectively referred to as aromatic hydrocarbons); nitriles such as acetonitrile (hereinafter, referred to as nitriles); water, and a mixture of two or more of them.
In the reaction, the compound (M-2) is usually used in a ratio of 0.8 to 10 mol relative to 1 mol of the compound (M-1).
The reaction can be carried out by mixing the compound (M-1) and the compound (M-2). The reaction may be carried out in the presence of a base if necessary. Examples of the base include alkali metal carbonates such as sodium

carbonate and potassium carbonate (hereinafter, collectively referred to as alkali metal carbonates); tertiary amines such as triethylamine (hereinafter, referred to as tertiary amines); and nitrogen-containing aromatic compounds such as pyridine (hereinafter, referred to as nitrogen-containing aromatic compounds). When a base is used in the reaction, the base is usually used in a ratio of 1 to 3 mol relative to 1 mol of the compound (M-1).

The reaction temperature is usually within a range of -20 to 200°C. The reaction time is usually within a range of 0.1 to 24 hour(s).

After completion of the reaction, water is added to the reaction mixture, then the mixture is extracted with an organic solvent, and the organic layer is subjected to post-treatment operations such as drying and concentration, whereby the Present compound N can be obtained.

The compound (M-2) is a commercially available compound, or can be produced using a known method.

Production Method 2

The compound represented by formula (I) (Present compound N) can be produced by reacting a compound represented by formula (M-3) (hereinafter, referred to as compound (M-3)) with a compound represented by formula (M-4) (hereinafter, referred to as compound (M-4)) in the presence of a base:

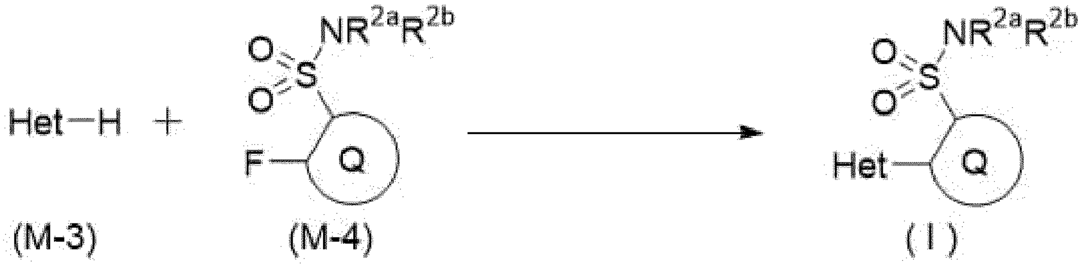

(M-3) (M-4) (I)

in which, in the formula, symbols represent the same meaning as described above.

The reaction is usually carried out in a solvent. Examples of the solvent include ethers; aromatic hydrocarbons; nitriles; aprotic polar solvents such as dimethylformamide (hereinafter, referred to as DMF), N-methylpyrrolidone (hereinafter, referred to as NMP), and dimethylsulfoxide (hereinafter, referred to as DMSO) (hereinafter, collectively referred to as aprotic polar solvents); water, and a mixture of two or more of them.

Examples of the base include alkali metal carbonates; and alkali metal hydrides such as sodium hydride (hereinafter, referred to as alkali metal hydrides).

In the reaction, the compound (M-4) is usually used in a ratio of 0.8 to 1.2 mol, and the base is usually used in a ratio of 1 to 3 mol, relative to 1 mol of the compound (M-3).

The reaction temperature is usually within a range of -20 to 200°C. The reaction time is usually within a range of 0.5 to 24 hour(s).

After completion of the reaction, water is added to the reaction mixture, then the mixture is extracted with an organic solvent, and the organic layer is subjected to post-treatment operations such as drying and concentration, whereby the Present compound N can be obtained.

The compound (M-3) is known, or can be produced, for example, according to the method described in Bioorganic & Medicinal Chemistry Letters 2016, 26, 3822-3825., Journal of Heterocyclic Chemistry 2003, 40, 677-679., Bioorg.Med.Chem.Lett. 2009, 19, 1773-1778., or Org.Process Res.Dev. 2021, 25, 858-870.

Production Method 3

The compound represented by formula (I) (Present compound N) can be produced by reacting the compound (M-3) with a compound represented by formula (M-5) (hereinafter, referred to as compound (M-5)) in the presence of a metal catalyst:

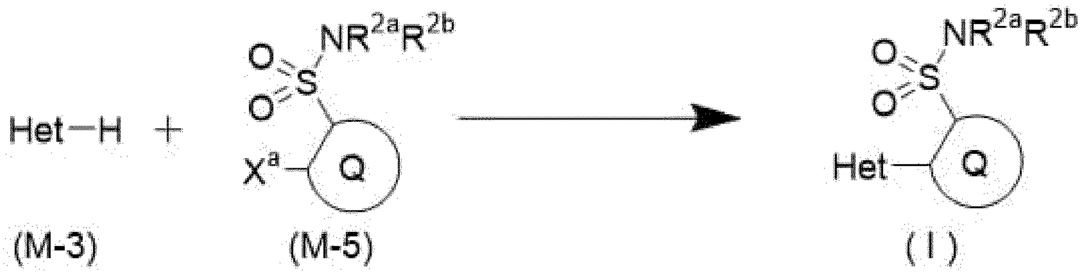

(M-3) (M-5) (I)

in which, in the formula, $X^a$ represents a chlorine atom, a bromine atom, or an iodine atom, and other symbols represent the same meaning as described above.

The reaction is usually carried out in a solvent. Examples of the solvent include ethers; aromatic hydrocarbons; nitriles; aprotic polar solvents; and a mixture of two or more thereof.

Examples of the metal catalyst include copper catalysts such as copper (I) iodide, copper (I) bromide, copper (I) chloride, copper (I) oxide, copper (I) trifluoromethanesulfonate benzene complex, tetrakis (acetonitrile) copper (I) hexafluorophosphate, and copper (I) 2-thiophenecarboxylate; nickel catalysts such as bis (cyclooctadiene) nickel (0) and nickel (II) chloride; palladium catalysts such as palladium (II) acetate, tetrakis (triphenylphosphine) palladium (0), and tris (dibenzylideneacetone) dipalladium (II).

In the reaction, the compound (M-5) is usually used in a ratio of 0.8 to 1.2 mol, and the metal catalyst is usually used in a ratio of 0.01 to 0.5 mol, relative to 1 mol of the compound (M-3).

In the reaction, a ligand may be used if necessary. Examples of the ligand include triphenylphosphine, 4,5-bis (diphenylphosphino)-9,9-dimethylxanthene (hereinafter, referred to as Xantphos), 2,2'-bis (diphenylphosphino)-1,1'-binaphthyl, 1,1'-bis (diphenylphosphino) ferrocene, 2-dicyclohexylphosphino-2',4',6'-triisopropylbiphenyl, 2-dicyclohexylphosphino-2',6'-dimethoxybiphenyl, 1,2-bis (diphenylphosphino) ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used in a ratio of 0.01 to 0.5 mol relative to 1 mol of the compound (M-1).

In the reaction, a base may be used if necessary. Examples of the base include organic bases such as tertiary amines and nitrogen-containing aromatic compounds (hereinafter, collectively referred to as organic bases); and alkali metal carbonates and alkali metal hydrides. When a base is used in the reaction, the base is usually used in a ratio of 1 to 3 mol relative to 1 mol of the compound (M-3).

The reaction temperature is usually in a range of - 20°C to 150°C. The reaction time is usually in a range of 0.5 to 24 hour(s).

After completion of the reaction, water is added to the reaction mixture, then the mixture is extracted with an organic solvent, and the organic layer is subjected to post-treatment operations such as drying and concentration, whereby the Present compound N can be obtained.

Reference Production Method 1

The compound (M-1) can be produced by reacting a compound represented by formula (M-7) (hereinafter, referred to as compound (M-7)) with a chlorinating agent in the presence of chlorosulfonic acid:

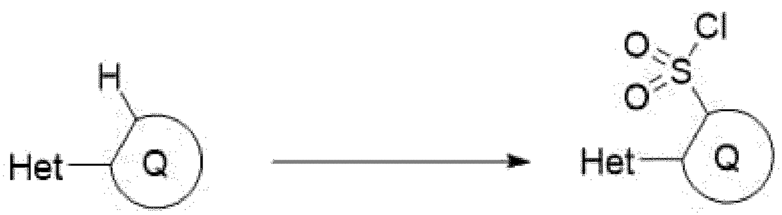

(M-7) (M-1)

in which, in the formula, symbols represent the same meaning as described above.

The reaction may be carried out in a solvent if necessary. Examples of the solvent used in the reaction include ethers; aromatic hydrocarbons; aromatic hydrocarbons, and a mixture of two or more of them.

Examples of the chlorinating agent include phosphorus oxychloride and thionyl chloride.

In the reaction, chlorosulfonic acid is usually used in a ratio of 1 to 10 mol, and the chlorinating agent is usually

used in a ratio of 1 to 10 mol, relative to 1 mol of the compound (M-7).

The reaction temperature is usually in a range of - 20°C to 200°C. The reaction time is usually in a range of 0.1 to 24 hour(s).

After completion of the reaction, water is added to the reaction mixture, then the mixture is extracted with an organic solvent, and the organic layer is subjected to post-treatment operations such as drying and concentration, whereby the compound (M-1) can be obtained.

The compound (M-7) is known, or can be produced, for example, in accordance with the method described in WO 2020/203763 A.

Reference Production Method 2

The compound represented by formula (M-9) (hereinafter, referred to as compound (M-9)) can be produced by reacting a compound represented by formula (M-8) (hereinafter, referred to as compound (M-8)) with a chlorinating agent in the presence of chlorosulfonic acid:

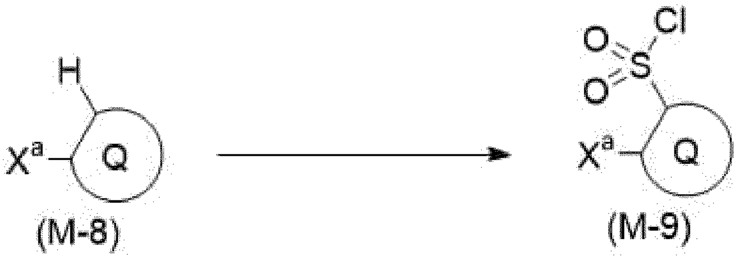

in which, in the formula, symbols represent the same meaning as described above.

The reaction can be carried out in accordance with the method described in Reference Production Method 1, using the compound (M-8) instead of the compound (M-7).

The compound (M-8) is a commercially available compound, or can be produced using a known method.

Reference Production Method 3

The compound (M-5) can be produced by reacting the compound (M-9) with the compound (M-2):

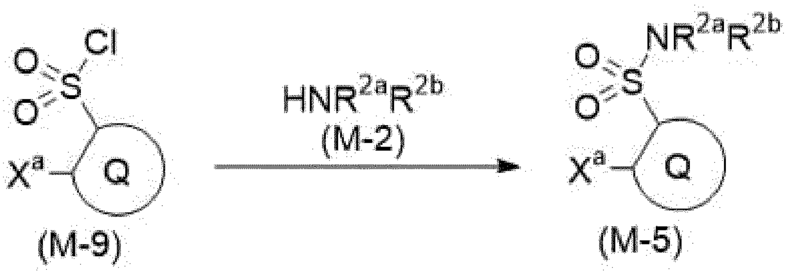


in which, in the formula, symbols represent the same meaning as described above.

The reaction can be carried out in accordance with the method described in Production Method 1, using the compound (M-9) instead of the compound (M-1).

Reference Production Method 4

The compound (M-4) can be produced by reacting the compound (M-5) with a fluorinating agent:

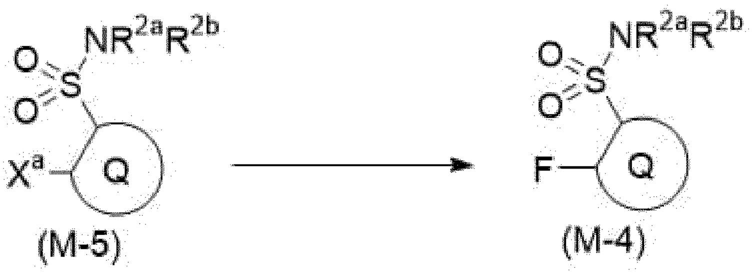

in which, in the formula, symbols represent the same meaning as described above.

The reaction is usually carried out in a solvent. Examples of the solvent include aromatic hydrocarbons; aprotic polar solvents; and a mixture of two or more thereof.

Examples of the fluorinating agent include cesium fluoride and potassium fluoride.

In the reaction, the fluorinating agent is usually used in a ratio of 1 to 10 mol relative to 1 mol of the compound (M-5).

The reaction temperature is usually in a range of 20°C to 300°C. The reaction time is usually in a range of 0.1 to 24 hour(s).

After completion of the reaction, water is added to the reaction mixture, then the mixture is extracted with an organic solvent, and the organic layer is subjected to post-treatment operations such as drying and concentration, whereby the compound (M-4) can be obtained.

The Present compound (including the Present compound N) may be mixed with or used in combination with one or more ingredient (s) selected from the group consisting of the following Group (a), Group (b), Group (c), and Group (d) (hereinafter referred to as "Present ingredient").

When the Present compound is mixed with or used in combination with the Present ingredient, they are used simultaneously, separately, or at time intervals with each other.

When the Present compound is used simultaneously with the Present ingredient, the Present compound and the Present ingredient may be contained in separate formulations or contained in one formulation.

One aspect of the present invention provides a composition comprising one or more ingredient (s) selected from the group consisting of Group (a), Group (b), Group (c), and Group (d), and the Present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides, and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Present compound (including the Present compound N) are described. For example, "alanycarb + SX" indicates a combination of alanycarb and SX.

The abbreviation of "SX" indicates any one of the Present compound (including the Present compound N) selected from the Compound groups SX1 to SX944 described in Examples. Also, all of the following Present ingredient are

known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Present compound (including the Present compound N):

abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin

+ SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl) phenyl]-4,5-dihydro-1,2-oxazol-3-yl}indan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl] phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis MNPV(multiple nucleocapsid nucleopolyhedrovirus) + SX, Autographa californica MNPV + SX, Cydia pomonella GV(granulovirus) strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis

+ SX.

Combination of the Present ingredient in the above Group (b) and the Present compound (including the Present compound N):

acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper (II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine

(1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl] ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl) butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl) carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, Agrobacterium radiobactor strain K1026 + SX, N-({4-[5-(trifluoromethyl)-1,2,4-oxadiazol-3-yl] phenyl}methyl)cyclopropanecarboxamide + SX, N-allyl-N-({4-[5-(trifluoromethyl)-1,2,4-oxadiazol-3-yl]phenyl}methyl) acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl) pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl] phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl) (2,6-difluoropyridin-4-yl)amino]-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, Agrobacterium radiobactor strain K84 + SX, Bacillus amyloliquefaciens strain PTA-4838 (Aveo(registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX,

Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Present compound (including the Present compound N) :

1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Present compound (including the Present compound N):

anthraquinone + SX, deet + SX, icaridin + SX.

The ratio of the Present compound (including the Present compound N) to the Present ingredient includes, but not limited thereto, as a ratio by weight (the Present compound : the Present ingredient) 1,000:1 to 1:1,000, 500:1 to 1:500, 100:1 to 1:100, 50:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, and 1:50, and the others.

The Present compound or the Present compound N has an efficacy against pests. Examples of the pests include plant phytopathogenic microorganism, harmful arthropods such as harmful insects and harmful mites, harmful nematicides, and harmful mollusks.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal leafhopper (Javesella pellucida), sugarcane leafhopper (Perkinsiella saccharicida), and Tagosodes orizicolus; from the family Cicadellidae, for example,
green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), and Amrasca biguttula biguttula;
from the family Aphrophoridae, for example, European spittlebug (Philaenus spumarius);
from the family Cercopidae, for example, Mahanarva posticata and Mahanarva fimbriolata;
from the family Aphididae, for example,
bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid(Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae);
from the family Phylloxeridae, for example,
grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);
from the family Adelgidae, for example, hemlock woolly aphid (Adelges tsugae), balsam woolly aphid (Adelges piceae), and Aphrastasia pectinatae;
from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug(Euschistus heros), red banded stink bug (Piezodorus guildinii), Oebalus pugnax, and Dichelops melacanthus;
from the family Cydnidae, for example, Scaptocoris castanea;
from the family Alydidae, for example, bean bug (Riptortus clavatus), corbett rice bug (Leptocorisa chinensis), and rice bug (Leptocorisa acuta);
from the family Coreidae, for example, Cletus punctiger and Australian leaf-footed bug (Leptoglossus australis);
from the family Lygaeidae, for example, oriental chinch bug (Cavelerius saccharivorus), seed bug (Togo hemipterus), and chinch bug (Blissus leucopterus);
from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);
from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;
from the family Diaspididae, for example, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), and citrus snow scale (Unaspis citri);
from the family Coccidae, for example, pink wax scale (Ceroplastes rubens);
from the family Margarodidae, for example, fluted scale (Icerya purchasi) and seychelles fluted scale (Icerya seychellarum);
from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);
from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);
from the family Tingidae, for example, sycamore lace bug (Corythucha ciliata), aster tingid (Corythucha marmorata), Japanese pear lace bug (Stephanitis nashi), and azalea lace bug (Stephanitis pyrioides);
from the family Cimicidae, for example, common bed bug (Cimex lectularius) and tropical bed bug (Cimex

hemipterus); from the family Cicadidae, for example, Quesada gigas; from the family Reduviidae, for example, Triatoma infestans, large kissing bug (Triatoma rubrofasciata), Triatoma dimidiata, and Rhodonius prolixus; and the others.

Lepidoptera:

from the family Crambidae, for example, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);
from the family Pyralidae, for example, lesser cornstalk borer (Elasmopalpus lignosellus), mealworm moth (Plodia interpunctella), persimmon bark borer (Euzophera batangensis), and fig moth (Cadra cautella);
from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), semitropical armyworm (Spodoptera eridania), black cutworm (Agrotis ipsilon), beet worm (Autographa nigrisigna), rice looper (Plusia festucae), Soybean looper (Chrysodeixis includens), Trichoplusia spp., Heliothis spp.(such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);
from the family Pieridae, for example, common cabbage worm (Pieris rapae);
from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);
from the family Gracillariidae, for example, tea leaf roller (Caloptilia theivora) and Asiatic apple leaf miner (Phyllonorycter ringoniella);
from the family Carposinidae, for example, peach fruit moth (Carposina sasakii);
from the family Lyonetiidae, for example, coffee leaf miner (Leucoptera coffeella), peach leaf miner (Lyonetia clerkella), and Lyonetia prunifoliella;
from the family Lymantriidae, for example, Lymantria spp. (such as gypsy moth (Lymantria dispar)) and Euproctis spp. (such as tea lymantriid (Euproctis pseudoconspersa));
from the family Plutellidae, for example, diamondback moth (Plutella xylostella);
from the family Gelechiidae, for example, peach worm (Anarsia lineatella), sweetpotato leaf folder (Helcystogramma triannulella), pink bollworm (Pectinophora gossypiella), potato moth (Phthorimaea operculella), and Tuta absoluta;
from the family Arctiidae, for example, American white moth (Hyphantria cunea);
from the family Castniidae, for example, giant sugarcane borer (Telchin licus);
from the family Cossidae, for example, Cossus insularis;
from the family Geometridae, for example, Ascotis selenaria;
from the family Limacodidae, for example, blue-striped nettle grub (Parasa lepida) ;
from the family Stathmopodidae, for example, persimmon fruit moth (Stathmopoda masinissa);
from the family Sphingidae, for example, tobacco hornworm (Acherontia lachesis);
from the family Sesiidae, for example, Nokona feralis, cherry borer (Synanthedon hector), and Synanthedon tenuis: from the family Hesperiidae, for example, rice skipper (Parnara guttata);
from the family Tineidae, for example, casemaking clothes moth (Tinea translucens), common clothes moth (Tineola bisselliella);
and the others.

Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella

intonsa), rice thrips (Stenchaetothrips biformis), Echinothrips americanus, and avocado thrips (Scirtothrips perseae);
from the family Phlaeothripidae, for example, aculeated rice thrips (Haplothrips aculeatus);
and the others.

Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);
from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis;
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus ,Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus;
from the family Muscidae, for example, house fly (Musca domestica), false stable fly (Muscina stabulans), biting house fly (Stomoxys calcitrans), and buffalo fly (Haematobia irritans);
from the family Calliphoridae;
from the family Sarcophagidae;
from the family Chironomidae, for example, Chironomus plumosus, Chironomus yoshimatsui, and Glyptotendipes tokunagai;
from the family Fannidae;
and the others.

Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle (Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa

decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, and tobacco flea beetle (Epitrix hirtipennis);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala rufocuprea), Anomala albopilosa, Japanese beetle (Popillia japonica), yellowish elongate chafer (Heptophylla picea), European Chafer(Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil (Araecerus coffeae);
from the family Aponidae, for example, sweet-potato weevil (Cylas formicarius);
from the family Bruchidae, for example, Mexican bean weevil (Zabrotes subfasciatus);
from the family Scolytidae, for example, pine beetle (Tomicus piniperda) and coffee berry borer (Hypothenemus hampei);
from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis);
from the family Tenebrionidae, for example, red meal beetle (Tribolium castaneum), mason beetle (Tribolium confusum), and lesser mealworm (Alphitobius diaperinus);
from the family Coccinellidae, for example, twenty-eight-spotted ladybird (Epilachna vigintioctopunctata);
from the family Bostrychidae, for example, common powder-post beetle (Lyctus brunneus), and lesser grain borer (Rhizopertha dominica);
from the family Ptinidae;
from the family Cerambycidae, for example, citrus long-horned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum) ;
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);
and the others.

Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust(Locustana pardalina), tree locust (Anacridium melanorhodon), Italian locust (Calliptamus italicus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust(Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma) ;
from the family Tettigoniidae, for example, mormon cricket (Anabrus simplex);
and the others.

Hymenoptera:

from the family Tenthredinidae, for example, beet sawfly (Athalia rosae) and nippon cabbage sawfly (Athalia japonica);
from the family Formicidae, for example, Solenopsis spp. (such as red imported fire ant (Solenopsis invicta), tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia, Vespa simillima, Vespa analis, Asian hornet(Vespa velutina), and Polistes jokahamae;
from the family Siricidae, for example, pine wood wasp (Urocerus gigas);
from the family Bethylidae;
and the others.

Blattodea:

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;
and the others.

Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);
from the family Ceratophyllidae, for example, European rat flea (Nosopsyllus fasciatus); and the others.

Psocodae:

from the family Pediculidae, for example, head louse (Pediculus humanus capitis);
from the family Pthiridae, for example, crab louse (Pthirus pubis) ;
from the family Haematopinidae, for example, short-nosed cattle louse (Haematopinus eurysternus) and pig louse (Haematopinus suis);
from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp. ;
from the family Trichodectidae, for example, dog biting louse (Trichodectes canis) and cat louse (Felicola subrostratus);
from the family Menoponidae, for example, common chicken louse (Menopon gallinae), chicken body louse (Menacanthus stramineus), and Trinoton spp;
from the family Trimenoponidae, for example, Cummingsia spp.;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani and Liposcelis entomophila;
and the other.

Thysanura:

from the family Lepismatidae, for example, oriental silverfish (Ctenolepisma villosa) andmoth fish (Lepisma saccharina);
and the others.

Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus; from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example,bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;
and the others.

Araneae:

from the family Eutichuridae, for example, Cheiracanthium japonicum;
from the family Theridiidae, for example, red-back spider (Latrodectus hasseltii);
and the others

Polydesmida:

from the family Paradoxosomatidae, for example, flat-backed millipede (Oxidus gracilis) and Nedyopus tambanus;
and the others.

Isopoda:

from the family Armadillidiidae, common pill bug (Armadillidium vulgare);
and the others.

Chilopoda:

from the family Scutigeridae, for example, Thereuonema hilgendorfi;
from the family Scolopendridae, giant tropical centipede (Scolopendra subspinipes);
from the family Ethopolyidae, Bothropolys rugosus;
and the others.

Gastropoda:

from the family Limacidae, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;
and the others.

Nematoda:

from the family Aphelenchoididae, for example, rice white-tip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), and white potato cyst nematode (Globodera pallida);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);
and the others.

The harmful arthropods such as harmful insects and harmful mites, harmful mollusks and harmful nematodes may be those having a reduced susceptibility to or a developed resistance to an insecticide, a mitecide, a molluscicide or a nematicide.

The method for controlling harmful arthropods of the present invention is carried out by applying an effective amount of the Present compound or the Composition A to a harmful arthropod directly and/or a habitat thereof (for example, plant bodies, soil, an interior of a house, animal bodies). Examples of the method for controlling harmful arthropods of the present invention include foliage treatment, soil treatment, root treatment, shower treatment, smoking treatment, water surface treatment and seed treatment.

The Present compound or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate,

ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the Present compound. The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), N092P-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark) .

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling harmful arthropods by applying an effective amount of the Present compound or the Composition A to soils include a method of applying an effective amount of the Present compound or the Composition A to soils before planting plants or after planting plants, a method of applying an effective amount of the Present compound or the Composition A to a root part of a crop to be protected from damage such as ingestion by harmful arthropods, and a method of controlling harmful arthropods that ingest a plant by permeating and transferring an effective amount of the Present compound or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before

sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated a surface of seeds or the vegetative reproductive organ; a soaking treatment in which the seeds are soaked into the solution of the Present compound or the Composition A for a certain time; and a method for coating the seeds or the vegetative reproductive organ with a carrier containing the Present compound or the Composition A (film coating treatment, pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the present compound and the Present ingredients are applied, and seeds or vegetative reproductive organs are air dried, followed by applying the formulations comprising the Present ingredients other than the already-applied Present ingredients, are included.

As used herein, seeds or vegetative reproductive organs carrying the present compound or the Composition A means seeds or vegetative reproductive organs in the state where the present compound or the Composition A is adhered to a surface of the seeds or the vegetative reproductive organ. The above-described seeds or vegetative reproductive organs carrying the present compound or the Composition A may be adhered by any other materials that are different from the present compound or the Composition A before or after being adhered the present compound or the Composition A to the seeds or vegetative reproductive organs.

Also, when the Composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound or the Composition A can be obtained, for example, by applying the formulations comprising the present compound or the Composition A by the above-described application method to seeds to seeds or vegetative reproductive organs.

When the Present compound or the Composition A is applied for harmful arthropods control in agricultural fields, the application dose thereof is usually within a range of 1 to 10,000 g of the Present compound per 10,000 $m^2$. In the case of being applied to seeds or vegetative reproductive organs, the dose of application dose thereof is usually within a range of 0.001 to 100 g of the Present compound per 1 Kg of seeds. When the Present compound or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the Present compound or the Composition A is used to control harmful arthropods that live inside a house, the application dose as an amount of the Present compound is usually within a range from 0.01 to 1,000 mg per 1 $m^2$ of an area to be treated, in the case of using it on a planar area. In the case of using it spatially, the application dose as an amount of the Present compound is usually within a range from 0.01 to 500 mg per 1 $m^3$ of the space to be treated. When the Present compound or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the Present compound or the composition is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On

the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the compound of the Present compound is usually within a range from 0.1 to 1,000 mg per 1 kg of an animal body weight.

Also, the composition of the Present compound or the Composition A may be used as an agent for controlling harmful arthropods in agricultural lands such as paddy fields, fields, turfs, and orchards. Examples of the plants include the followings:

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

## EXAMPLES

Hereinafter, the present invention will be described in more detail with reference to production examples, formulation examples, test examples, and the like, but the present invention is not limited to these examples.

In the present specification, Me represents a methyl group, Et represents an ethyl group, Pr represents a propyl group, i-Pr represents an isopropyl group, Bu represents a butyl group, t-Bu represents a tert-butyl group, Boc represents a tert-butoxycarbonyl group, Hex represents a hexyl group, c-Pr represents a cyclopropyl group, c-Bu represents a cyclobutyl group, Ph represents a phenyl group, Py2 represents a 2-pyridyl group, Py3 represents a 3-pyridyl group, and Py4 represents a 4-pyridyl group. When c-Pr, c-Bu, Ph, Py2, Py3, and Py4 are substituted with a substituent, the substituent is described together with the substitution position before the symbol. For example, 1-CN-c-Pr represents a 1-cyanocyclopropyl group, 3,4-$F_2$-Ph represents a 3,4-difluorophenyl group, and 4-$CF_3$-Py2 represents a 4-(trifluoromethyl)-2-pyridyl group.

First, production examples of the Present compound and production intermediates thereof will be shown.

When the physical property value of the compound is measured by liquid chromatography/mass spectrometry (hereinafter, referred to as LCMS), the measured molecular ion value $[M+H]^+$ or $[M-H]^-$ and retention time (hereinafter, referred to as RT) are described. The conditions of liquid chromatography (hereinafter, referred to as LC) are as follows.

[LC Conditions]

Column: L-column 2 ODS, inner diameter: 4.6 mm, length: 30 mm, particle diameter: 3 μm (Chemicals Evaluation and Research Institute, Japan)
UV measurement wavelength: 254 nm
Mobile phase: A solution: 0.1% formic acid aqueous solution, B solution: 0.1% formic acid acetonitrile Flow rate: 2.0 mL/min
Gradient conditions: Feeding is performed at the concentration gradient described in Table LC1.

[Table LC1]

| Time (min) | A solution (%) | B solution (%) |
|---|---|---|
| 0.01 | 90 | 10 |
| 2.00 | 0 | 100 |
| 4.00 | 0 | 100 |
| 4.01 | 90 | 10 |

[MS Conditions]

Detector: LCMS-2020 (manufactured by Shimadzu Corporation)
Ionization method: DUIS method

Reference Production Example 1

To a mixture of 5.9 g of ethyl 3-(trifluoromethyl)-1H-pyrazole-5-carboxylate and 70 mL of THF, 9.0 g of triphenylphosphine, 5.3 mL of 2-(tert-butoxycarbonylamino)-1-ethanol, and 10 g of bis (2-methoxyethyl) azodicarboxylate were added at 0°C, and the mixture was stirred at room temperature for 6 hours. Water was added to the resulting mixture, and the mixture was extracted with ethyl acetate. The resulting organic layer was dried with sodium sulfate and concentrated under reduced pressure. The resulting residue was applied to silica gel column chromatography to obtain 7.8 g of the Intermediate 1 represented by the following formula.

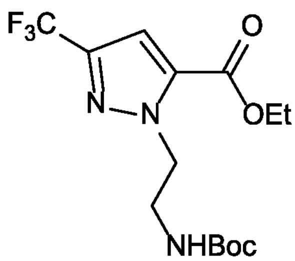

Intermediate 1: $^1$H-NMR ($CDCl_3$) δ: 7.09 (1H, s), 4.79 (1H, br s), 4.74-4.71 (2H, m), 4.37 (2H, q), 3.63 (2H, q), 1.40-1.37 (12 H, m).

Reference Production Example 2

To a mixture of 0.51 g of the Intermediate 1 and 5 mL of cyclopentyl methyl ether, 6 mL of a hydrogen chloride solution (about 4 mol/L of cyclopentyl methyl ether solution) was added at 0°C, and the mixture was stirred at room temperature for 4 hours, and the resulting mixture was concentrated under reduced pressure. A saturated aqueous sodium carbonate solution was added to the obtained residue, the mixture was stirred at room temperature for 3 hours, and extracted with chloroform containing 25% of 2-propanol. The resulting organic layer was dried with sodium sulfate and concentrated under reduced pressure. The resulting residue was applied to silica gel column chromatography to obtain

0.26 g of the Intermediate 2 represented by the following formula.

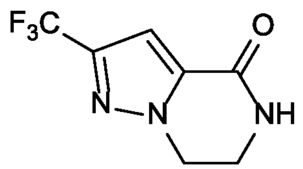

Intermediate 2: $^1$H-NMR ($CDCl_3$) $\delta$: 7.12 (1H, s), 6.16 (1H, br s), 4.48 (2H, t), 3.87-3.83 (2H, m).

Production Example 1

To a mixture of 0.1 g of 6-bromo-3,4-dihydroisoquinoline-1(2H)-one, 0.09 g of 2-fluoro-N,N-dimethylpyridine-3-sulfonamide, and 4 mL of dimethylformamide, 0.040 g of sodium hydride (Oil, 60%) was added at 0°C under a nitrogen atmosphere, and the mixture was stirred at 0°C for 4 hours. Water was added to the resulting mixture, and the mixture was extracted with ethyl acetate. The resulting organic layer was dried with magnesium sulfate and concentrated under reduced pressure. The resulting residue was applied to silica gel column chromatography to obtain 0.05 g of the Present compound 1 represented by the following formula.

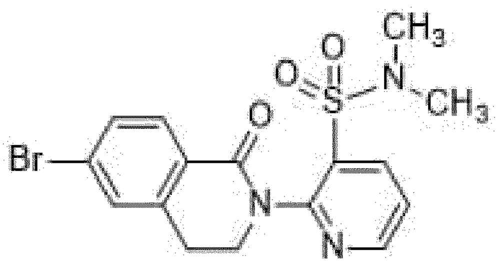

Present compound 1: LCMS: 410 $[M+H]^+$, RT = 1.64 min

Production Example 2

The compounds produced in accordance with Production Example 1 and the physical properties thereof are shown below.

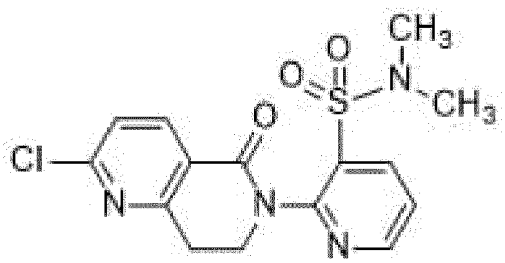

Present compound 2: $^1$H-NMR ($CDCl_3$) $\delta$: 8.79 (1H, dd), 8.43 (1H, dd), 8.32 (1H, d), 7.55 (1H, dd), 7.37 (1H, d), 4.12-4.20 (1H, m), 3.90-3.96 (1H, m), 3.68-3.77 (1H, m), 3.20-3.24 (1H, m), 2.76 (6H, s).

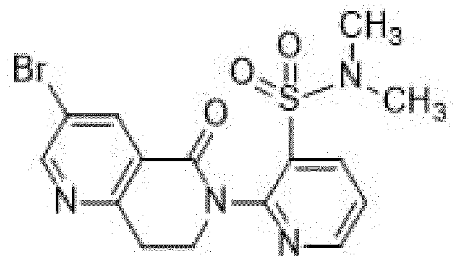

Present compound 3: $^1$H-NMR ($CDCl_3$) δ: 8.79 (1H, dd), 8.75 (1H, d), 8.49 (1H, d), 8.43 (1H, dd), 7.55 (1H, dd), 4.12-4.19 (1H, m), 3.92-3.97 (1H, m), 3.63-3.72 (1H, m), 3.19-3.25 (1H, m), 2.77 (6H, s).

Production Example 3

To a mixture of 0.3 g of the Intermediate 2, 0.3 g of 2-fluoro-N,N-dimethylpyridine-3-sulfonamide, and 5 mL of dimethyl sulfoxide, 0.95 g of cesium carbonate was added, and the mixture was stirred at 40°C for 10 hours. The resulting mixture was allowed to cool to room temperature, water was added thereto, and the precipitated solid was filtered off to obtain 0.38 g of the Present compound 4 represented by the following formula.

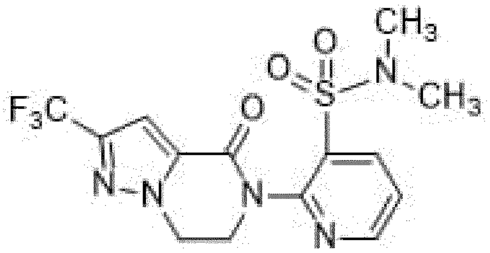

Present compound 4: $^1$H-NMR ($CDCl_3$) δ: 8.80 (1H, dd), 8.42 (1H, dd), 7.59 (1H, dd), 7.18 (1H, s), 4.79-4.87 (1H, m), 4.61-4.65 (1H, m), 4.34-4.41 (1H, m), 4.00-4.05 (1H, m), 2.78 (6H, s).

Next, examples of the Present compound produced according to any of the production examples described in the examples and the production methods described in the present specification are shown below.

A compound represented by formula (L-1):

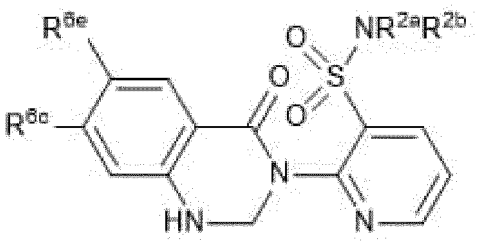

(L-1)

(hereinafter, referred to as compound (L-1)) in which $R^{2a}$ and $R^{2b}$ are hydrogen atoms, $R^{6c}$ is a hydrogen atom, and $R^{6e}$ is any of the substituents shown in Table 1A to Table 3A (hereinafter, referred to as compound group SX1).

| [Table 1A] |
|---|
| $CF_3$ |
| $CHF_2$ |
| $CH_2CF_3$ |
| $CF_2CF_3$ |
| $CH_2CF_2CF_3$ |
| $CF_2CF_2CF_3$ |
| $CF_2CF_2CF_2CF_3$ |
| $CF_2CF_2CF_2CF_2CF_3$ |
| $OCF_3$ |
| $OCHF_2$ |
| $OCH_2CF_3$ |
| $OCH_2CHF_2$ |
| $OCF_2CF_3$ |
| $OCH(CH_3)CF_3$ |
| $OCH_2CF_2CHF_2$ |
| $OCH_2CF_2CF_3$ |
| $OCF_2CF_2CF_3$ |
| $OCH_2CF_2CHFCF_3$ |
| $OCH_2CF_2CF_2CF_3$ |
| $OCF_2CF_2CF_2CF_3$ |
| $OCH_2CF_2CF_2CF_2CF_3$ |

| [Table 2A] |
|---|
| $SCF_3$ |
| $SCH_2CF_3$ |
| $SCF_2CF_3$ |
| $SCH_2CF_2CF_3$ |
| $SCF_2CF_2CF_3$ |
| $SCH_2CF_2CF_2CF_3$ |
| $SCF_2CF_2CF_2CF_3$ |
| $S(0)CF_3$ |
| $S(0)CH_2CF_3$ |
| $S(0)CF_2CF_3$ |
| $S(0)CH_2CF_2CF_3$ |
| $S(0)CF_2CF_2CF_3$ |
| $S(0)CH_2CF_2CF_2CF_3$ |
| $S(0)CF_2CF_2CF_2CF_3$ |
| $S(0)_2CF_3$ |
| $S(0)_2CH_2CF_3$ |
| $S(0)_2CF_2CF_3$ |
| $S(0)_2CH_2CF_2CF_3$ |
| $S(0)_2CF_2CF_2CF_3$ |
| $S(0)_2CH_2CF_2CF_2CF_3$ |
| $S(0)_2CF_2CF_2CF_2CF_3$ |

| [Table 3A] |
|---|
| F |
| Cl |
| Br |
| I |
| Me |
| Et |
| Pr |
| i-Pr |
| Bu |
| Hex |
| $CH=CH_2$ |
| $CMe=CH_2$ |
| 1-F-c-Pr |
| $2,2-F_2-c-Pr$ |
| c-Pr |
| c-Bu |
| 1-CN-c-Pr |
| 1-CN-c-Bu |

| |
|---|
| $OCH_2C(Me)_2CN$ |
| $OCH_2(1-CN-c-Pr)$ |
| $OCH_2(2,2-F_2-c-Pr)$ |
| $OCH_2(l-CF_3-c-Pr)$ |
| $OS(0)_2CF_3$ |
| $OS(0)_2CF_2CF_3$ |
| $OS(0)_2CF_2CF_2CF_3$ |

| |
|---|
| $OS(0)_2CF_3$ |
| $OS(0)_2CH_2CF_3$ |
| $OS(0)_2CF_2CF_3$ |
| $OS(0)_2CH_2CF_2CF_3$ |
| $OS(0)_2CF_2CF_2CF_3$ |
| $OS(0)_2CH_2CF_2CF_2CF_3$ |
| $OS(0)_2CF_2CF_2CF_2CF_3$ |

A compound (L-1) in which $R^{2a}$ is a methyl group, $R^{2b}$ is a hydrogen atom, $R^{6c}$ is a hydrogen atom, and $R^{6e}$ is any of the substituents shown in Table 1A to Table 3A (hereinafter, referred to as compound group SX2).

A compound (L-1) in which $R^{2a}$ is a methyl group, $R^{2b}$ is a methyl group, $R^{6c}$ is a hydrogen atom, and $R^{6e}$ is any of the substituents shown in Table 1A to Table 3A (hereinafter, referred to as compound group SX3).

A compound (L-1) in which $R^{2a}$ is an ethyl group, $R^{2b}$ is a hydrogen atom, $R^{6c}$ is a hydrogen atom, and $R^{6e}$ is any of the substituents shown in Table 1A to Table 3A (hereinafter, referred to as compound group SX4).

A compound (L-1) in which $R^{2a}$ and $R^{2b}$ are ethyl groups, $R^{6c}$ is a hydrogen atom, and $R^{6e}$ is any of the substituents shown in Table 1A to Table 3A (hereinafter, referred to as compound group SX5).

A compound (L-1) in which $R^{2a}$ is a propyl group, $R^{2b}$ is a hydrogen atom, $R^{6c}$ is a hydrogen atom, and $R^{6e}$ is any of the substituents shown in Table 1A to Table 3A (hereinafter, referred to as compound group SX6).

A compound (L-1) in which $R^{2a}$ and $R^{2b}$ are propyl groups, $R^{6c}$ is a hydrogen atom, and $R^{6e}$ is any of the substituents shown in Table 1A to Table 3A (hereinafter, referred to as compound group SX7).

A compound (L-1) in which $R^{2a}$ is a methyl group, $R^{2b}$ is an ethyl group, $R^{6c}$ is a hydrogen atom, and $R^{6e}$ is any of the substituents shown in Table 1A to Table 3A (hereinafter, referred to as compound group SX8).

A compound (L-1) in which $R^{2a}$ and $R^{2b}$ are hydrogen atoms, $R^{6c}$ is a fluorine atom, and $R^{6e}$ is any of the substituents shown in Table 1A to Table 3A (hereinafter, referred to as compound group SX9).

A compound (L-1) in which $R^{2a}$ is a methyl group, $R^{2b}$ is a hydrogen atom, $R^{6c}$ is a fluorine atom, and $R^{6e}$ is any of the substituents shown in Table 1A to Table 3A (hereinafter, referred to as compound group SX10).

A compound (L-1) in which $R^{2a}$ and $R^{2b}$ are methyl groups, $R^{6c}$ is a fluorine atom, and $R^{6e}$ is any of the substituents shown in Table 1A to Table 3A (hereinafter, referred to as compound group SX11).

A compound (L-1) in which $R^{2a}$ is an ethyl group, $R^{2b}$ is a hydrogen atom, $R^{6c}$ is a fluorine atom, and $R^{6e}$ is any of the substituents shown in Table 1A to Table 3A (hereinafter, referred to as compound group SX12).

A compound (L-1) in which $R^{2a}$ and $R^{2b}$ are ethyl groups, $R^{6c}$ is a fluorine atom, and $R^{6e}$ is any of the substituents shown in Table 1A to Table 3A (hereinafter, referred to as compound group SX13).

A compound (L-1) in which $R^{2a}$ is a propyl group, $R^{2b}$ is a hydrogen atom, $R^{6c}$ is a fluorine atom, and $R^{6e}$ is any of the substituents shown in Table 1A to Table 3A (hereinafter, referred to as compound group SX14).

A compound (L-1) in which $R^{2a}$ and $R^{2b}$ are propyl groups, $R^{6c}$ is a fluorine atom, and $R^{6e}$ is any of the substituents shown in Table 1A to Table 3A (hereinafter, referred to as compound group SX15).

A compound (L-1) in which $R^{2a}$ is a methyl group, $R^{2b}$ is an ethyl group, $R^{6c}$ is a fluorine atom, and $R^{6e}$ is any of the substituents shown in Table 1A to Table 3A (hereinafter, referred to as compound group SX16).

A compound (L-1) in which $R^{2a}$ and $R^{2b}$ are hydrogen atoms, $R^{6c}$ is a chlorine atom, and $R^{6e}$ is any of the substituents shown in Table 1A to Table 3A (hereinafter, referred to as compound group SX17).

A compound (L-1) in which $R^{2a}$ is a methyl group, $R^{2b}$ is a hydrogen atom, $R^{6c}$ is a chlorine atom, and $R^{6e}$ is any of the substituents shown in Table 1A to Table 3A (hereinafter, referred to as compound group SX18).

A compound (L-1) in which $R^{2a}$ and $R^{2b}$ are methyl groups, $R^{6c}$ is a chlorine atom, and $R^{6e}$ is any of the substituents shown in Table 1A to Table 3A (hereinafter, referred to as compound group SX19).

A compound (L-1) in which $R^{2a}$ is an ethyl group, $R^{2b}$ is a hydrogen atom, $R^{6c}$ is a chlorine atom, and $R^{6e}$ is any of the substituents shown in Table 1A to Table 3A (hereinafter, referred to as compound group SX20).

A compound (L-1) in which $R^{2a}$ and $R^{2b}$ are ethyl groups, $R^{6c}$ is a chlorine atom, and $R^{6e}$ is any of the substituents shown in Table 1A to Table 3A (hereinafter, referred to as compound group SX21).

A compound (L-1) in which $R^{2a}$ is a propyl group, $R^{2b}$ is a hydrogen atom, $R^{6c}$ is a chlorine atom, and $R^{6e}$ is any of the substituents shown in Table 1A to Table 3A (hereinafter, referred to as compound group SX22).

A compound (L-1) in which $R^{2a}$ and $R^{2b}$ are propyl groups, $R^{6c}$ is a chlorine atom, and $R^{6e}$ is any of the substituents shown in Table 1A to Table 3A (hereinafter, referred to as compound group SX23).

A compound (L-1) in which $R^{2a}$ is a methyl group, $R^{2b}$ is an ethyl group, $R^{6c}$ is a chlorine atom, and $R^{6e}$ is any of the substituents shown in Table 1A to Table 3A (hereinafter, referred to as compound group SX24).

A compound (L-1) in which $R^{2a}$ and $R^{2b}$ are hydrogen atoms, $R^{6c}$ is a bromine atom, and $R^{6e}$ is any of the substituents shown in Table 1A to Table 3A (hereinafter, referred to as compound group SX25).

A compound (L-1) in which $R^{2a}$ is a methyl group, $R^{2b}$ is a hydrogen atom, $R^{6c}$ is a bromine atom, and $R^{6e}$ is any of the substituents shown in Table 1A to Table 3A (hereinafter, referred to as compound group SX26).

A compound (L-1) in which $R^{2a}$ and $R^{2b}$ are methyl groups, $R^{6c}$ is a bromine atom, and $R^{6e}$ is any of the substituents shown in Table 1A to Table 3A (hereinafter, referred to as compound group SX27).

A compound (L-1) in which $R^{2a}$ is an ethyl group, $R^{2b}$ is a hydrogen atom, $R^{6c}$ is a bromine atom, and $R^{6e}$ is any of the substituents shown in Table 1A to Table 3A (hereinafter, referred to as compound group SX28).

A compound (L-1) in which $R^{2a}$ and $R^{2b}$ are ethyl groups, $R^{6c}$ is a bromine atom, and $R^{6e}$ is any of the substituents shown in Table 1A to Table 3A (hereinafter, referred to as compound group SX29).

A compound (L-1) in which $R^{2a}$ is a propyl group, $R^{2b}$ is a hydrogen atom, $R^{6c}$ is a bromine atom, and $R^{6e}$ is any of the substituents shown in Table 1A to Table 3A (hereinafter, referred to as compound group SX30).

A compound (L-1) in which $R^{2a}$ and $R^{2b}$ are propyl groups, $R^{6c}$ is a bromine atom, and $R^{6e}$ is any of the substituents shown in Table 1A to Table 3A (hereinafter, referred to as compound group SX31).

A compound (L-1) in which $R^{2a}$ is a methyl group, $R^{2b}$ is an ethyl group, $R^{6c}$ is a bromine atom, and $R^{6e}$ is any of the substituents shown in Table 1A to Table 3A (hereinafter, referred to as compound group SX32).

A compound (L-1) in which $R^{2a}$ and $R^{2b}$ are hydrogen atoms, $R^{6c}$ is an iodine atom, and $R^{6e}$ is any of the substituents shown in Table 1A to Table 3A (hereinafter, referred to as compound group SX33).

A compound (L-1) in which $R^{2a}$ is a methyl group, $R^{2b}$ is a hydrogen atom, $R^{6c}$ is an iodine atom, and $R^{6e}$ is any of the substituents shown in Table 1A to Table 3A (hereinafter, referred to as compound group SX34).

A compound (L-1) in which $R^{2a}$ and $R^{2b}$ are methyl groups, $R^{6c}$ is an iodine atom, and $R^{6e}$ is any of the substituents shown in Table 1A to Table 3A (hereinafter, referred to as compound group SX35).

A compound (L-1) in which $R^{2a}$ is an ethyl group, $R^{2b}$ is a hydrogen atom, $R^{6c}$ is an iodine atom, and $R^{6e}$ is any of the substituents shown in Table 1A to Table 3A (hereinafter, referred to as compound group SX36).

A compound (L-1) in which $R^{2a}$ and $R^{2b}$ are ethyl groups, $R^{6c}$ is an iodine atom, and $R^{6e}$ is any of the substituents shown in Table 1A to Table 3A (hereinafter, referred to as compound group SX37).

A compound (L-1) in which $R^{2a}$ is a propyl group, $R^{2b}$ is a hydrogen atom, $R^{6c}$ is an iodine atom, and $R^{6e}$ is any of the substituents shown in Table 1A to Table 3A (hereinafter, referred to as compound group SX38).

A compound (L-1) in which $R^{2a}$ and $R^{2b}$ are propyl groups, $R^{6c}$ is an iodine atom, and $R^{6e}$ is any of the substituents shown in Table 1A to Table 3A (hereinafter, referred to as compound group SX39).

A compound (L-1) in which $R^{2a}$ is a methyl group, $R^{2b}$ is an ethyl group, $R^{6c}$ is an iodine atom, and $R^{6e}$ is any of the substituents shown in Table 1A to Table 3A (hereinafter, referred to as compound group SX40).

A compound represented by formula (L-2):

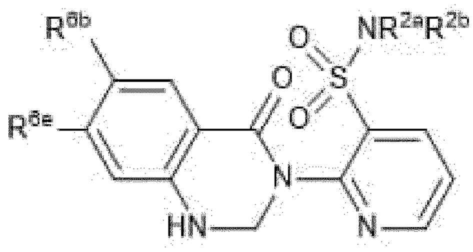

(L-2)

(hereinafter, referred to as compound (L-2)) in which $R^{2a}$ and $R^{2b}$ are hydrogen atoms, $R^{6b}$ is a hydrogen atom, and $R^{6e}$ is any of the substituents shown in Table 1A to Table 3A (hereinafter, referred to as compound group SX41).

A compound (L-2) in which $R^{2a}$ is a methyl group, $R^{2b}$ is a hydrogen atom, $R^{6b}$ is a hydrogen atom, and $R^{6e}$ is any of the substituents shown in Table 1A to Table 3A (hereinafter, referred to as compound group SX42).

A compound (L-2) in which $R^{2a}$ is a methyl group, $R^{2b}$ is a methyl group, $R^{6b}$ is a hydrogen atom, and $R^{6e}$ is any of the substituents shown in Table 1A to Table 3A (hereinafter, referred to as compound group SX43).

A compound (L-2) in which $R^{2a}$ is an ethyl group, $R^{2b}$ is a hydrogen atom, $R^{6b}$ is a hydrogen atom, and $R^{6e}$ is any of the substituents shown in Table 1A to Table 3A (hereinafter, referred to as compound group SX44).

A compound (L-2) in which $R^{2a}$ and $R^{2b}$ are ethyl groups, $R^{6b}$ is a hydrogen atom, and $R^{6e}$ is any of the substituents shown in Table 1A to Table 3A (hereinafter, referred to as compound group SX45).

A compound (L-2) in which $R^{2a}$ is a propyl group, $R^{2b}$ is a hydrogen atom, $R^{6b}$ is a hydrogen atom, and $R^{6e}$ is any of the substituents shown in Table 1A to Table 3A (hereinafter, referred to as compound group SX46).

A compound (L-2) in which $R^{2a}$ and $R^{2b}$ are propyl groups, $R^{6b}$ is a hydrogen atom, and $R^{6e}$ is any of the substituents shown in Table 1A to Table 3A (hereinafter, referred to as compound group SX47).

A compound (L-2) in which $R^{2a}$ is a methyl group, $R^{2b}$ is an ethyl group, $R^{6b}$ is a hydrogen atom, and $R^{6e}$ is any of the substituents shown in Table 1A to Table 3A (hereinafter, referred to as compound group SX48).

A compound (L-2) in which $R^{2a}$ and $R^{2b}$ are hydrogen atoms, $R^{6b}$ is a fluorine atom, and $R^{6e}$ is any of the substituents shown in Table 1A to Table 3A (hereinafter, referred to as compound group SX49).

A compound (L-2) in which $R^{2a}$ is a methyl group, $R^{2b}$ is a hydrogen atom, $R^{6b}$ is a fluorine atom, and $R^{6e}$ is any of the substituents shown in Table 1A to Table 3A (hereinafter, referred to as compound group SX50).

A compound (L-2) in which $R^{2a}$ and $R^{2b}$ are methyl groups, $R^{6b}$ is a fluorine atom, and $R^{6e}$ is any of the substituents shown in Table 1A to Table 3A (hereinafter, referred to as compound group SX51).

A compound (L-2) in which $R^{2a}$ is an ethyl group, $R^{2b}$ is a hydrogen atom, $R^{6b}$ is a fluorine atom, and $R^{6e}$ is any of the substituents shown in Table 1A to Table 3A (hereinafter, referred to as compound group SX52).

A compound (L-2) in which $R^{2a}$ and $R^{2b}$ are ethyl groups, $R^{6b}$ is a fluorine atom, and $R^{6e}$ is any of the substituents shown in Table 1A to Table 3A (hereinafter, referred to as compound group SX53).

A compound (L-2) in which $R^{2a}$ is a propyl group, $R^{2b}$ is a hydrogen atom, $R^{6b}$ is a fluorine atom, and $R^{6e}$ is any of the substituents shown in Table 1A to Table 3A (hereinafter, referred to as compound group SX54).

A compound (L-2) in which $R^{2a}$ and $R^{2b}$ are propyl groups, $R^{6b}$ is a fluorine atom, and $R^{6e}$ is any of the substituents shown in Table 1A to Table 3A (hereinafter, referred to as compound group SX55).

A compound (L-2) in which $R^{2a}$ is a methyl group, $R^{2b}$ is an ethyl group, $R^{6b}$ is a fluorine atom, and $R^{6e}$ is any of the substituents shown in Table 1A to Table 3A (hereinafter, referred to as compound group SX56).

A compound (L-2) in which $R^{2a}$ and $R^{2b}$ are hydrogen atoms, $R^{6b}$ is a chlorine atom, and $R^{6e}$ is any of the substituents shown in Table 1A to Table 3A (hereinafter, referred to as compound group SX57).

A compound (L-2) in which $R^{2a}$ is a methyl group, $R^{2b}$ is a hydrogen atom, $R^{6b}$ is a chlorine atom, and $R^{6e}$ is any of the substituents shown in Table 1A to Table 3A (hereinafter, referred to as compound group SX58).

A compound (L-2) in which $R^{2a}$ and $R^{2b}$ are methyl groups, $R^{6b}$ is a chlorine atom, and $R^{6e}$ is any of the substituents shown in Table 1A to Table 3A (hereinafter, referred to as compound group SX59).

A compound (L-2) in which $R^{2a}$ is an ethyl group, $R^{2b}$ is a hydrogen atom, $R^{6b}$ is a chlorine atom, and $R^{6e}$ is any of the substituents shown in Table 1A to Table 3A (hereinafter, referred to as compound group SX60).

A compound (L-2) in which $R^{2a}$ and $R^{2b}$ are ethyl groups, $R^{6b}$ is a chlorine atom, and $R^{6e}$ is any of the substituents shown in Table 1A to Table 3A (hereinafter, referred to as compound group SX61).

A compound (L-2) in which $R^{2a}$ is a propyl group, $R^{2b}$ is a hydrogen atom, $R^{6b}$ is a chlorine atom, and $R^{6e}$ is any of the substituents shown in Table 1A to Table 3A (hereinafter, referred to as compound group SX62).

A compound (L-2) in which $R^{2a}$ and $R^{2b}$ are propyl groups, $R^{6b}$ is a chlorine atom, and $R^{6e}$ is any of the

substituents shown in Table 1A to Table 3A (hereinafter, referred to as compound group SX63).

A compound (L-2) in which $R^{2a}$ is a methyl group, $R^{2b}$ is an ethyl group, $R^{6b}$ is a chlorine atom, and $R^{6e}$ is any of the substituents shown in Table 1A to Table 3A (hereinafter, referred to as compound group SX64).

A compound (L-2) in which $R^{2a}$ and $R^{2b}$ are hydrogen atoms, $R^{6b}$ is a bromine atom, and $R^{6e}$ is any of the substituents shown in Table 1A to Table 3A (hereinafter, referred to as compound group SX65).

A compound (L-2) in which $R^{2a}$ is a methyl group, $R^{2b}$ is a hydrogen atom, $R^{6b}$ is a bromine atom, and $R^{6e}$ is any of the substituents shown in Table 1A to Table 3A (hereinafter, referred to as compound group SX66).

A compound (L-2) in which $R^{2a}$ and $R^{2b}$ are methyl groups, $R^{6b}$ is a bromine atom, and $R^{6e}$ is any of the substituents shown in Table 1A to Table 3A (hereinafter, referred to as compound group SX67).

A compound (L-2) in which $R^{2a}$ is an ethyl group, $R^{2b}$ is a hydrogen atom, $R^{6b}$ is a bromine atom, and $R^{6e}$ is any of the substituents shown in Table 1A to Table 3A (hereinafter, referred to as compound group SX68).

A compound (L-2) in which $R^{2a}$ and $R^{2b}$ are ethyl groups, $R^{6b}$ is a bromine atom, and $R^{6e}$ is any of the substituents shown in Table 1A to Table 3A (hereinafter, referred to as compound group SX69).

A compound (L-2) in which $R^{2a}$ is a propyl group, $R^{2b}$ is a hydrogen atom, $R^{6b}$ is a bromine atom, and $R^{6e}$ is any of the substituents shown in Table 1A to Table 3A (hereinafter, referred to as compound group SX70).

A compound (L-2) in which $R^{2a}$ and $R^{2b}$ are propyl groups, $R^{6b}$ is a bromine atom, and $R^{6e}$ is any of the substituents shown in Table 1A to Table 3A (hereinafter, referred to as compound group SX71).

A compound (L-2) in which $R^{2a}$ is a methyl group, $R^{2b}$ is an ethyl group, $R^{6b}$ is a bromine atom, and $R^{6e}$ is any of the substituents shown in Table 1A to Table 3A (hereinafter, referred to as compound group SX72).

A compound (L-2) in which $R^{2a}$ and $R^{2b}$ are hydrogen atoms, $R^{6b}$ is an iodine atom, and $R^{6e}$ is any of the substituents shown in Table 1A to Table 3A (hereinafter, referred to as compound group SX73).

A compound (L-2) in which $R^{2a}$ is a methyl group, $R^{2b}$ is a hydrogen atom, $R^{6b}$ is an iodine atom, and $R^{6e}$ is any of the substituents shown in Table 1A to Table 3A (hereinafter, referred to as compound group SX74).

A compound (L-2) in which $R^{2a}$ and $R^{2b}$ are methyl groups, $R^{6b}$ is an iodine atom, and $R^{6e}$ is any of the substituents shown in Table 1A to Table 3A (hereinafter, referred to as compound group SX75).

A compound (L-2) in which $R^{2a}$ is an ethyl group, $R^{2b}$ is a hydrogen atom, $R^{6b}$ is an iodine atom, and $R^{6e}$ is any of the substituents shown in Table 1A to Table 3A (hereinafter, referred to as compound group SX76).

A compound (L-2) in which $R^{2a}$ and $R^{2b}$ are ethyl groups, $R^{6b}$ is an iodine atom, and $R^{6e}$ is any of the substituents shown in Table 1A to Table 3A (hereinafter, referred to as compound group SX77).

A compound (L-2) in which $R^{2a}$ is a propyl group, $R^{2b}$ is a hydrogen atom, $R^{6b}$ is an iodine atom, and $R^{6e}$ is any of the substituents shown in Table 1A to Table 3A (hereinafter, referred to as compound group SX78).

A compound (L-2) in which $R^{2a}$ and $R^{2b}$ are propyl groups, $R^{6b}$ is an iodine atom, and $R^{6e}$ is any of the substituents shown in Table 1A to Table 3A (hereinafter, referred to as compound group SX79).

A compound (L-2) in which $R^{2a}$ is a methyl group, $R^{2b}$ is an ethyl group, $R^{6b}$ is an iodine atom, and $R^{6e}$ is any of the substituents shown in Table 1A to Table 3A (hereinafter, referred to as compound group SX80).

A compound represented by formula (L-3):

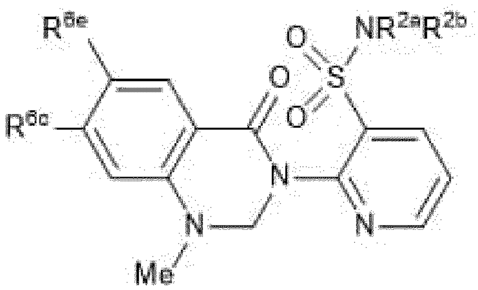

(L-3)

(hereinafter, referred to as compound (L-3)) in which $R^{2a}$ and $R^{2b}$ are hydrogen atoms, $R^{6c}$ is a hydrogen atom, and $R^{6e}$ is any of the substituents shown in Table 1A to Table 3A (hereinafter, referred to as compound group SX81).

A compound (L-3) in which $R^{2a}$ is a methyl group, $R^{2b}$ is a hydrogen atom, $R^{6c}$ is a hydrogen atom, and $R^{6e}$ is any of the substituents shown in Table 1A to Table 3A (hereinafter, referred to as compound group SX82).

A compound (L-3) in which $R^{2a}$ is a methyl group, $R^{2b}$ is a methyl group, $R^{6c}$ is a hydrogen atom, and $R^{6e}$ is any of the substituents shown in Table 1A to Table 3A (hereinafter, referred to as compound group SX83).

A compound (L-3) in which $R^{2a}$ is an ethyl group, $R^{2b}$ is a hydrogen atom, $R^{6c}$ is a hydrogen atom, and $R^{6e}$ is any of the substituents shown in Table 1A to Table 3A (hereinafter, referred to as compound group SX84).

A compound (L-3) in which $R^{2a}$ and $R^{2b}$ are ethyl groups, $R^{6c}$ is a hydrogen atom, and $R^{6e}$ is any of the substituents shown in Table 1A to Table 3A (hereinafter, referred to as compound group SX85).

A compound (L-3) in which $R^{2a}$ is a propyl group, $R^{2b}$ is a hydrogen atom, $R^{6c}$ is a hydrogen atom, and $R^{6e}$ is any

of the substituents shown in Table 1A to Table 3A (hereinafter, referred to as compound group SX86).

A compound (L-3) in which $R^{2a}$ and $R^{2b}$ are propyl groups, $R^{6c}$ is a hydrogen atom, and $R^{6e}$ is any of the substituents shown in Table 1A to Table 3A (hereinafter, referred to as compound group SX87).

A compound (L-3) in which $R^{2a}$ is a methyl group, $R^{2b}$ is an ethyl group, $R^{6c}$ is a hydrogen atom, and $R^{6e}$ is any of the substituents shown in Table 1A to Table 3A (hereinafter, referred to as compound group SX88).

A compound (L-3) in which $R^{2a}$ and $R^{2b}$ are hydrogen atoms, $R^{6c}$ is a fluorine atom, and $R^{6e}$ is any of the substituents shown in Table 1A to Table 3A (hereinafter, referred to as compound group SX89).

A compound (L-3) in which $R^{2a}$ is a methyl group, $R^{2b}$ is a hydrogen atom, $R^{6c}$ is a fluorine atom, and $R^{6e}$ is any of the substituents shown in Table 1A to Table 3A (hereinafter, referred to as compound group SX90).

A compound (L-3) in which $R^{2a}$ and $R^{2b}$ are methyl groups, $R^{6c}$ is a fluorine atom, and $R^{6e}$ is any of the substituents shown in Table 1A to Table 3A (hereinafter, referred to as compound group SX91).

A compound (L-3) in which $R^{2a}$ is an ethyl group, $R^{2b}$ is a hydrogen atom, $R^{6c}$ is a fluorine atom, and $R^{6e}$ is any of the substituents shown in Table 1A to Table 3A (hereinafter, referred to as compound group SX92).

A compound (L-3) in which $R^{2a}$ and $R^{2b}$ are ethyl groups, $R^{6c}$ is a fluorine atom, and $R^{6e}$ is any of the substituents shown in Table 1A to Table 3A (hereinafter, referred to as compound group SX93).

A compound (L-3) in which $R^{2a}$ is a propyl group, $R^{2b}$ is a hydrogen atom, $R^{6c}$ is a fluorine atom, and $R^{6e}$ is any of the substituents shown in Table 1A to Table 3A (hereinafter, referred to as compound group SX94).

A compound (L-3) in which $R^{2a}$ and $R^{2b}$ are propyl groups, $R^{6c}$ is a fluorine atom, and $R^{6e}$ is any of the substituents shown in Table 1A to Table 3A (hereinafter, referred to as compound group SX95).

A compound (L-3) in which $R^{2a}$ is a methyl group, $R^{2b}$ is an ethyl group, $R^{6c}$ is a fluorine atom, and $R^{6e}$ is any of the substituents shown in Table 1A to Table 3A (hereinafter, referred to as compound group SX96).

A compound (L-3) in which $R^{2a}$ and $R^{2b}$ are hydrogen atoms, $R^{6c}$ is a chlorine atom, and $R^{6e}$ is any of the substituents shown in Table 1A to Table 3A (hereinafter, referred to as compound group SX97).

A compound (L-3) in which $R^{2a}$ is a methyl group, $R^{2b}$ is a hydrogen atom, $R^{6c}$ is a chlorine atom, and $R^{6e}$ is any of the substituents shown in Table 1A to Table 3A (hereinafter, referred to as compound group SX98).

A compound (L-3) in which $R^{2a}$ and $R^{2b}$ are methyl groups, $R^{6c}$ is a chlorine atom, and $R^{6e}$ is any of the substituents shown in Table 1A to Table 3A (hereinafter, referred to as compound group SX99).

A compound (L-3) in which $R^{2a}$ is an ethyl group, $R^{2b}$ is a hydrogen atom, $R^{6c}$ is a chlorine atom, and $R^{6e}$ is any of the substituents shown in Table 1A to Table 3A (hereinafter, referred to as compound group SX100).

A compound (L-3) in which $R^{2a}$ and $R^{2b}$ are ethyl groups, $R^{6c}$ is a chlorine atom, and $R^{6e}$ is any of the substituents shown in Table 1A to Table 3A (hereinafter, referred to as compound group SX101).

A compound (L-3) in which $R^{2a}$ is a propyl group, $R^{2b}$ is a hydrogen atom, $R^{6c}$ is a chlorine atom, and $R^{6e}$ is any of the substituents shown in Table 1A to Table 3A (hereinafter, referred to as compound group SX102).

A compound (L-3) in which $R^{2a}$ and $R^{2b}$ are propyl groups, $R^{6c}$ is a chlorine atom, and $R^{6e}$ is any of the substituents shown in Table 1A to Table 3A (hereinafter, referred to as compound group SX103).

A compound (L-3) in which $R^{2a}$ is a methyl group, $R^{2b}$ is an ethyl group, $R^{6c}$ is a chlorine atom, and $R^{6e}$ is any of the substituents shown in Table 1A to Table 3A (hereinafter, referred to as compound group SX104).

A compound (L-3) in which $R^{2a}$ and $R^{2b}$ are hydrogen atoms, $R^{6c}$ is a bromine atom, and $R^{6e}$ is any of the substituents shown in Table 1A to Table 3A (hereinafter, referred to as compound group SX105).

A compound (L-3) in which $R^{2a}$ is a methyl group, $R^{2b}$ is a hydrogen atom, $R^{6c}$ is a bromine atom, and $R^{6e}$ is any of the substituents shown in Table 1A to Table 3A (hereinafter, referred to as compound group SX106).

A compound (L-3) in which $R^{2a}$ and $R^{2b}$ are methyl groups, $R^{6c}$ is a bromine atom, and $R^{6e}$ is any of the substituents shown in Table 1A to Table 3A (hereinafter, referred to as compound group SX107).

A compound (L-3) in which $R^{2a}$ is an ethyl group, $R^{2b}$ is a hydrogen atom, $R^{6c}$ is a bromine atom, and $R^{6e}$ is any of the substituents shown in Table 1A to Table 3A (hereinafter, referred to as compound group SX108).

A compound (L-3) in which $R^{2a}$ and $R^{2b}$ are ethyl groups, $R^{6c}$ is a bromine atom, and $R^{6e}$ is any of the substituents shown in Table 1A to Table 3A (hereinafter, referred to as compound group SX109).

A compound (L-3) in which $R^{2a}$ is a propyl group, $R^{2b}$ is a hydrogen atom, $R^{6c}$ is a bromine atom, and $R^{6e}$ is any of the substituents shown in Table 1A to Table 3A (hereinafter, referred to as compound group SX110).

A compound (L-3) in which $R^{2a}$ and $R^{2b}$ are propyl groups, $R^{6c}$ is a bromine atom, and $R^{6e}$ is any of the substituents shown in Table 1A to Table 3A (hereinafter, referred to as compound group SX111).

A compound (L-3) in which $R^{2a}$ is a methyl group, $R^{2b}$ is an ethyl group, $R^{6c}$ is a bromine atom, and $R^{6e}$ is any of the substituents shown in Table 1A to Table 3A (hereinafter, referred to as compound group SX112).

A compound (L-3) in which $R^{2a}$ and $R^{2b}$ are hydrogen atoms, $R^{6c}$ is an iodine atom, and $R^{6e}$ is any of the substituents shown in Table 1A to Table 3A (hereinafter, referred to as compound group SX113).

A compound (L-3) in which $R^{2a}$ is a methyl group, $R^{2b}$ is a hydrogen atom, $R^{6c}$ is an iodine atom, and $R^{6e}$ is any of the substituents shown in Table 1A to Table 3A (hereinafter, referred to as compound group SX114).

A compound (L-3) in which $R^{2a}$ and $R^{2b}$ are methyl groups, $R^{6c}$ is an iodine atom, and $R^{6e}$ is any of the

substituents shown in Table 1A to Table 3A (hereinafter, referred to as compound group SX115).

A compound (L-3) in which $R^{2a}$ is an ethyl group, $R^{2b}$ is a hydrogen atom, $R^{6c}$ is an iodine atom, and $R^{6e}$ is any of the substituents shown in Table 1A to Table 3A (hereinafter, referred to as compound group SX116).

A compound (L-3) in which $R^{2a}$ and $R^{2b}$ are ethyl groups, $R^{6c}$ is an iodine atom, and $R^{6e}$ is any of the substituents shown in Table 1A to Table 3A (hereinafter, referred to as compound group SX117).

A compound (L-3) in which $R^{2a}$ is a propyl group, $R^{2b}$ is a hydrogen atom, $R^{6c}$ is an iodine atom, and $R^{6e}$ is any of the substituents shown in Table 1A to Table 3A (hereinafter, referred to as compound group SX118).

A compound (L-3) in which $R^{2a}$ and $R^{2b}$ are propyl groups, $R^{6c}$ is an iodine atom, and $R^{6e}$ is any of the substituents shown in Table 1A to Table 3A (hereinafter, referred to as compound group SX119).

A compound (L-3) in which $R^{2a}$ is a methyl group, $R^{2b}$ is an ethyl group, $R^{6c}$ is an iodine atom, and $R^{6e}$ is any of the substituents shown in Table 1A to Table 3A (hereinafter, referred to as compound group SX120).

A compound represented by formula (L-4):

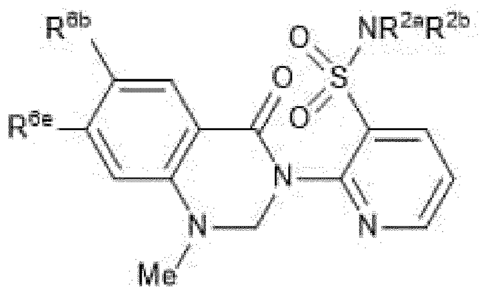

(L-4)

(hereinafter, referred to as compound (L-4)) in which $R^{2a}$ and $R^{2b}$ are hydrogen atoms, $R^{6b}$ is a hydrogen atom, and $R^{6e}$ is any of the substituents shown in Table 1A to Table 3A (hereinafter, referred to as compound group SX121).

A compound (L-4) in which $R^{2a}$ is a methyl group, $R^{2b}$ is a hydrogen atom, $R^{6b}$ is a hydrogen atom, and $R^{6e}$ is any of the substituents shown in Table 1A to Table 3A (hereinafter, referred to as compound group SX122).

A compound (L-4) in which $R^{2a}$ is a methyl group, $R^{2b}$ is a methyl group, $R^{6b}$ is a hydrogen atom, and $R^{6e}$ is any of the substituents shown in Table 1A to Table 3A (hereinafter, referred to as compound group SX123).

A compound (L-4) in which $R^{2a}$ is an ethyl group, $R^{2b}$ is a hydrogen atom, $R^{6b}$ is a hydrogen atom, and $R^{6e}$ is any of the substituents shown in Table 1A to Table 3A (hereinafter, referred to as compound group SX124).

A compound (L-4) in which $R^{2a}$ and $R^{2b}$ are ethyl groups, $R^{6b}$ is a hydrogen atom, and $R^{6e}$ is any of the substituents shown in Table 1A to Table 3A (hereinafter, referred to as compound group SX125).

A compound (L-4) in which $R^{2a}$ is a propyl group, $R^{2b}$ is a hydrogen atom, $R^{6b}$ is a hydrogen atom, and $R^{6e}$ is any of the substituents shown in Table 1A to Table 3A (hereinafter, referred to as compound group SX126).

A compound (L-4) in which $R^{2a}$ and $R^{2b}$ are propyl groups, $R^{6b}$ is a hydrogen atom, and $R^{6e}$ is any of the substituents shown in Table 1A to Table 3A (hereinafter, referred to as compound group SX127).

A compound (L-4) in which $R^{2a}$ is a methyl group, $R^{2b}$ is an ethyl group, $R^{6b}$ is a hydrogen atom, and $R^{6e}$ is any of the substituents shown in Table 1A to Table 3A (hereinafter, referred to as compound group SX128).

A compound (L-4) in which $R^{2a}$ and $R^{2b}$ are hydrogen atoms, $R^{6b}$ is a fluorine atom, and $R^{6e}$ is any of the substituents shown in Table 1A to Table 3A (hereinafter, referred to as compound group SX129).

A compound (L-4) in which $R^{2a}$ is a methyl group, $R^{2b}$ is a hydrogen atom, $R^{6b}$ is a fluorine atom, and $R^{6e}$ is any of the substituents shown in Table 1A to Table 3A (hereinafter, referred to as compound group SX130).

A compound (L-4) in which $R^{2a}$ and $R^{2b}$ are methyl groups, $R^{6b}$ is a fluorine atom, and $R^{6e}$ is any of the substituents shown in Table 1A to Table 3A (hereinafter, referred to as compound group SX131).

A compound (L-4) in which $R^{2a}$ is an ethyl group, $R^{2b}$ is a hydrogen atom, $R^{6b}$ is a fluorine atom, and $R^{6e}$ is any of the substituents shown in Table 1A to Table 3A (hereinafter, referred to as compound group SX132).

A compound (L-4) in which $R^{2a}$ and $R^{2b}$ are ethyl groups, $R^{6b}$ is a fluorine atom, and $R^{6e}$ is any of the substituents shown in Table 1A to Table 3A (hereinafter, referred to as compound group SX133).

A compound (L-4) in which $R^{2a}$ is a propyl group, $R^{2b}$ is a hydrogen atom, $R^{6b}$ is a fluorine atom, and $R^{6e}$ is any of the substituents shown in Table 1A to Table 3A (hereinafter, referred to as compound group SX134).

A compound (L-4) in which $R^{2a}$ and $R^{2b}$ are propyl groups, $R^{6b}$ is a fluorine atom, and $R^{6e}$ is any of the substituents shown in Table 1A to Table 3A (hereinafter, referred to as compound group SX135).

A compound (L-4) in which $R^{2a}$ is a methyl group, $R^{2b}$ is an ethyl group, $R^{6b}$ is a fluorine atom, and $R^{6e}$ is any of the substituents shown in Table 1A to Table 3A (hereinafter, referred to as compound group SX136).

A compound (L-4) in which $R^{2a}$ and $R^{2b}$ are hydrogen atoms, $R^{6b}$ is a chlorine atom, and $R^{6e}$ is any of the substituents shown in Table 1A to Table 3A (hereinafter, referred to as compound group SX137).

A compound (L-4) in which $R^{2a}$ is a methyl group, $R^{2b}$ is a hydrogen atom, $R^{6b}$ is a chlorine atom, and $R^{6e}$ is any of

the substituents shown in Table 1A to Table 3A (hereinafter, referred to as compound group SX138).

A compound (L-4) in which $R^{2a}$ and $R^{2b}$ are methyl groups, $R^{6b}$ is a chlorine atom, and $R^{6e}$ is any of the substituents shown in Table 1A to Table 3A (hereinafter, referred to as compound group SX139).

A compound (L-4) in which $R^{2a}$ is an ethyl group, $R^{2b}$ is a hydrogen atom, $R^{6b}$ is a chlorine atom, and $R^{6e}$ is any of the substituents shown in Table 1A to Table 3A (hereinafter, referred to as compound group SX140).

A compound (L-4) in which $R^{2a}$ and $R^{2b}$ are ethyl groups, $R^{6b}$ is a chlorine atom, and $R^{6e}$ is any of the substituents shown in Table 1A to Table 3A (hereinafter, referred to as compound group SX141).

A compound (L-4) in which $R^{2a}$ is a propyl group, $R^{2b}$ is a hydrogen atom, $R^{6b}$ is a chlorine atom, and $R^{6e}$ is any of the substituents shown in Table 1A to Table 3A (hereinafter, referred to as compound group SX142).

A compound (L-4) in which $R^{2a}$ and $R^{2b}$ are propyl groups, $R^{6b}$ is a chlorine atom, and $R^{6e}$ is any of the substituents shown in Table 1A to Table 3A (hereinafter, referred to as compound group SX143).

A compound (L-4) in which $R^{2a}$ is a methyl group, $R^{2b}$ is an ethyl group, $R^{6b}$ is a chlorine atom, and $R^{6e}$ is any of the substituents shown in Table 1A to Table 3A (hereinafter, referred to as compound group SX144).

A compound (L-4) in which $R^{2a}$ and $R^{2b}$ are hydrogen atoms, $R^{6b}$ is a bromine atom, and $R^{6e}$ is any of the substituents shown in Table 1A to Table 3A (hereinafter, referred to as compound group SX145).

A compound (L-4) in which $R^{2a}$ is a methyl group, $R^{2b}$ is a hydrogen atom, $R^{6b}$ is a bromine atom, and $R^{6e}$ is any of the substituents shown in Table 1A to Table 3A (hereinafter, referred to as compound group SX146).

A compound (L-4) in which $R^{2a}$ and $R^{2b}$ are methyl groups, $R^{6b}$ is a bromine atom, and $R^{6e}$ is any of the substituents shown in Table 1A to Table 3A (hereinafter, referred to as compound group SX147).

A compound (L-4) in which $R^{2a}$ is an ethyl group, $R^{2b}$ is a hydrogen atom, $R^{6b}$ is a bromine atom, and $R^{6e}$ is any of the substituents shown in Table 1A to Table 3A (hereinafter, referred to as compound group SX148).

A compound (L-4) in which $R^{2a}$ and $R^{2b}$ are ethyl groups, $R^{6b}$ is a bromine atom, and $R^{6e}$ is any of the substituents shown in Table 1A to Table 3A (hereinafter, referred to as compound group SX149).

A compound (L-4) in which $R^{2a}$ is a propyl group, $R^{2b}$ is a hydrogen atom, $R^{6b}$ is a bromine atom, and $R^{6e}$ is any of the substituents shown in Table 1A to Table 3A (hereinafter, referred to as compound group SX150).

A compound (L-4) in which $R^{2a}$ and $R^{2b}$ are propyl groups, $R^{6b}$ is a bromine atom, and $R^{6e}$ is any of the substituents shown in Table 1A to Table 3A (hereinafter, referred to as compound group SX151).

A compound (L-4) in which $R^{2a}$ is a methyl group, $R^{2b}$ is an ethyl group, $R^{6b}$ is a bromine atom, and $R^{6e}$ is any of the substituents shown in Table 1A to Table 3A (hereinafter, referred to as compound group SX152).

A compound (L-4) in which $R^{2a}$ and $R^{2b}$ are hydrogen atoms, $R^{6b}$ is an iodine atom, and $R^{6e}$ is any of the substituents shown in Table 1A to Table 3A (hereinafter, referred to as compound group SX153).

A compound (L-4) in which $R^{2a}$ is a methyl group, $R^{2b}$ is a hydrogen atom, $R^{6b}$ is an iodine atom, and $R^{6e}$ is any of the substituents shown in Table 1A to Table 3A (hereinafter, referred to as compound group SX154).

A compound (L-4) in which $R^{2a}$ and $R^{2b}$ are methyl groups, $R^{6b}$ is an iodine atom, and $R^{6e}$ is any of the substituents shown in Table 1A to Table 3A (hereinafter, referred to as compound group SX155).

A compound (L-4) in which $R^{2a}$ is an ethyl group, $R^{2b}$ is a hydrogen atom, $R^{6b}$ is an iodine atom, and $R^{6e}$ is any of the substituents shown in Table 1A to Table 3A (hereinafter, referred to as compound group SX156).

A compound (L-4) in which $R^{2a}$ and $R^{2b}$ are ethyl groups, $R^{6b}$ is an iodine atom, and $R^{6e}$ is any of the substituents shown in Table 1A to Table 3A (hereinafter, referred to as compound group SX157).

A compound (L-4) in which $R^{2a}$ is a propyl group, $R^{2b}$ is a hydrogen atom, $R^{6b}$ is an iodine atom, and $R^{6e}$ is any of the substituents shown in Table 1A to Table 3A (hereinafter, referred to as compound group SX158).

A compound (L-4) in which $R^{2a}$ and $R^{2b}$ are propyl groups, $R^{6b}$ is an iodine atom, and $R^{6e}$ is any of the substituents shown in Table 1A to Table 3A (hereinafter, referred to as compound group SX159).

A compound (L-4) in which $R^{2a}$ is a methyl group, $R^{2b}$ is an ethyl group, $R^{6b}$ is an iodine atom, and $R^{6e}$ is any of the substituents shown in Table 1A to Table 3A (hereinafter, referred to as compound group SX160).

A compound represented by formula (L-5):

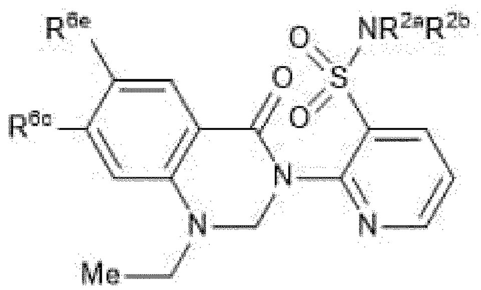

(L-5)

(hereinafter, referred to as compound (L-5)) in which $R^{2a}$ and $R^{2b}$ are hydrogen atoms, $R^{6c}$ is a hydrogen atom, and $R^{6e}$ is

any of the substituents shown in Table 1A to Table 3A (hereinafter, referred to as compound group SX161).
A compound (L-5) in which $R^{2a}$ is a methyl group, $R^{2b}$ is a hydrogen atom, $R^{6c}$ is a hydrogen atom, and $R^{6e}$ is any of the substituents shown in Table 1A to Table 3A (hereinafter, referred to as compound group SX162).
A compound (L-5) in which $R^{2a}$ is a methyl group, $R^{2b}$ is a methyl group, $R^{6c}$ is a hydrogen atom, and $R^{6e}$ is any of the substituents shown in Table 1A to Table 3A (hereinafter, referred to as compound group SX163).
A compound (L-5) in which $R^{2a}$ is an ethyl group, $R^{2b}$ is a hydrogen atom, $R^{6c}$ is a hydrogen atom, and $R^{6e}$ is any of the substituents shown in Table 1A to Table 3A (hereinafter, referred to as compound group SX164).
A compound (L-5) in which $R^{2a}$ and $R^{2b}$ are ethyl groups, $R^{6c}$ is a hydrogen atom, and $R^{6e}$ is any of the substituents shown in Table 1A to Table 3A (hereinafter, referred to as compound group SX165).
A compound (L-5) in which $R^{2a}$ is a propyl group, $R^{2b}$ is a hydrogen atom, $R^{6c}$ is a hydrogen atom, and $R^{6e}$ is any of the substituents shown in Table 1A to Table 3A (hereinafter, referred to as compound group SX166).
A compound (L-5) in which $R^{2a}$ and $R^{2b}$ are propyl groups, $R^{6c}$ is a hydrogen atom, and $R^{6e}$ is any of the substituents shown in Table 1A to Table 3A (hereinafter, referred to as compound group SX167).
A compound (L-5) in which $R^{2a}$ is a methyl group, $R^{2b}$ is an ethyl group, $R^{6c}$ is a hydrogen atom, and $R^{6e}$ is any of the substituents shown in Table 1A to Table 3A (hereinafter, referred to as compound group SX168).
A compound (L-5) in which $R^{2a}$ and $R^{2b}$ are hydrogen atoms, $R^{6c}$ is a fluorine atom, and $R^{6e}$ is any of the substituents shown in Table 1A to Table 3A (hereinafter, referred to as compound group SX169).
A compound (L-5) in which $R^{2a}$ is a methyl group, $R^{2b}$ is a hydrogen atom, $R^{6c}$ is a fluorine atom, and $R^{6e}$ is any of the substituents shown in Table 1A to Table 3A (hereinafter, referred to as compound group SX170).
A compound (L-5) in which $R^{2a}$ and $R^{2b}$ are methyl groups, $R^{6c}$ is a fluorine atom, and $R^{6e}$ is any of the substituents shown in Table 1A to Table 3A (hereinafter, referred to as compound group SX171).
A compound (L-5) in which $R^{2a}$ is an ethyl group, $R^{2b}$ is a hydrogen atom, $R^{6c}$ is a fluorine atom, and $R^{6e}$ is any of the substituents shown in Table 1A to Table 3A (hereinafter, referred to as compound group SX172).
A compound (L-5) in which $R^{2a}$ and $R^{2b}$ are ethyl groups, $R^{6c}$ is a fluorine atom, and $R^{6e}$ is any of the substituents shown in Table 1A to Table 3A (hereinafter, referred to as compound group SX173).
A compound (L-5) in which $R^{2a}$ is a propyl group, $R^{2b}$ is a hydrogen atom, $R^{6c}$ is a fluorine atom, and $R^{6e}$ is any of the substituents shown in Table 1A to Table 3A (hereinafter, referred to as compound group SX174).
A compound (L-5) in which $R^{2a}$ and $R^{2b}$ are propyl groups, $R^{6c}$ is a fluorine atom, and $R^{6e}$ is any of the substituents shown in Table 1A to Table 3A (hereinafter, referred to as compound group SX175).
A compound (L-5) in which $R^{2a}$ is a methyl group, $R^{2b}$ is an ethyl group, $R^{6c}$ is a fluorine atom, and $R^{6e}$ is any of the substituents shown in Table 1A to Table 3A (hereinafter, referred to as compound group SX176).
A compound (L-5) in which $R^{2a}$ and $R^{2b}$ are hydrogen atoms, $R^{6c}$ is a chlorine atom, and $R^{6e}$ is any of the substituents shown in Table 1A to Table 3A (hereinafter, referred to as compound group SX177).
A compound (L-5) in which $R^{2a}$ is a methyl group, $R^{2b}$ is a hydrogen atom, $R^{6c}$ is a chlorine atom, and $R^{6e}$ is any of the substituents shown in Table 1A to Table 3A (hereinafter, referred to as compound group SX178).
A compound (L-5) in which $R^{2a}$ and $R^{2b}$ are methyl groups, $R^{6c}$ is a chlorine atom, and $R^{6e}$ is any of the substituents shown in Table 1A to Table 3A (hereinafter, referred to as compound group SX179).
A compound (L-5) in which $R^{2a}$ is an ethyl group, $R^{2b}$ is a hydrogen atom, $R^{6c}$ is a chlorine atom, and $R^{6e}$ is any of the substituents shown in Table 1A to Table 3A (hereinafter, referred to as compound group SX180).
A compound (L-5) in which $R^{2a}$ and $R^{2b}$ are ethyl groups, $R^{6c}$ is a chlorine atom, and $R^{6e}$ is any of the substituents shown in Table 1A to Table 3A (hereinafter, referred to as compound group SX181).
A compound (L-5) in which $R^{2a}$ is a propyl group, $R^{2b}$ is a hydrogen atom, $R^{6c}$ is a chlorine atom, and $R^{6e}$ is any of the substituents shown in Table 1A to Table 3A (hereinafter, referred to as compound group SX182).
A compound (L-5) in which $R^{2a}$ and $R^{2b}$ are propyl groups, $R^{6c}$ is a chlorine atom, and $R^{6e}$ is any of the substituents shown in Table 1A to Table 3A (hereinafter, referred to as compound group SX183).
A compound (L-5) in which $R^{2a}$ is a methyl group, $R^{2b}$ is an ethyl group, $R^{6c}$ is a chlorine atom, and $R^{6e}$ is any of the substituents shown in Table 1A to Table 3A (hereinafter, referred to as compound group SX184).
A compound (L-5) in which $R^{2a}$ and $R^{2b}$ are hydrogen atoms, $R^{6c}$ is a bromine atom, and $R^{6e}$ is any of the substituents shown in Table 1A to Table 3A (hereinafter, referred to as compound group SX185).
A compound (L-5) in which $R^{2a}$ is a methyl group, $R^{2b}$ is a hydrogen atom, $R^{6c}$ is a bromine atom, and $R^{6e}$ is any of the substituents shown in Table 1A to Table 3A (hereinafter, referred to as compound group SX186).
A compound (L-5) in which $R^{2a}$ and $R^{2b}$ are methyl groups, $R^{6c}$ is a bromine atom, and $R^{6e}$ is any of the substituents shown in Table 1A to Table 3A (hereinafter, referred to as compound group SX187).
A compound (L-5) in which $R^{2a}$ is an ethyl group, $R^{2b}$ is a hydrogen atom, $R^{6c}$ is a bromine atom, and $R^{6e}$ is any of the substituents shown in Table 1A to Table 3A (hereinafter, referred to as compound group SX188).
A compound (L-5) in which $R^{2a}$ and $R^{2b}$ are ethyl groups, $R^{6c}$ is a bromine atom, and $R^{6e}$ is any of the substituents shown in Table 1A to Table 3A (hereinafter, referred to as compound group SX189).
A compound (L-5) in which $R^{2a}$ is a propyl group, $R^{2b}$ is a hydrogen atom, $R^{6c}$ is a bromine atom, and $R^{6e}$ is any of

the substituents shown in Table 1A to Table 3A (hereinafter, referred to as compound group SX190).

A compound (L-5) in which $R^{2a}$ and $R^{2b}$ are propyl groups, $R^{6c}$ is a bromine atom, and $R^{6e}$ is any of the substituents shown in Table 1A to Table 3A (hereinafter, referred to as compound group SX191).

A compound (L-5) in which $R^{2a}$ is a methyl group, $R^{2b}$ is an ethyl group, $R^{6c}$ is a bromine atom, and $R^{6e}$ is any of the substituents shown in Table 1A to Table 3A (hereinafter, referred to as compound group SX192).

A compound (L-5) in which $R^{2a}$ and $R^{2b}$ are hydrogen atoms, $R^{6c}$ is an iodine atom, and $R^{6e}$ is any of the substituents shown in Table 1A to Table 3A (hereinafter, referred to as compound group SX193).

A compound (L-5) in which $R^{2a}$ is a methyl group, $R^{2b}$ is a hydrogen atom, $R^{6c}$ is an iodine atom, and $R^{6e}$ is any of the substituents shown in Table 1A to Table 3A (hereinafter, referred to as compound group SX194).

A compound (L-5) in which $R^{2a}$ and $R^{2b}$ are methyl groups, $R^{6c}$ is an iodine atom, and $R^{6e}$ is any of the substituents shown in Table 1A to Table 3A (hereinafter, referred to as compound group SX195).

A compound (L-5) in which $R^{2a}$ is an ethyl group, $R^{2b}$ is a hydrogen atom, $R^{6c}$ is an iodine atom, and $R^{6e}$ is any of the substituents shown in Table 1A to Table 3A (hereinafter, referred to as compound group SX196).

A compound (L-5) in which $R^{2a}$ and $R^{2b}$ are ethyl groups, $R^{6c}$ is an iodine atom, and $R^{6e}$ is any of the substituents shown in Table 1A to Table 3A (hereinafter, referred to as compound group SX197).

A compound (L-5) in which $R^{2a}$ is a propyl group, $R^{2b}$ is a hydrogen atom, $R^{6c}$ is an iodine atom, and $R^{6e}$ is any of the substituents shown in Table 1A to Table 3A (hereinafter, referred to as compound group SX198).

A compound (L-5) in which $R^{2a}$ and $R^{2b}$ are propyl groups, $R^{6c}$ is an iodine atom, and $R^{6e}$ is any of the substituents shown in Table 1A to Table 3A (hereinafter, referred to as compound group SX199).

A compound (L-5) in which $R^{2a}$ is a methyl group, $R^{2b}$ is an ethyl group, $R^{6c}$ is an iodine atom, and $R^{6e}$ is any of the substituents shown in Table 1A to Table 3A (hereinafter, referred to as compound group SX200).

A compound represented by formula (L-6):

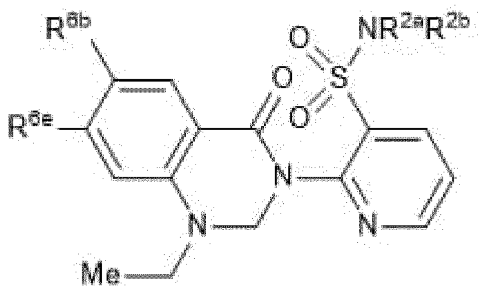

(L-6)

(hereinafter, referred to as compound (L-6)) in which $R^{2a}$ and $R^{2b}$ are hydrogen atoms, $R^{6b}$ is a hydrogen atom, and $R^{6e}$ is any of the substituents shown in Table 1A to Table 3A (hereinafter, referred to as compound group SX201).

A compound (L-6) in which $R^{2a}$ is a methyl group, $R^{2b}$ is a hydrogen atom, $R^{6b}$ is a hydrogen atom, and $R^{6e}$ is any of the substituents shown in Table 1A to Table 3A (hereinafter, referred to as compound group SX202).

A compound (L-6) in which $R^{2a}$ is a methyl group, $R^{2b}$ is a methyl group, $R^{6b}$ is a hydrogen atom, and $R^{6e}$ is any of the substituents shown in Table 1A to Table 3A (hereinafter, referred to as compound group SX203).

A compound (L-6) in which $R^{2a}$ is an ethyl group, $R^{2b}$ is a hydrogen atom, $R^{6b}$ is a hydrogen atom, and $R^{6e}$ is any of the substituents shown in Table 1A to Table 3A (hereinafter, referred to as compound group SX204).

A compound (L-6) in which $R^{2a}$ and $R^{2b}$ are ethyl groups, $R^{6b}$ is a hydrogen atom, and $R^{6e}$ is any of the substituents shown in Table 1A to Table 3A (hereinafter, referred to as compound group SX205).

A compound (L-6) in which $R^{2a}$ is a propyl group, $R^{2b}$ is a hydrogen atom, $R^{6b}$ is a hydrogen atom, and $R^{6e}$ is any of the substituents shown in Table 1A to Table 3A (hereinafter, referred to as compound group SX206).

A compound (L-6) in which $R^{2a}$ and $R^{2b}$ are propyl groups, $R^{6b}$ is a hydrogen atom, and $R^{6e}$ is any of the substituents shown in Table 1A to Table 3A (hereinafter, referred to as compound group SX207).

A compound (L-6) in which $R^{2a}$ is a methyl group, $R^{2b}$ is an ethyl group, $R^{6b}$ is a hydrogen atom, and $R^{6e}$ is any of the substituents shown in Table 1A to Table 3A (hereinafter, referred to as compound group SX208).

A compound (L-6) in which $R^{2a}$ and $R^{2b}$ are hydrogen atoms, $R^{6b}$ is a fluorine atom, and $R^{6e}$ is any of the substituents shown in Table 1A to Table 3A (hereinafter, referred to as compound group SX209).

A compound (L-6) in which $R^{2a}$ is a methyl group, $R^{2b}$ is a hydrogen atom, $R^{6b}$ is a fluorine atom, and $R^{6e}$ is any of the substituents shown in Table 1A to Table 3A (hereinafter, referred to as compound group SX210).

A compound (L-6) in which $R^{2a}$ and $R^{2b}$ are methyl groups, $R^{6b}$ is a fluorine atom, and $R^{6e}$ is any of the substituents shown in Table 1A to Table 3A (hereinafter, referred to as compound group SX211).

A compound (L-6) in which $R^{2a}$ is an ethyl group, $R^{2b}$ is a hydrogen atom, $R^{6b}$ is a fluorine atom, and $R^{6e}$ is any of the substituents shown in Table 1A to Table 3A (hereinafter, referred to as compound group SX212).

A compound (L-6) in which $R^{2a}$ and $R^{2b}$ are ethyl groups, $R^{6b}$ is a fluorine atom, and $R^{6e}$ is any of the substituents

shown in Table 1A to Table 3A (hereinafter, referred to as compound group SX213).

A compound (L-6) in which $R^{2a}$ is a propyl group, $R^{2b}$ is a hydrogen atom, $R^{6b}$ is a fluorine atom, and $R^{6e}$ is any of the substituents shown in Table 1A to Table 3A (hereinafter, referred to as compound group SX214).

A compound (L-6) in which $R^{2a}$ and $R^{2b}$ are propyl groups, $R^{6b}$ is a fluorine atom, and $R^{6e}$ is any of the substituents shown in Table 1A to Table 3A (hereinafter, referred to as compound group SX215).

A compound (L-6) in which $R^{2a}$ is a methyl group, $R^{2b}$ is an ethyl group, $R^{6b}$ is a fluorine atom, and $R^{6e}$ is any of the substituents shown in Table 1A to Table 3A (hereinafter, referred to as compound group SX216).

A compound (L-6) in which $R^{2a}$ and $R^{2b}$ are hydrogen atoms, $R^{6b}$ is a chlorine atom, and $R^{6e}$ is any of the substituents shown in Table 1A to Table 3A (hereinafter, referred to as compound group SX217).

A compound (L-6) in which $R^{2a}$ is a methyl group, $R^{2b}$ is a hydrogen atom, $R^{6b}$ is a chlorine atom, and $R^{6e}$ is any of the substituents shown in Table 1A to Table 3A (hereinafter, referred to as compound group SX218).

A compound (L-6) in which $R^{2a}$ and $R^{2b}$ are methyl groups, $R^{6b}$ is a chlorine atom, and $R^{6e}$ is any of the substituents shown in Table 1A to Table 3A (hereinafter, referred to as compound group SX219).

A compound (L-6) in which $R^{2a}$ is an ethyl group, $R^{2b}$ is a hydrogen atom, $R^{6b}$ is a chlorine atom, and $R^{6e}$ is any of the substituents shown in Table 1A to Table 3A (hereinafter, referred to as compound group SX220).

A compound (L-6) in which $R^{2a}$ and $R^{2b}$ are ethyl groups, $R^{6b}$ is a chlorine atom, and $R^{6e}$ is any of the substituents shown in Table 1A to Table 3A (hereinafter, referred to as compound group SX221).

A compound (L-6) in which $R^{2a}$ is a propyl group, $R^{2b}$ is a hydrogen atom, $R^{6b}$ is a chlorine atom, and $R^{6e}$ is any of the substituents shown in Table 1A to Table 3A (hereinafter, referred to as compound group SX222).

A compound (L-6) in which $R^{2a}$ and $R^{2b}$ are propyl groups, $R^{6b}$ is a chlorine atom, and $R^{6e}$ is any of the substituents shown in Table 1A to Table 3A (hereinafter, referred to as compound group SX223).

A compound (L-6) in which $R^{2a}$ is a methyl group, $R^{2b}$ is an ethyl group, $R^{6b}$ is a chlorine atom, and $R^{6e}$ is any of the substituents shown in Table 1A to Table 3A (hereinafter, referred to as compound group SX224).

A compound (L-6) in which $R^{2a}$ and $R^{2b}$ are hydrogen atoms, $R^{6b}$ is a bromine atom, and $R^{6e}$ is any of the substituents shown in Table 1A to Table 3A (hereinafter, referred to as compound group SX225).

A compound (L-6) in which $R^{2a}$ is a methyl group, $R^{2b}$ is a hydrogen atom, $R^{6b}$ is a bromine atom, and $R^{6e}$ is any of the substituents shown in Table 1A to Table 3A (hereinafter, referred to as compound group SX226).

A compound (L-6) in which $R^{2a}$ and $R^{2b}$ are methyl groups, $R^{6b}$ is a bromine atom, and $R^{6e}$ is any of the substituents shown in Table 1A to Table 3A (hereinafter, referred to as compound group SX227).

A compound (L-6) in which $R^{2a}$ is an ethyl group, $R^{2b}$ is a hydrogen atom, $R^{6b}$ is a bromine atom, and $R^{6e}$ is any of the substituents shown in Table 1A to Table 3A (hereinafter, referred to as compound group SX228).

A compound (L-6) in which $R^{2a}$ and $R^{2b}$ are ethyl groups, $R^{6b}$ is a bromine atom, and $R^{6e}$ is any of the substituents shown in Table 1A to Table 3A (hereinafter, referred to as compound group SX229).

A compound (L-6) in which $R^{2a}$ is a propyl group, $R^{2b}$ is a hydrogen atom, $R^{6b}$ is a bromine atom, and $R^{6e}$ is any of the substituents shown in Table 1A to Table 3A (hereinafter, referred to as compound group SX230).

A compound (L-6) in which $R^{2a}$ and $R^{2b}$ are propyl groups, $R^{6b}$ is a bromine atom, and $R^{6e}$ is any of the substituents shown in Table 1A to Table 3A (hereinafter, referred to as compound group SX231).

A compound (L-6) in which $R^{2a}$ is a methyl group, $R^{2b}$ is an ethyl group, $R^{6b}$ is a bromine atom, and $R^{6e}$ is any of the substituents shown in Table 1A to Table 3A (hereinafter, referred to as compound group SX232).

A compound (L-6) in which $R^{2a}$ and $R^{2b}$ are hydrogen atoms, $R^{6b}$ is an iodine atom, and $R^{6e}$ is any of the substituents shown in Table 1A to Table 3A (hereinafter, referred to as compound group SX233).

A compound (L-6) in which $R^{2a}$ is a methyl group, $R^{2b}$ is a hydrogen atom, $R^{6b}$ is an iodine atom, and $R^{6e}$ is any of the substituents shown in Table 1A to Table 3A (hereinafter, referred to as compound group SX234).

A compound (L-6) in which $R^{2a}$ and $R^{2b}$ are methyl groups, $R^{6b}$ is an iodine atom, and $R^{6e}$ is any of the substituents shown in Table 1A to Table 3A (hereinafter, referred to as compound group SX235).

A compound (L-6) in which $R^{2a}$ is an ethyl group, $R^{2b}$ is a hydrogen atom, $R^{6b}$ is an iodine atom, and $R^{6e}$ is any of the substituents shown in Table 1A to Table 3A (hereinafter, referred to as compound group SX236).

A compound (L-6) in which $R^{2a}$ and $R^{2b}$ are ethyl groups, $R^{6b}$ is an iodine atom, and $R^{6e}$ is any of the substituents shown in Table 1A to Table 3A (hereinafter, referred to as compound group SX237).

A compound (L-6) in which $R^{2a}$ is a propyl group, $R^{2b}$ is a hydrogen atom, $R^{6b}$ is an iodine atom, and $R^{6e}$ is any of the substituents shown in Table 1A to Table 3A (hereinafter, referred to as compound group SX238).

A compound (L-6) in which $R^{2a}$ and $R^{2b}$ are propyl groups, $R^{6b}$ is an iodine atom, and $R^{6e}$ is any of the substituents shown in Table 1A to Table 3A (hereinafter, referred to as compound group SX239).

A compound (L-6) in which $R^{2a}$ is a methyl group, $R^{2b}$ is an ethyl group, $R^{6b}$ is an iodine atom, and $R^{6e}$ is any of the substituents shown in Table 1A to Table 3A (hereinafter, referred to as compound group SX240).

A compound represented by formula (L-7):

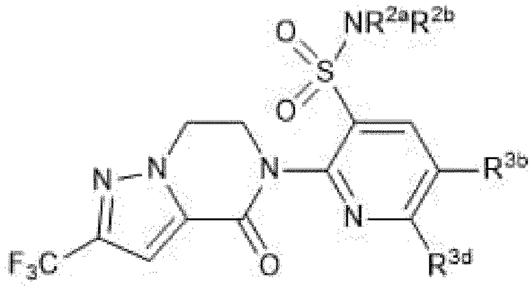

(L-7)

(hereinafter, referred to as compound (L-7)) in which $R^{2a}$ and $R^{2b}$ are hydrogen atoms, $R^{3b}$ is a hydrogen atom, and $R^{3d}$ is any of the substituents shown in Tables 7A to 15A (hereinafter, referred to as compound group SX241).

[Table 7A]

| |
|---|
| F |
| Cl |
| Br |
| Me |
| Et |
| Pr |
| i-Pr |
| c-Pr |
| 1-CN-c-Pr |
| OMe |
| OEt |
| OPr |
| Oi-Pr |
| $CF_3$ |
| $NH_2$ |
| $NHCH_2CF_3$ |
| CN |
| C(O)OEt |
| NHC(O)c-Pr |
| NMeC(O)c-Pr |
| CH=N-OH |
| CH=N-OMe |

[Table 8A]

| |
|---|
| Ph |
| 3-F-Ph |
| 4-F-Ph |
| 3-Cl-Ph |
| 4-Cl-Ph |
| 3-$CF_3$-Ph |
| 4-$CF_3$-Ph |
| 3-$NMe_2$-Ph |
| 4-$NMe_2$-Ph |
| 3-CN-Ph |
| 4-CN-Ph |
| 4-C(O)$NMe_2$-Ph |
| 4-NHC(O)Me-Ph |
| 3,4-$F_2$-Ph |
| 3,5-$F_2$-Ph |
| 2,4-$F_2$-Ph |
| 3,4,5-$F_3$-Ph |
| 3,4-$Cl_2$-Ph |
| 3,5-$Cl_2$-Ph |
| 3,5-$Cl_2$-4-F-Ph |
| OPh |
| O-2-F-Ph |

[Table 9A]

| |
|---|
| Py2 |
| 4-F-Py2 |
| 5-F-Py2 |
| 4-Cl-Py2 |
| 5-Cl-Py2 |
| 4-$CF_3$-Py2 |
| 5-$CF_3$-Py2 |
| 3-Me-Py2 |
| 4-Me-Py2 |
| 5-Me-Py2 |
| 6-Me-Py2 |
| 5-CN-Py2 |
| 5-$OCH_2CF_2CF_3$-Py2 |
| 3,5-$F_2$-Py2 |
| Py3 |
| 6-$CF_3$-Py3 |
| 5-$CF_3$-Py3 |
| 6-F-Py3 |
| 6-Cl-Py3 |
| Py4 |
| OPy2 |
| OPy3 |

[Table 10A]

| |
|---|
| 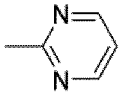 |
| 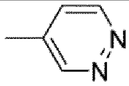 |
| 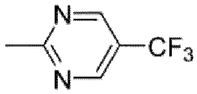 |
| 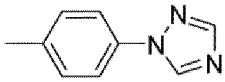 |
| 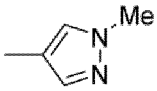 |
| 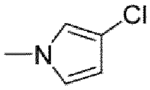 |
| 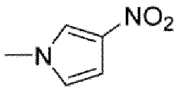 |

[Table 11A]

| |
|---|
| 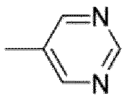 |
| 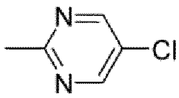 |
| 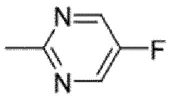 |
| 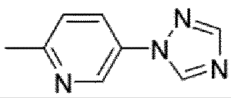 |
| 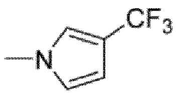 |
| 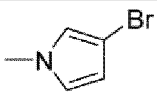 |
| 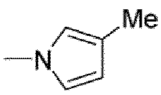 |

[Table 12A]

| |
|---|
| 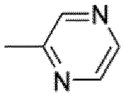 |
| 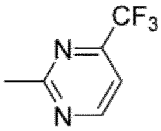 |
| 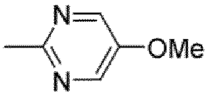 |
| 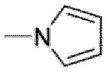 |
| 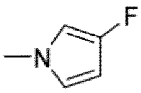 |
| 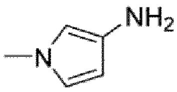 |
| 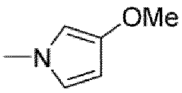 |

[Table 13A]

| |
|---|
| 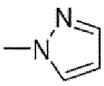 |
| 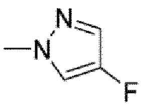 |

[Table 14A]

| |
|---|
| 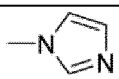 |
| 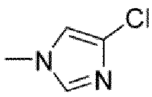 |

[Table 15A]

| |
|---|
| 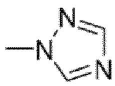 |
| 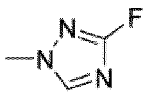 |

| | | |
|---|---|---|
| 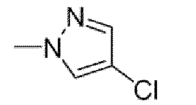 | 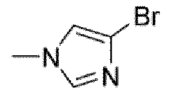 | 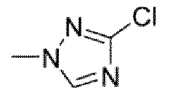 |
| 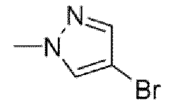 | 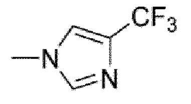 | 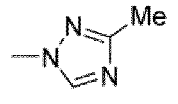 |
| 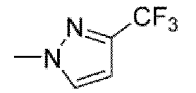 | 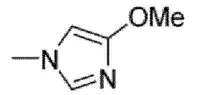 | 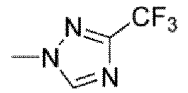 |
| 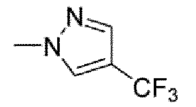 | 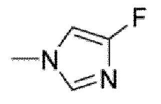 | 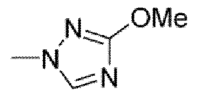 |
| 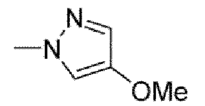 | 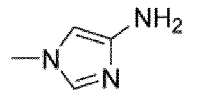 | 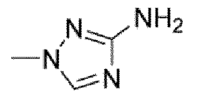 |
| 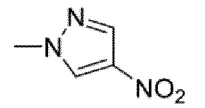 | 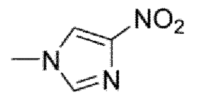 | 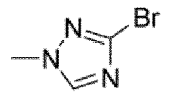 |
| 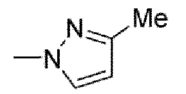 | 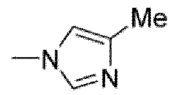 | 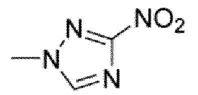 |

A compound (L-7) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX242).

A compound (L-7) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX243).

A compound (L-7) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX244).

A compound (L-7) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX245).

A compound (L-7) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX246).

A compound (L-7) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX247).

A compound (L-7) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX248).

A compound represented by formula (L-8):

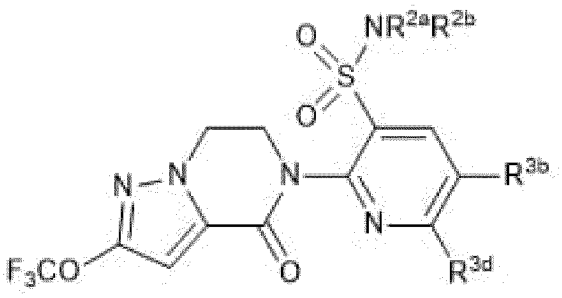

(L-8)

(hereinafter, referred to as compound (L-8)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX249).

A compound (L-8) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX250).

A compound (L-8) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX251).

A compound (L-8) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX252).

A compound (L-8) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX253).

A compound (L-8) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX254).

A compound (L-8) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX255).

A compound (L-8) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX256).

A compound represented by formula (L-9):

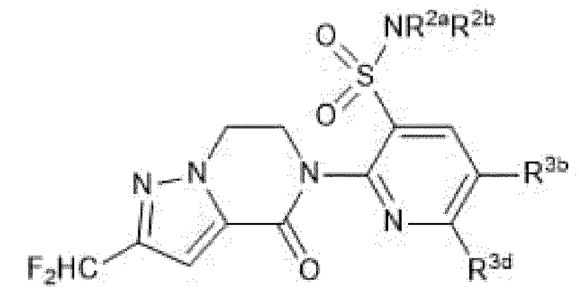

(L-9)

(hereinafter, referred to as compound (L-9)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX257).

A compound (L-9) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX258).

A compound (L-9) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX259).

A compound (L-9) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX260).

A compound (L-9) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX261).

A compound (L-9) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX262).

A compound (L-9) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX263).

A compound (L-9) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX264).

A compound represented by formula (L-10):

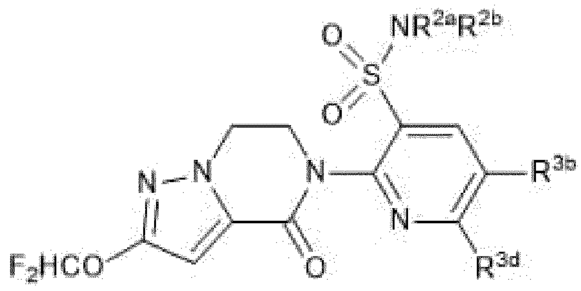

(L-10)

(hereinafter, referred to as compound (L-10)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX265).

A compound (L-10) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX266).

A compound (L-10) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX267).

A compound (L-10) in which $R^{2a}$ is a methyl group, $R^{2b}$ is a hydrogen atom, $R^{3d}$ is a hydrogen atom, and $R^{3b}$ is any

of the substituents shown in Table 7A to Table 15A (hereinafter, referred to as compound group SX268).

A compound (L-10) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX269).

A compound (L-10) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX270).

A compound (L-10) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX271).

A compound (L-10) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX272).

A compound represented by formula (L-11):

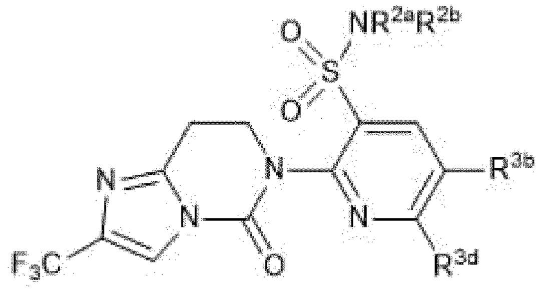

(L-11)

(hereinafter, referred to as compound (L-11)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX273).

A compound (L-11) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX274).

A compound (L-11) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX275).

A compound (L-11) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX276).

A compound (L-11) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX277).

A compound (L-11) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX278).

A compound (L-11) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX279).

A compound (L-11) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX280).

A compound represented by formula (L-12):

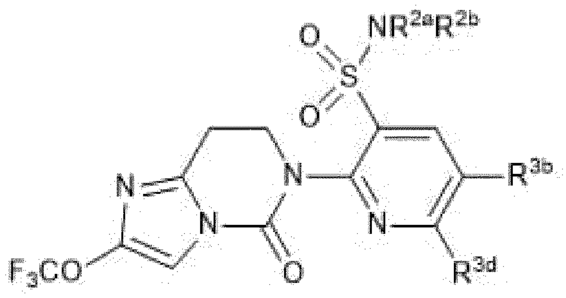

(L-12)

(hereinafter, referred to as compound (L-12)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX281).

A compound (L-12) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX282).

A compound (L-12) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX283).

A compound (L-12) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX284).

A compound (L-12) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX285).

A compound (L-12) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX286).

A compound (L-12) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX287).

A compound (L-12) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX288).

A compound represented by formula (L-13):

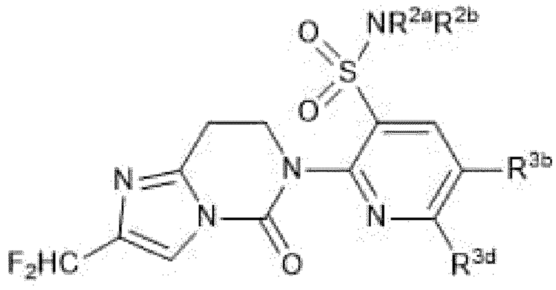

(L-13)

(hereinafter, referred to as compound (L-13)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX289).

A compound (L-13) in which $R^{2e}$ and $R^{2b}$ are hydrogen atoms, $R^{3d}$ is a hydrogen atom, and $R^{3b}$ is any of the substituents shown in Table 7A to Table 15A (hereinafter, referred to as compound group SX290).

A compound (L-13) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX291).

A compound (L-13) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX292).

A compound (L-13) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX293).

A compound (L-13) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX294).

A compound (L-13) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX295).

A compound (L-13) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX296).

A compound represented by formula (L-14):

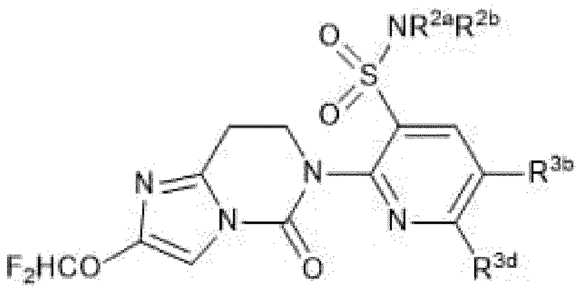

(L-14)

(hereinafter, referred to as compound (L-14)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX297) .

A compound (L-14) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX298).

A compound (L-14) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX299).

A compound (L-14) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX300).

A compound (L-14) in which $R^{2a}$ is an ethyl group, $R^{2b}$ is a hydrogen atom, $R^{3b}$ is a hydrogen atom, and $R^{3d}$ is any

of the substituents shown in Table 7A to Table 15A (hereinafter, referred to as compound group SX301).

A compound (L-14) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX302).

A compound (L-14) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX303).

A compound (L-14) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX304).

A compound represented by formula (L-15):

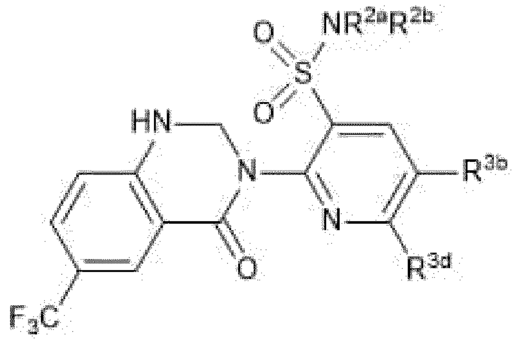

(L-15)

(hereinafter, referred to as compound (L-15)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX305).

A compound (L-15) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX306).

A compound (L-15) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX307).

A compound (L-15) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX308).

A compound (L-15) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX309).

A compound (L-15) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX310).

A compound (L-15) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX311).

A compound (L-15) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX312).

A compound represented by formula (L-16):

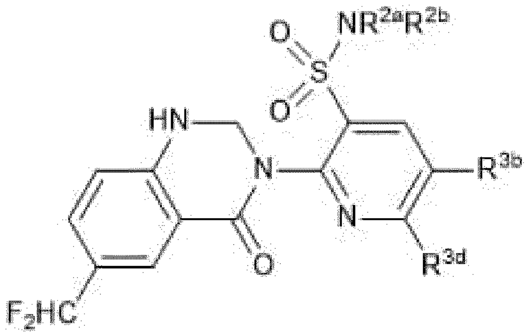

(L-16)

(hereinafter, referred to as compound (L-16)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX313).

A compound (L-16) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX314).

A compound (L-16) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX315).

A compound (L-16) in which $R^{2a}$ is a methyl group, $R^{2b}$ is a hydrogen atom, $R^{3d}$ is a hydrogen atom, and $R^{3b}$ is any

of the substituents shown in Table 7A to Table 15A (hereinafter, referred to as compound group SX316).

A compound (L-16) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX317).

A compound (L-16) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX318).

A compound (L-16) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX319).

A compound (L-16) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX320).

A compound represented by formula (L-17):

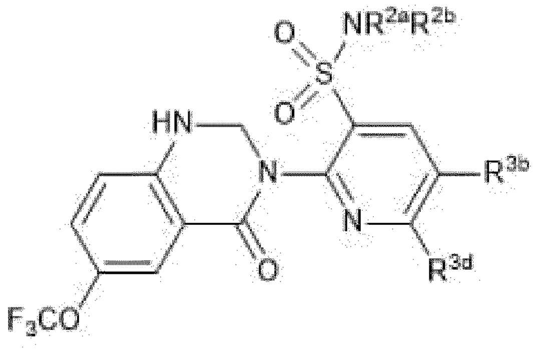

(L-17)

(hereinafter, referred to as compound (L-17)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX321).

A compound (L-17) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX322).

A compound (L-17) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX323).

A compound (L-17) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX324).

A compound (L-17) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX325).

A compound (L-17) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX326).

A compound (L-17) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX327).

A compound (L-17) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX328).

A compound represented by formula (L-18):

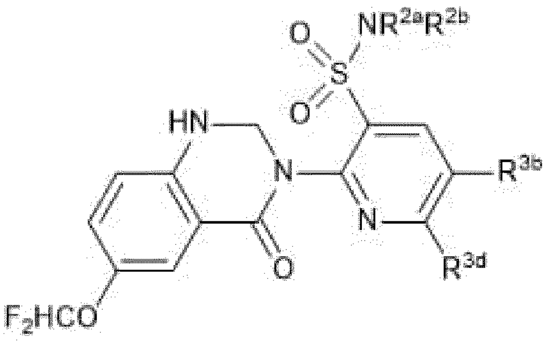

(L-18)

(hereinafter, referred to as compound (L-18)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX329).

A compound (L-18) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX330).

A compound (L-18) in which $R^{2a}$ is a methyl group, $R^{2b}$ is a hydrogen atom, $R^{3b}$ is a hydrogen atom, and $R^{3d}$ is any

of the substituents shown in Table 7A to Table 15A (hereinafter, referred to as compound group SX331).

A compound (L-18) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX332).

A compound (L-18) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX333).

A compound (L-18) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX334).

A compound (L-18) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX335).

A compound (L-18) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX336).

A compound represented by formula (L-19):

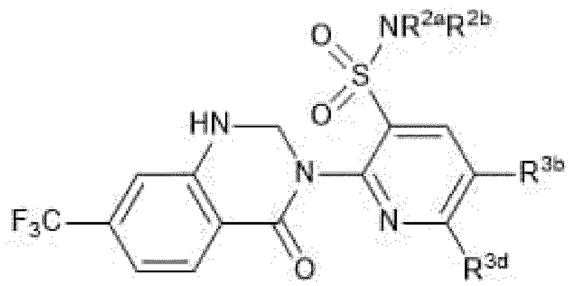

(L-19)

(hereinafter, referred to as compound (L-19)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX337).

A compound (L-19) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX338).

A compound (L-19) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX339).

A compound (L-19) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX340).

A compound (L-19) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX341).

A compound (L-19) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX342).

A compound (L-19) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX343).

A compound (L-19) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX344).

A compound represented by formula (L-20):

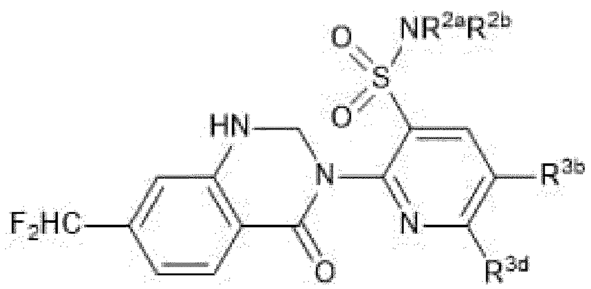

(L-20)

(hereinafter, referred to as compound (L-20)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX345).

A compound (L-20) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX346).

A compound (L-20) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX347).

A compound (L-20) in which $R^{2a}$ is a methyl group, $R^{2b}$ is a hydrogen atom, $R^{3d}$ is a hydrogen atom, and $R^{3b}$ is any

of the substituents shown in Table 7A to Table 15A (hereinafter, referred to as compound group SX348).

A compound (L-20) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX349).

A compound (L-20) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX350).

A compound (L-20) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX351).

A compound (L-20) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX352).

A compound represented by formula (L-21):

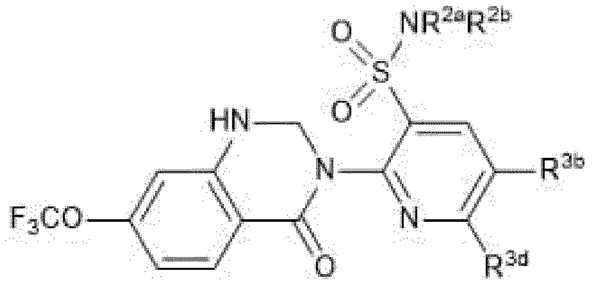

(L-21)

(hereinafter, referred to as compound (L-21)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX353).

A compound (L-21) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX354).

A compound (L-21) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX355).

A compound (L-21) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX356).

A compound (L-21) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX357).

A compound (L-21) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX358).

A compound (L-21) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX359).

A compound (L-21) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX360).

A compound represented by formula (L-22):

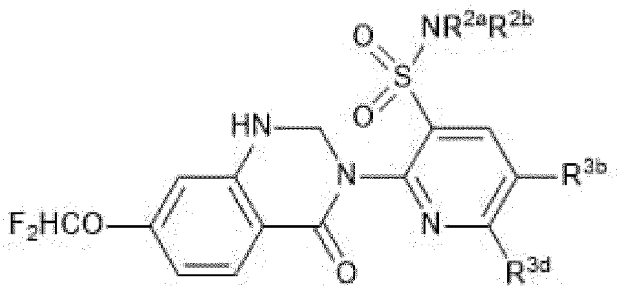

(L-22)

(hereinafter, referred to as compound (L-22)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX361).

A compound (L-22) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX362).

A compound (L-22) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX363).

A compound (L-22) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX364).

A compound (L-22) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX365).
A compound (L-22) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX366).
A compound (L-22) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX367).
A compound (L-22) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX368).
A compound represented by formula (L-23):

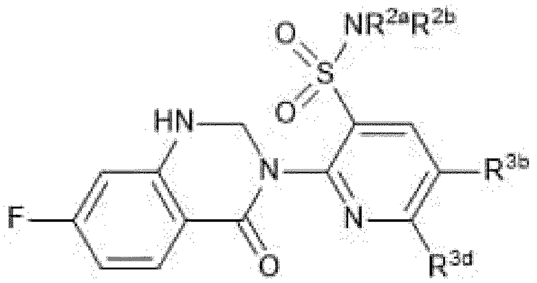

(L-23)

(hereinafter, referred to as compound (L-23)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX369).
A compound (L-23) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX370).
A compound (L-23) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX371).
A compound (L-23) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX372).
A compound (L-23) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX373).
A compound (L-23) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX374).
A compound (L-23) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX375).
A compound (L-23) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX376).
A compound represented by formula (L-24):

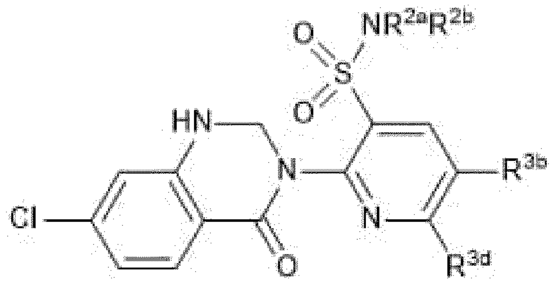

(L-24)

(hereinafter, referred to as compound (L-24)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX377) .
A compound (L-24) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX378).
A compound (L-24) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX379).
A compound (L-24) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX380).
A compound (L-24) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX381).

A compound (L-24) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX382).
A compound (L-24) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX383).
A compound (L-24) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX384).
A compound represented by formula (L-25):

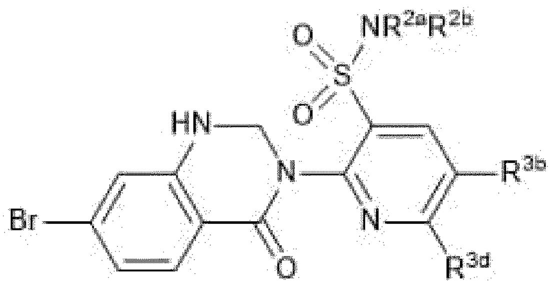

(L-25)

(hereinafter, referred to as compound (L-25)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX385).
A compound (L-25) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX386).
A compound (L-25) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX387).
A compound (L-25) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX388).
A compound (L-25) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX389).
A compound (L-25) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX390).
A compound (L-25) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX391).
A compound (L-25) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX392).
A compound represented by formula (L-26):

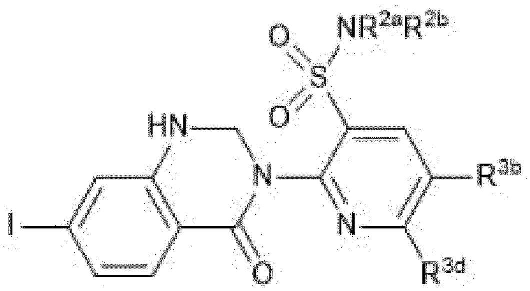

(L-26)

(hereinafter, referred to as compound (L-26)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX393).
A compound (L-26) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX394).
A compound (L-26) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX395).
A compound (L-26) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX396).
A compound (L-26) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX397).
A compound (L-26) in which $R^{2a}$ is an ethyl group, $R^{2b}$ is a hydrogen atom, $R^{3d}$ is a hydrogen atom, and $R^{3b}$ is any

of the substituents shown in Table 7A to Table 15A (hereinafter, referred to as compound group SX398).

A compound (L-26) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX399).

A compound (L-26) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX400).

A compound represented by formula (L-27):

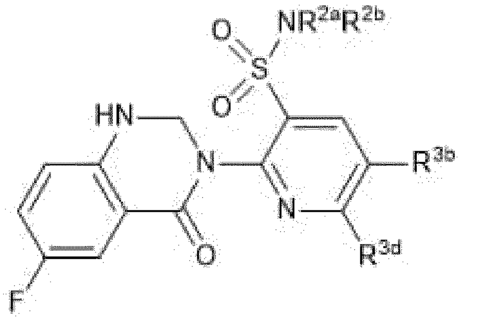

(L-27)

(hereinafter, referred to as compound (L-27)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX401).

A compound (L-27) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX402).

A compound (L-27) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX403).

A compound (L-27) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX404).

A compound (L-27) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX405).

A compound (L-27) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX406).

A compound (L-27) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX407).

A compound (L-27) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX408).

A compound represented by formula (L-28):

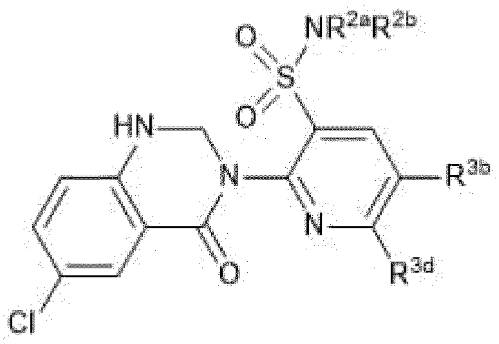

(L-28)

(hereinafter, referred to as compound (L-28)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX409).

A compound (L-28) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX410).

A compound (L-28) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX411).

A compound (L-28) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX412).

A compound (L-28) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX413).

A compound (L-28) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX414).
A compound (L-28) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX415).
A compound (L-28) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX416).
A compound represented by formula (L-29):

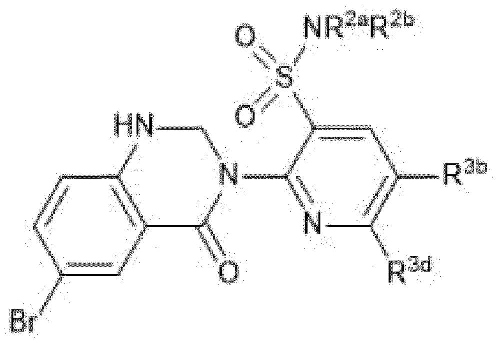

(L-29)

(hereinafter, referred to as compound (L-29)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX417).
A compound (L-29) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX418).
A compound (L-29) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX419).
A compound (L-29) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX420).
A compound (L-29) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX421).
A compound (L-29) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX422).
A compound (L-29) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX423).
A compound (L-29) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX424).
A compound represented by formula (L-30):

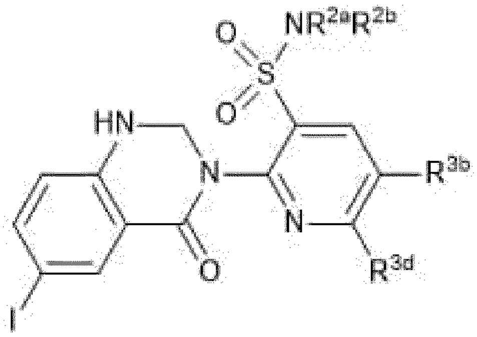

(L-30)

(hereinafter, referred to as compound (L-30)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX425).
A compound (L-30) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX426).
A compound (L-30) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX427).
A compound (L-30) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX428).

A compound (L-30) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX429).

A compound (L-30) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX430).

A compound (L-30) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX431).

A compound (L-30) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX432).

A compound represented by formula (L-31):

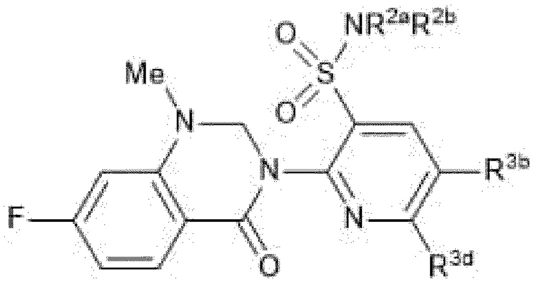

( L-31 )

(hereinafter, referred to as compound (L-31)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX433).

A compound (L-31) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX434).

A compound (L-31) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX435).

A compound (L-31) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX436).

A compound (L-31) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX437).

A compound (L-31) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX438).

A compound (L-31) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX439).

A compound (L-31) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX440).

A compound represented by formula (L-32):

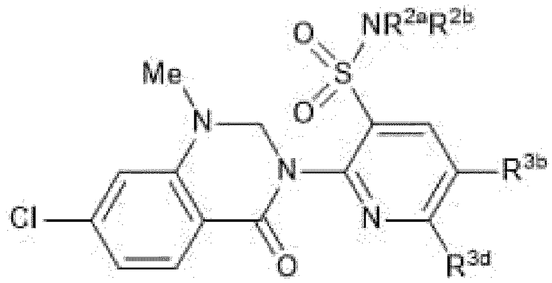

( L-32 )

(hereinafter, referred to as compound (L-32)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX441).

A compound (L-32) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX442).

A compound (L-32) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX443).

A compound (L-32) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX444).

A compound (L-32) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX445).

A compound (L-32) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX446).
A compound (L-32) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX447).
A compound (L-32) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX448).
A compound represented by formula (L-33):

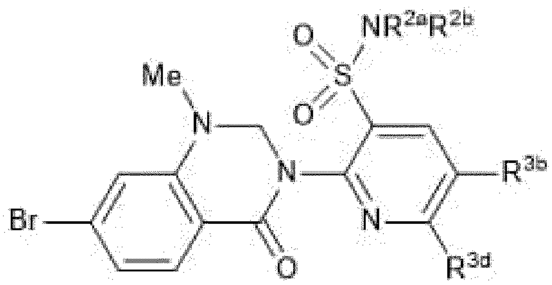

(L-33)

(hereinafter, referred to as compound (L-33)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX449) .
A compound (L-33) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX450).
A compound (L-33) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX451).
A compound (L-33) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX452).
A compound (L-33) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX453).
A compound (L-33) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX454).
A compound (L-33) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX455).
A compound (L-33) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX456).
A compound represented by formula (L-34):

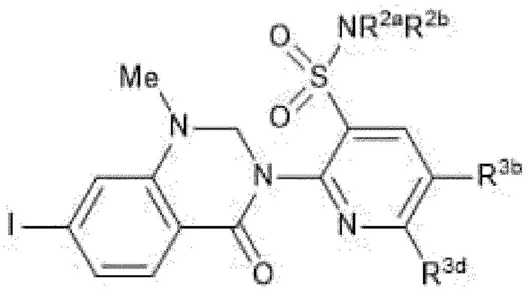

(L-34)

(hereinafter, referred to as compound (L-34)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX457).
A compound (L-34) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX458).
A compound (L-34) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX459).
A compound (L-34) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX460).
A compound (L-34) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX461).
A compound (L-34) in which $R^{2a}$ is an ethyl group, $R^{2b}$ is a hydrogen atom, $R^{3d}$ is a hydrogen atom, and $R^{3b}$ is any

of the substituents shown in Table 7A to Table 15A (hereinafter, referred to as compound group SX462).

A compound (L-34) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX463).

A compound (L-34) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX464).

A compound represented by formula (L-35):

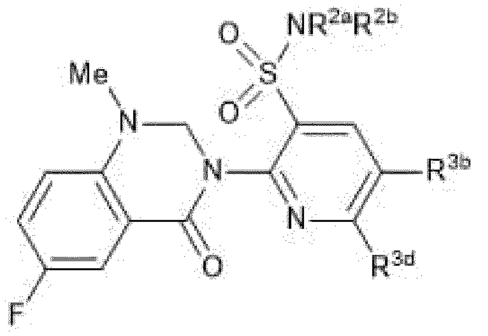

(L-35)

(hereinafter, referred to as compound (L-35)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX465).

A compound (L-35) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX466).

A compound (L-35) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX467).

A compound (L-35) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX468).

A compound (L-35) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX469).

A compound (L-35) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX470).

A compound (L-35) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX471).

A compound (L-35) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX472).

A compound represented by formula (L-36):

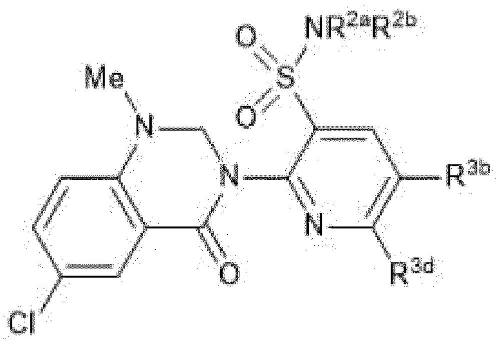

(L-36)

(hereinafter, referred to as compound (L-36)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX473).

A compound (L-36) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX474).

A compound (L-36) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX475).

A compound (L-36) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX476).

A compound (L-36) in which $R^{2a}$ is an ethyl group, $R^{2b}$ is a hydrogen atom, $R^{3b}$ is a hydrogen atom, and $R^{3d}$ is any

of the substituents shown in Table 7A to Table 15A (hereinafter, referred to as compound group SX477).

A compound (L-36) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX478).

A compound (L-36) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX479).

A compound (L-36) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX480).

A compound represented by formula (L-37):

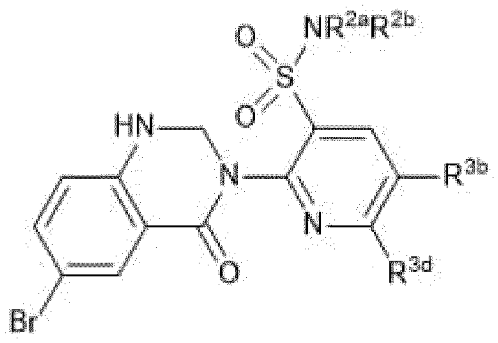

(L-37)

(hereinafter, referred to as compound (L-37)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX481).

A compound (L-37) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX482).

A compound (L-37) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX483).

A compound (L-37) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX484).

A compound (L-37) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX485).

A compound (L-37) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX486).

A compound (L-37) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX487).

A compound (L-37) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX488).

A compound represented by formula (L-38):

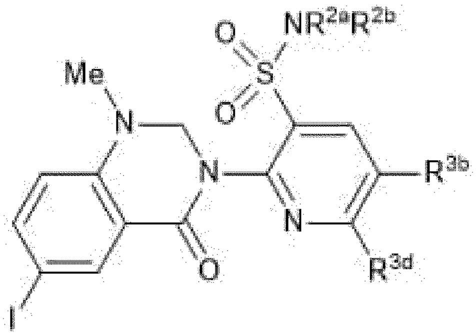

(L-38)

(hereinafter, referred to as compound (L-38)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX489).

A compound (L-38) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX490).

A compound (L-38) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX491).

A compound (L-38) in which $R^{2a}$ is a methyl group, $R^{2b}$ is a hydrogen atom, $R^{3d}$ is a hydrogen atom, and $R^{3b}$ is any

of the substituents shown in Table 7A to Table 15A (hereinafter, referred to as compound group SX492).

A compound (L-38) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX493).

A compound (L-38) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX494).

A compound (L-38) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX495).

A compound (L-38) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX496).

A compound represented by formula (L-39):

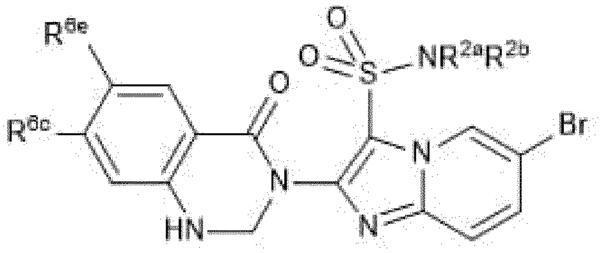

(L-39)

(hereinafter, referred to as compound (L-39)) in which $R^{2a}$ and $R^{2b}$ are hydrogen atoms, $R^{6c}$ is a fluorine atom, and $R^{6e}$ is any of the substituents shown in Table 1A to Table 3A (hereinafter, referred to as compound group SX497) .

A compound (L-39) in which $R^{2a}$ is a methyl group, $R^{2b}$ is a hydrogen atom, $R^{6c}$ is a fluorine atom, and $R^{6e}$ is any of the substituents shown in Table 1A to Table 3A (hereinafter, referred to as compound group SX498).

A compound (L-39) in which $R^{2a}$ and $R^{2b}$ are methyl groups, $R^{6c}$ is a fluorine atom, and $R^{6e}$ is any of the substituents shown in Table 1A to Table 3A (hereinafter, referred to as compound group SX499).

A compound (L-39) in which $R^{2a}$ is an ethyl group, $R^{2b}$ is a hydrogen atom, $R^{6c}$ is a fluorine atom, and $R^{6e}$ is any of the substituents shown in Table 1A to Table 3A (hereinafter, referred to as compound group SX500).

A compound (L-39) in which $R^{2a}$ and $R^{2b}$ are ethyl groups, $R^{6c}$ is a fluorine atom, and $R^{6e}$ is any of the substituents shown in Table 1A to Table 3A (hereinafter, referred to as compound group SX501).

A compound (L-39) in which $R^{2a}$ is a propyl group, $R^{2b}$ is a hydrogen atom, $R^{6c}$ is a fluorine atom, and $R^{6e}$ is any of the substituents shown in Table 1A to Table 3A (hereinafter, referred to as compound group SX502).

A compound (L-39) in which $R^{2a}$ and $R^{2b}$ are propyl groups, $R^{6c}$ is a fluorine atom, and $R^{6e}$ is any of the substituents shown in Table 1A to Table 3A (hereinafter, referred to as compound group SX503).

A compound (L-39) in which $R^{2a}$ is a methyl group, $R^{2b}$ is an ethyl group, $R^{6c}$ is a fluorine atom, and $R^{6e}$ is any of the substituents shown in Table 1A to Table 3A (hereinafter, referred to as compound group SX504).

A compound (L-39) in which $R^{2a}$ and $R^{2b}$ are hydrogen atoms, $R^{6c}$ is a chlorine atom, and $R^{6e}$ is any of the substituents shown in Table 1A to Table 3A (hereinafter, referred to as compound group SX505).

A compound (L-39) in which $R^{2a}$ is a methyl group, $R^{2b}$ is a hydrogen atom, $R^{6c}$ is a chlorine atom, and $R^{6e}$ is any of the substituents shown in Table 1A to Table 3A (hereinafter, referred to as compound group SX506).

A compound (L-39) in which $R^{2a}$ and $R^{2b}$ are methyl groups, $R^{6c}$ is a chlorine atom, and $R^{6e}$ is any of the substituents shown in Table 1A to Table 3A (hereinafter, referred to as compound group SX507).

A compound (L-39) in which $R^{2a}$ is an ethyl group, $R^{2b}$ is a hydrogen atom, $R^{6c}$ is a chlorine atom, and $R^{6e}$ is any of the substituents shown in Table 1A to Table 3A (hereinafter, referred to as compound group SX508).

A compound (L-39) in which $R^{2a}$ and $R^{2b}$ are ethyl groups, $R^{6c}$ is a chlorine atom, and $R^{6e}$ is any of the substituents shown in Table 1A to Table 3A (hereinafter, referred to as compound group SX509).

A compound (L-39) in which $R^{2a}$ is a propyl group, $R^{2b}$ is a hydrogen atom, $R^{6c}$ is a chlorine atom, and $R^{6e}$ is any of the substituents shown in Table 1A to Table 3A (hereinafter, referred to as compound group SX510).

A compound (L-39) in which $R^{2a}$ and $R^{2b}$ are propyl groups, $R^{6c}$ is a chlorine atom, and $R^{6e}$ is any of the substituents shown in Table 1A to Table 3A (hereinafter, referred to as compound group SX511).

A compound (L-39) in which $R^{2a}$ is a methyl group, $R^{2b}$ is an ethyl group, $R^{6c}$ is a chlorine atom, and $R^{6e}$ is any of the substituents shown in Table 1A to Table 3A (hereinafter, referred to as compound group SX512).

A compound (L-39) in which $R^{2a}$ and $R^{2b}$ are hydrogen atoms, $R^{6c}$ is a bromine atom, and $R^{6e}$ is any of the substituents shown in Table 1A to Table 3A (hereinafter, referred to as compound group SX513).

A compound (L-39) in which $R^{2a}$ is a methyl group, $R^{2b}$ is a hydrogen atom, $R^{6c}$ is a bromine atom, and $R^{6e}$ is any of the substituents shown in Table 1A to Table 3A (hereinafter, referred to as compound group SX514).

A compound (L-39) in which $R^{2a}$ and $R^{2b}$ are methyl groups, $R^{6c}$ is a bromine atom, and $R^{6e}$ is any of the substituents shown in Table 1A to Table 3A (hereinafter, referred to as compound group SX515).

A compound (L-39) in which $R^{2a}$ is an ethyl group, $R^{2b}$ is a hydrogen atom, $R^{6c}$ is a bromine atom, and $R^{6e}$ is any of the substituents shown in Table 1A to Table 3A (hereinafter, referred to as compound group SX516).

A compound (L-39) in which $R^{2a}$ and $R^{2b}$ are ethyl groups, $R^{6c}$ is a bromine atom, and $R^{6e}$ is any of the substituents shown in Table 1A to Table 3A (hereinafter, referred to as compound group SX517).

A compound (L-39) in which $R^{2a}$ is a propyl group, $R^{2b}$ is a hydrogen atom, $R^{6c}$ is a bromine atom, and $R^{6e}$ is any of the substituents shown in Table 1A to Table 3A (hereinafter, referred to as compound group SX518).

A compound (L-39) in which $R^{2a}$ and $R^{2b}$ are propyl groups, $R^{6c}$ is a bromine atom, and $R^{6e}$ is any of the substituents shown in Table 1A to Table 3A (hereinafter, referred to as compound group SX519).

A compound (L-39) in which $R^{2a}$ is a methyl group, $R^{2b}$ is an ethyl group, $R^{6c}$ is a bromine atom, and $R^{6e}$ is any of the substituents shown in Table 1A to Table 3A (hereinafter, referred to as compound group SX520).

A compound (L-39) in which $R^{2a}$ and $R^{2b}$ are hydrogen atoms, $R^{6c}$ is an iodine atom, and $R^{6e}$ is any of the substituents shown in Table 1A to Table 3A (hereinafter, referred to as compound group SX521).

A compound (L-39) in which $R^{2a}$ is a methyl group, $R^{2b}$ is a hydrogen atom, $R^{6c}$ is an iodine atom, and $R^{6e}$ is any of the substituents shown in Table 1A to Table 3A (hereinafter, referred to as compound group SX522).

A compound (L-39) in which $R^{2a}$ and $R^{2b}$ are methyl groups, $R^{6c}$ is an iodine atom, and $R^{6e}$ is any of the substituents shown in Table 1A to Table 3A (hereinafter, referred to as compound group SX523).

A compound (L-39) in which $R^{2a}$ is an ethyl group, $R^{2b}$ is a hydrogen atom, $R^{6c}$ is an iodine atom, and $R^{6e}$ is any of the substituents shown in Table 1A to Table 3A (hereinafter, referred to as compound group SX524).

A compound (L-39) in which $R^{2a}$ and $R^{2b}$ are ethyl groups, $R^{6c}$ is an iodine atom, and $R^{6e}$ is any of the substituents shown in Table 1A to Table 3A (hereinafter, referred to as compound group SX525).

A compound (L-39) in which $R^{2a}$ is a propyl group, $R^{2b}$ is a hydrogen atom, $R^{6c}$ is an iodine atom, and $R^{6e}$ is any of the substituents shown in Table 1A to Table 3A (hereinafter, referred to as compound group SX526).

A compound (L-39) in which $R^{2a}$ and $R^{2b}$ are propyl groups, $R^{6c}$ is an iodine atom, and $R^{6e}$ is any of the substituents shown in Table 1A to Table 3A (hereinafter, referred to as compound group SX527).

A compound (L-39) in which $R^{2a}$ is a methyl group, $R^{2b}$ is an ethyl group, $R^{6c}$ is an iodine atom, and $R^{6e}$ is any of the substituents shown in Table 1A to Table 3A (hereinafter, referred to as compound group SX528).

A compound represented by formula (L-40):

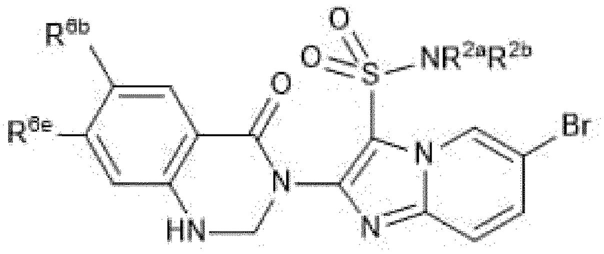

(L-40)

(hereinafter, referred to as compound (L-40)) in which $R^{2a}$ and $R^{2b}$ are hydrogen atoms, $R^{6b}$ is a fluorine atom, and $R^{6e}$ is any of the substituents shown in Table 1A to Table 3A (hereinafter, referred to as compound group SX529).

A compound (L-40) in which $R^{2a}$ is a methyl group, $R^{2b}$ is a hydrogen atom, $R^{6b}$ is a fluorine atom, and $R^{6e}$ is any of the substituents shown in Table 1A to Table 3A (hereinafter, referred to as compound group SX530).

A compound (L-40) in which $R^{2a}$ and $R^{2b}$ are methyl groups, $R^{6b}$ is a fluorine atom, and $R^{6e}$ is any of the substituents shown in Table 1A to Table 3A (hereinafter, referred to as compound group SX531).

A compound (L-40) in which $R^{2a}$ is an ethyl group, $R^{2b}$ is a hydrogen atom, $R^{6b}$ is a fluorine atom, and $R^{6e}$ is any of the substituents shown in Table 1A to Table 3A (hereinafter, referred to as compound group SX532).

A compound (L-40) in which $R^{2a}$ and $R^{2b}$ are ethyl groups, $R^{6b}$ is a fluorine atom, and $R^{6e}$ is any of the substituents shown in Table 1A to Table 3A (hereinafter, referred to as compound group SX533).

A compound (L-40) in which $R^{2a}$ is a propyl group, $R^{2b}$ is a hydrogen atom, $R^{6b}$ is a fluorine atom, and $R^{6e}$ is any of the substituents shown in Table 1A to Table 3A (hereinafter, referred to as compound group SX534).

A compound (L-40) in which $R^{2a}$ and $R^{2b}$ are propyl groups, $R^{6b}$ is a fluorine atom, and $R^{6e}$ is any of the substituents shown in Table 1A to Table 3A (hereinafter, referred to as compound group SX535).

A compound (L-40) in which $R^{2a}$ is a methyl group, $R^{2b}$ is an ethyl group, $R^{6b}$ is a fluorine atom, and $R^{6e}$ is any of the substituents shown in Table 1A to Table 3A (hereinafter, referred to as compound group SX536).

A compound (L-40) in which $R^{2a}$ and $R^{2b}$ are hydrogen atoms, $R^{6b}$ is a chlorine atom, and $R^{6e}$ is any of the substituents shown in Table 1A to Table 3A (hereinafter, referred to as compound group SX537).

A compound (L-40) in which $R^{2a}$ is a methyl group, $R^{2b}$ is a hydrogen atom, $R^{6b}$ is a chlorine atom, and $R^{6e}$ is any of the substituents shown in Table 1A to Table 3A (hereinafter, referred to as compound group SX538).

A compound (L-40) in which $R^{2a}$ and $R^{2b}$ are methyl groups, $R^{6b}$ is a chlorine atom, and $R^{6e}$ is any of the

substituents shown in Table 1A to Table 3A (hereinafter, referred to as compound group SX539).

A compound (L-40) in which $R^{2a}$ is an ethyl group, $R^{2b}$ is a hydrogen atom, $R^{6b}$ is a chlorine atom, and $R^{6e}$ is any of the substituents shown in Table 1A to Table 3A (hereinafter, referred to as compound group SX540).

A compound (L-40) in which $R^{2a}$ and $R^{2b}$ are ethyl groups, $R^{6b}$ is a chlorine atom, and $R^{6e}$ is any of the substituents shown in Table 1A to Table 3A (hereinafter, referred to as compound group SX541).

A compound (L-40) in which $R^{2a}$ is a propyl group, $R^{2b}$ is a hydrogen atom, $R^{6b}$ is a chlorine atom, and $R^{6e}$ is any of the substituents shown in Table 1A to Table 3A (hereinafter, referred to as compound group SX542).

A compound (L-40) in which $R^{2a}$ and $R^{2b}$ are propyl groups, $R^{6b}$ is a chlorine atom, and $R^{6e}$ is any of the substituents shown in Table 1A to Table 3A (hereinafter, referred to as compound group SX543).

A compound (L-40) in which $R^{2a}$ is a methyl group, $R^{2b}$ is an ethyl group, $R^{6b}$ is a chlorine atom, and $R^{6e}$ is any of the substituents shown in Table 1A to Table 3A (hereinafter, referred to as compound group SX544).

A compound (L-40) in which $R^{2a}$ and $R^{2b}$ are hydrogen atoms, $R^{6b}$ is a bromine atom, and $R^{6e}$ is any of the substituents shown in Table 1A to Table 3A (hereinafter, referred to as compound group SX545).

A compound (L-40) in which $R^{2a}$ is a methyl group, $R^{2b}$ is a hydrogen atom, $R^{6b}$ is a bromine atom, and $R^{6e}$ is any of the substituents shown in Table 1A to Table 3A (hereinafter, referred to as compound group SX546).

A compound (L-40) in which $R^{2a}$ and $R^{2b}$ are methyl groups, $R^{6b}$ is a bromine atom, and $R^{6e}$ is any of the substituents shown in Table 1A to Table 3A (hereinafter, referred to as compound group SX547).

A compound (L-40) in which $R^{2a}$ is an ethyl group, $R^{2b}$ is a hydrogen atom, $R^{6b}$ is a bromine atom, and $R^{6e}$ is any of the substituents shown in Table 1A to Table 3A (hereinafter, referred to as compound group SX548).

A compound (L-40) in which $R^{2a}$ and $R^{2b}$ are ethyl groups, $R^{6b}$ is a bromine atom, and $R^{6e}$ is any of the substituents shown in Table 1A to Table 3A (hereinafter, referred to as compound group SX549).

A compound (L-40) in which $R^{2a}$ is a propyl group, $R^{2b}$ is a hydrogen atom, $R^{6b}$ is a bromine atom, and $R^{6e}$ is any of the substituents shown in Table 1A to Table 3A (hereinafter, referred to as compound group SX550).

A compound (L-40) in which $R^{2a}$ and $R^{2b}$ are propyl groups, $R^{6b}$ is a bromine atom, and $R^{6e}$ is any of the substituents shown in Table 1A to Table 3A (hereinafter, referred to as compound group SX551).

A compound (L-40) in which $R^{2a}$ is a methyl group, $R^{2b}$ is an ethyl group, $R^{6b}$ is a bromine atom, and $R^{6e}$ is any of the substituents shown in Table 1A to Table 3A (hereinafter, referred to as compound group SX552).

A compound (L-40) in which $R^{2a}$ and $R^{2b}$ are hydrogen atoms, $R^{6b}$ is an iodine atom, and $R^{6e}$ is any of the substituents shown in Table 1A to Table 3A (hereinafter, referred to as compound group SX553).

A compound (L-40) in which $R^{2a}$ is a methyl group, $R^{2b}$ is a hydrogen atom, $R^{6b}$ is an iodine atom, and $R^{6e}$ is any of the substituents shown in Table 1A to Table 3A (hereinafter, referred to as compound group SX554).

A compound (L-40) in which $R^{2a}$ and $R^{2b}$ are methyl groups, $R^{6b}$ is an iodine atom, and $R^{6e}$ is any of the substituents shown in Table 1A to Table 3A (hereinafter, referred to as compound group SX555).

A compound (L-40) in which $R^{2a}$ is an ethyl group, $R^{2b}$ is a hydrogen atom, $R^{6b}$ is an iodine atom, and $R^{6e}$ is any of the substituents shown in Table 1A to Table 3A (hereinafter, referred to as compound group SX556).

A compound (L-40) in which $R^{2a}$ and $R^{2b}$ are ethyl groups, $R^{6b}$ is an iodine atom, and $R^{6e}$ is any of the substituents shown in Table 1A to Table 3A (hereinafter, referred to as compound group SX557).

A compound (L-40) in which $R^{2a}$ is a propyl group, $R^{2b}$ is a hydrogen atom, $R^{6b}$ is an iodine atom, and $R^{6e}$ is any of the substituents shown in Table 1A to Table 3A (hereinafter, referred to as compound group SX558).

A compound (L-40) in which $R^{2a}$ and $R^{2b}$ are propyl groups, $R^{6b}$ is an iodine atom, and $R^{6e}$ is any of the substituents shown in Table 1A to Table 3A (hereinafter, referred to as compound group SX559).

A compound (L-40) in which $R^{2a}$ is a methyl group, $R^{2b}$ is an ethyl group, $R^{6b}$ is an iodine atom, and $R^{6e}$ is any of the substituents shown in Table 1A to Table 3A (hereinafter, referred to as compound group SX560).

A compound represented by formula (L-41):

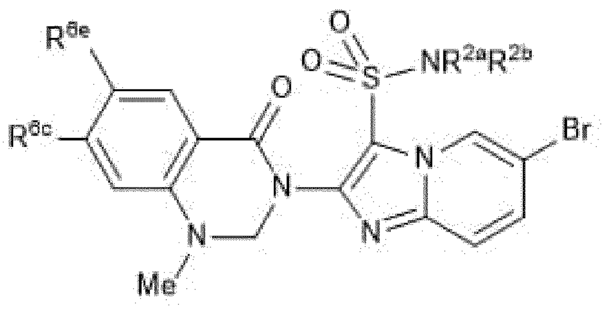

(L-41)

(hereinafter, referred to as compound (L-41)) in which $R^{2a}$ and $R^{2b}$ are hydrogen atoms, $R^{6c}$ is a fluorine atom, and $R^{6e}$ is any of the substituents shown in Table 1A to Table 3A (hereinafter, referred to as compound group SX561).

A compound (L-41) in which $R^{2a}$ is a methyl group, $R^{2b}$ is a hydrogen atom, $R^{6c}$ is a fluorine atom, and $R^{6e}$ is any of the substituents shown in Table 1A to Table 3A (hereinafter, referred to as compound group SX562).

A compound (L-41) in which $R^{2a}$ and $R^{2b}$ are methyl groups, $R^{6c}$ is a fluorine atom, and $R^{6e}$ is any of the substituents shown in Table 1A to Table 3A (hereinafter, referred to as compound group SX563).

A compound (L-41) in which $R^{2a}$ is an ethyl group, $R^{2b}$ is a hydrogen atom, $R^{6c}$ is a fluorine atom, and $R^{6e}$ is any of the substituents shown in Table 1A to Table 3A (hereinafter, referred to as compound group SX564).

A compound (L-41) in which $R^{2a}$ and $R^{2b}$ are ethyl groups, $R^{6c}$ is a fluorine atom, and $R^{6e}$ is any of the substituents shown in Table 1A to Table 3A (hereinafter, referred to as compound group SX565).

A compound (L-41) in which $R^{2a}$ is a propyl group, $R^{2b}$ is a hydrogen atom, $R^{6c}$ is a fluorine atom, and $R^{6e}$ is any of the substituents shown in Table 1A to Table 3A (hereinafter, referred to as compound group SX566).

A compound (L-41) in which $R^{2a}$ and $R^{2b}$ are propyl groups, $R^{6c}$ is a fluorine atom, and $R^{6e}$ is any of the substituents shown in Table 1A to Table 3A (hereinafter, referred to as compound group SX567).

A compound (L-41) in which $R^{2a}$ is a methyl group, $R^{2b}$ is an ethyl group, $R^{6c}$ is a fluorine atom, and $R^{6e}$ is any of the substituents shown in Table 1A to Table 3A (hereinafter, referred to as compound group SX568).

A compound (L-41) in which $R^{2a}$ and $R^{2b}$ are hydrogen atoms, $R^{6c}$ is a chlorine atom, and $R^{6e}$ is any of the substituents shown in Table 1A to Table 3A (hereinafter, referred to as compound group SX569).

A compound (L-41) in which $R^{2a}$ is a methyl group, $R^{2b}$ is a hydrogen atom, $R^{6c}$ is a chlorine atom, and $R^{6e}$ is any of the substituents shown in Table 1A to Table 3A (hereinafter, referred to as compound group SX570).

A compound (L-41) in which $R^{2a}$ and $R^{2b}$ are methyl groups, $R^{6c}$ is a chlorine atom, and $R^{6e}$ is any of the substituents shown in Table 1A to Table 3A (hereinafter, referred to as compound group SX571).

A compound (L-41) in which $R^{2a}$ is an ethyl group, $R^{2b}$ is a hydrogen atom, $R^{6c}$ is a chlorine atom, and $R^{6e}$ is any of the substituents shown in Table 1A to Table 3A (hereinafter, referred to as compound group SX572).

A compound (L-41) in which $R^{2a}$ and $R^{2b}$ are ethyl groups, $R^{6c}$ is a chlorine atom, and $R^{6e}$ is any of the substituents shown in Table 1A to Table 3A (hereinafter, referred to as compound group SX573).

A compound (L-41) in which $R^{2a}$ is a propyl group, $R^{2b}$ is a hydrogen atom, $R^{6c}$ is a chlorine atom, and $R^{6e}$ is any of the substituents shown in Table 1A to Table 3A (hereinafter, referred to as compound group SX574).

A compound (L-41) in which $R^{2a}$ and $R^{2b}$ are propyl groups, $R^{6c}$ is a chlorine atom, and $R^{6e}$ is any of the substituents shown in Table 1A to Table 3A (hereinafter, referred to as compound group SX575).

A compound (L-41) in which $R^{2a}$ is a methyl group, $R^{2b}$ is an ethyl group, $R^{6c}$ is a chlorine atom, and $R^{6e}$ is any of the substituents shown in Table 1A to Table 3A (hereinafter, referred to as compound group SX576).

A compound (L-41) in which $R^{2a}$ and $R^{2b}$ are hydrogen atoms, $R^{6c}$ is a bromine atom, and $R^{6e}$ is any of the substituents shown in Table 1A to Table 3A (hereinafter, referred to as compound group SX577).

A compound (L-41) in which $R^{2a}$ is a methyl group, $R^{2b}$ is a hydrogen atom, $R^{6c}$ is a bromine atom, and $R^{6e}$ is any of the substituents shown in Table 1A to Table 3A (hereinafter, referred to as compound group SX578).

A compound (L-41) in which $R^{2a}$ and $R^{2b}$ are methyl groups, $R^{6c}$ is a bromine atom, and $R^{6e}$ is any of the substituents shown in Table 1A to Table 3A (hereinafter, referred to as compound group SX579).

A compound (L-41) in which $R^{2a}$ is an ethyl group, $R^{2b}$ is a hydrogen atom, $R^{6c}$ is a bromine atom, and $R^{6e}$ is any of the substituents shown in Table 1A to Table 3A (hereinafter, referred to as compound group SX580).

A compound (L-41) in which $R^{2a}$ and $R^{2b}$ are ethyl groups, $R^{6c}$ is a bromine atom, and $R^{6e}$ is any of the substituents shown in Table 1A to Table 3A (hereinafter, referred to as compound group SX581).

A compound (L-41) in which $R^{2a}$ is a propyl group, $R^{2b}$ is a hydrogen atom, $R^{6c}$ is a bromine atom, and $R^{6e}$ is any of the substituents shown in Table 1A to Table 3A (hereinafter, referred to as compound group SX582).

A compound (L-41) in which $R^{2a}$ and $R^{2b}$ are propyl groups, $R^{6c}$ is a bromine atom, and $R^{6e}$ is any of the substituents shown in Table 1A to Table 3A (hereinafter, referred to as compound group SX583).

A compound (L-41) in which $R^{2a}$ is a methyl group, $R^{2b}$ is an ethyl group, $R^{6c}$ is a bromine atom, and $R^{6e}$ is any of the substituents shown in Table 1A to Table 3A (hereinafter, referred to as compound group SX584).

A compound (L-41) in which $R^{2a}$ and $R^{2b}$ are hydrogen atoms, $R^{6c}$ is an iodine atom, and $R^{6e}$ is any of the substituents shown in Table 1A to Table 3A (hereinafter, referred to as compound group SX585).

A compound (L-41) in which $R^{2a}$ is a methyl group, $R^{2b}$ is a hydrogen atom, $R^{6c}$ is an iodine atom, and $R^{6e}$ is any of the substituents shown in Table 1A to Table 3A (hereinafter, referred to as compound group SX586).

A compound (L-41) in which $R^{2a}$ and $R^{2b}$ are methyl groups, $R^{6c}$ is an iodine atom, and $R^{6e}$ is any of the substituents shown in Table 1A to Table 3A (hereinafter, referred to as compound group SX587).

A compound (L-41) in which $R^{2a}$ is an ethyl group, $R^{2b}$ is a hydrogen atom, $R^{6c}$ is an iodine atom, and $R^{6e}$ is any of the substituents shown in Table 1A to Table 3A (hereinafter, referred to as compound group SX588).

A compound (L-41) in which $R^{2a}$ and $R^{2b}$ are ethyl groups, $R^{6c}$ is an iodine atom, and $R^{6e}$ is any of the substituents shown in Table 1A to Table 3A (hereinafter, referred to as compound group SX589).

A compound (L-41) in which $R^{2a}$ is a propyl group, $R^{2b}$ is a hydrogen atom, $R^{6c}$ is an iodine atom, and $R^{6e}$ is any of the substituents shown in Table 1A to Table 3A (hereinafter, referred to as compound group SX590).

A compound (L-41) in which $R^{2a}$ and $R^{2b}$ are propyl groups, $R^{6c}$ is an iodine atom, and $R^{6e}$ is any of the substituents shown in Table 1A to Table 3A (hereinafter, referred to as compound group SX591).

A compound (L-41) in which $R^{2a}$ is a methyl group, $R^{2b}$ is an ethyl group, $R^{6c}$ is an iodine atom, and $R^{6e}$ is any of the substituents shown in Table 1A to Table 3A (hereinafter, referred to as compound group SX592).

A compound represented by formula (L-42):

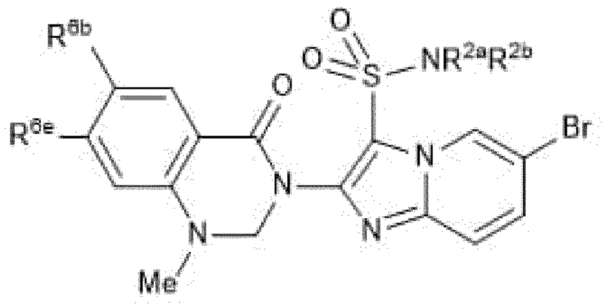

(L-42)

(hereinafter, referred to as compound (L-42)) in which $R^{2a}$ and $R^{2b}$ are hydrogen atoms, $R^{6b}$ is a fluorine atom, and $R^{6e}$ is any of the substituents shown in Table 1A to Table 3A (hereinafter, referred to as compound group SX593).

A compound (L-42) in which $R^{2a}$ is a methyl group, $R^{2b}$ is a hydrogen atom, $R^{6b}$ is a fluorine atom, and $R^{6e}$ is any of the substituents shown in Table 1A to Table 3A (hereinafter, referred to as compound group SX594).

A compound (L-42) in which $R^{2a}$ and $R^{2b}$ are methyl groups, $R^{6b}$ is a fluorine atom, and $R^{6e}$ is any of the substituents shown in Table 1A to Table 3A (hereinafter, referred to as compound group SX595).

A compound (L-42) in which $R^{2a}$ is an ethyl group, $R^{2b}$ is a hydrogen atom, $R^{6b}$ is a fluorine atom, and $R^{6e}$ is any of the substituents shown in Table 1A to Table 3A (hereinafter, referred to as compound group SX596).

A compound (L-42) in which $R^{2a}$ and $R^{2b}$ are ethyl groups, $R^{6b}$ is a fluorine atom, and $R^{6e}$ is any of the substituents shown in Table 1A to Table 3A (hereinafter, referred to as compound group SX597).

A compound (L-42) in which $R^{2a}$ is a propyl group, $R^{2b}$ is a hydrogen atom, $R^{6b}$ is a fluorine atom, and $R^{6e}$ is any of the substituents shown in Table 1A to Table 3A (hereinafter, referred to as compound group SX598).

A compound (L-42) in which $R^{2a}$ and $R^{2b}$ are propyl groups, $R^{6b}$ is a fluorine atom, and $R^{6e}$ is any of the substituents shown in Table 1A to Table 3A (hereinafter, referred to as compound group SX599).

A compound (L-42) in which $R^{2a}$ is a methyl group, $R^{2b}$ is an ethyl group, $R^{6b}$ is a fluorine atom, and $R^{6e}$ is any of the substituents shown in Table 1A to Table 3A (hereinafter, referred to as compound group SX600).

A compound (L-42) in which $R^{2a}$ and $R^{2b}$ are hydrogen atoms, $R^{6b}$ is a chlorine atom, and $R^{6e}$ is any of the substituents shown in Table 1A to Table 3A (hereinafter, referred to as compound group SX601).

A compound (L-42) in which $R^{2a}$ is a methyl group, $R^{2b}$ is a hydrogen atom, $R^{6b}$ is a chlorine atom, and $R^{6e}$ is any of the substituents shown in Table 1A to Table 3A (hereinafter, referred to as compound group SX602).

A compound (L-42) in which $R^{2a}$ and $R^{2b}$ are methyl groups, $R^{6b}$ is a chlorine atom, and $R^{6e}$ is any of the substituents shown in Table 1A to Table 3A (hereinafter, referred to as compound group SX603).

A compound (L-42) in which $R^{2a}$ is an ethyl group, $R^{2b}$ is a hydrogen atom, $R^{6b}$ is a chlorine atom, and $R^{6e}$ is any of the substituents shown in Table 1A to Table 3A (hereinafter, referred to as compound group SX604).

A compound (L-42) in which $R^{2a}$ and $R^{2b}$ are ethyl groups, $R^{6b}$ is a chlorine atom, and $R^{6e}$ is any of the substituents shown in Table 1A to Table 3A (hereinafter, referred to as compound group SX605).

A compound (L-42) in which $R^{2a}$ is a propyl group, $R^{2b}$ is a hydrogen atom, $R^{6b}$ is a chlorine atom, and $R^{6e}$ is any of the substituents shown in Table 1A to Table 3A (hereinafter, referred to as compound group SX606).

A compound (L-42) in which $R^{2a}$ and $R^{2b}$ are propyl groups, $R^{6b}$ is a chlorine atom, and $R^{6e}$ is any of the substituents shown in Table 1A to Table 3A (hereinafter, referred to as compound group SX607).

A compound (L-42) in which $R^{2a}$ is a methyl group, $R^{2b}$ is an ethyl group, $R^{6b}$ is a chlorine atom, and $R^{6e}$ is any of the substituents shown in Table 1A to Table 3A (hereinafter, referred to as compound group SX608).

A compound (L-42) in which $R^{2a}$ and $R^{2b}$ are hydrogen atoms, $R^{6b}$ is a bromine atom, and $R^{6e}$ is any of the substituents shown in Table 1A to Table 3A (hereinafter, referred to as compound group SX609).

A compound (L-42) in which $R^{2a}$ is a methyl group, $R^{2b}$ is a hydrogen atom, $R^{6b}$ is a bromine atom, and $R^{6e}$ is any of the substituents shown in Table 1A to Table 3A (hereinafter, referred to as compound group SX610).

A compound (L-42) in which $R^{2a}$ and $R^{2b}$ are methyl groups, $R^{6b}$ is a bromine atom, and $R^{6e}$ is any of the substituents shown in Table 1A to Table 3A (hereinafter, referred to as compound group SX611).

A compound (L-42) in which $R^{2a}$ is an ethyl group, $R^{2b}$ is a hydrogen atom, $R^{6b}$ is a bromine atom, and $R^{6e}$ is any of the substituents shown in Table 1A to Table 3A (hereinafter, referred to as compound group SX612).

A compound (L-42) in which $R^{2a}$ and $R^{2b}$ are ethyl groups, $R^{6b}$ is a bromine atom, and $R^{6e}$ is any of the

substituents shown in Table 1A to Table 3A (hereinafter, referred to as compound group SX613).

A compound (L-42) in which $R^{2a}$ is a propyl group, $R^{2b}$ is a hydrogen atom, $R^{6b}$ is a bromine atom, and $R^{6e}$ is any of the substituents shown in Table 1A to Table 3A (hereinafter, referred to as compound group SX614).

A compound (L-42) in which $R^{2a}$ and $R^{2b}$ are propyl groups, $R^{6b}$ is a bromine atom, and $R^{6e}$ is any of the substituents shown in Table 1A to Table 3A (hereinafter, referred to as compound group SX615).

A compound (L-42) in which $R^{2a}$ is a methyl group, $R^{2b}$ is an ethyl group, $R^{6b}$ is a bromine atom, and $R^{6e}$ is any of the substituents shown in Table 1A to Table 3A (hereinafter, referred to as compound group SX616).

A compound (L-42) in which $R^{2a}$ and $R^{2b}$ are hydrogen atoms, $R^{6b}$ is an iodine atom, and $R^{6e}$ is any of the substituents shown in Table 1A to Table 3A (hereinafter, referred to as compound group SX617).

A compound (L-42) in which $R^{2a}$ is a methyl group, $R^{2b}$ is a hydrogen atom, $R^{6b}$ is an iodine atom, and $R^{6e}$ is any of the substituents shown in Table 1A to Table 3A (hereinafter, referred to as compound group SX618).

A compound (L-42) in which $R^{2a}$ and $R^{2b}$ are methyl groups, $R^{6b}$ is an iodine atom, and $R^{6e}$ is any of the substituents shown in Table 1A to Table 3A (hereinafter, referred to as compound group SX619).

A compound (L-42) in which $R^{2a}$ is an ethyl group, $R^{2b}$ is a hydrogen atom, $R^{6b}$ is an iodine atom, and $R^{6e}$ is any of the substituents shown in Table 1A to Table 3A (hereinafter, referred to as compound group SX620).

A compound (L-42) in which $R^{2a}$ and $R^{2b}$ are ethyl groups, $R^{6b}$ is an iodine atom, and $R^{6e}$ is any of the substituents shown in Table 1A to Table 3A (hereinafter, referred to as compound group SX621).

A compound (L-42) in which $R^{2a}$ is a propyl group, $R^{2b}$ is a hydrogen atom, $R^{6b}$ is an iodine atom, and $R^{6e}$ is any of the substituents shown in Table 1A to Table 3A (hereinafter, referred to as compound group SX622).

A compound (L-42) in which $R^{2a}$ and $R^{2b}$ are propyl groups, $R^{6b}$ is an iodine atom, and $R^{6e}$ is any of the substituents shown in Table 1A to Table 3A (hereinafter, referred to as compound group SX623).

A compound (L-42) in which $R^{2a}$ is a methyl group, $R^{2b}$ is an ethyl group, $R^{6b}$ is an iodine atom, and $R^{6e}$ is any of the substituents shown in Table 1A to Table 3A (hereinafter, referred to as compound group SX624).

A compound represented by formula (L-43):

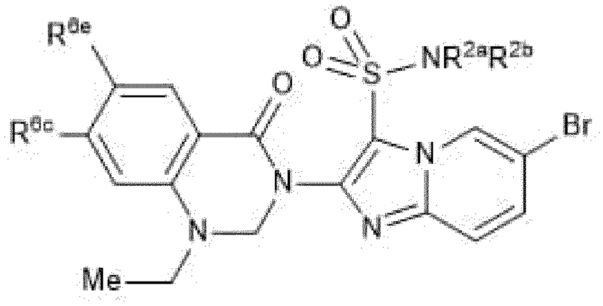

(L-43)

(hereinafter, referred to as compound (L-43)) in which $R^{2a}$ and $R^{2b}$ are hydrogen atoms, $R^{6c}$ is a fluorine atom, and $R^{6e}$ is any of the substituents shown in Table 1A to Table 3A (hereinafter, referred to as compound group SX625).

A compound (L-43) in which $R^{2a}$ is a methyl group, $R^{2b}$ is a hydrogen atom, $R^{6c}$ is a fluorine atom, and $R^{6e}$ is any of the substituents shown in Table 1A to Table 3A (hereinafter, referred to as compound group SX626).

A compound (L-43) in which $R^{2a}$ and $R^{2b}$ are methyl groups, $R^{6c}$ is a fluorine atom, and $R^{6e}$ is any of the substituents shown in Table 1A to Table 3A (hereinafter, referred to as compound group SX627).

A compound (L-43) in which $R^{2a}$ is an ethyl group, $R^{2b}$ is a hydrogen atom, $R^{6c}$ is a fluorine atom, and $R^{6e}$ is any of the substituents shown in Table 1A to Table 3A (hereinafter, referred to as compound group SX628).

A compound (L-43) in which $R^{2a}$ and $R^{2b}$ are ethyl groups, $R^{6c}$ is a fluorine atom, and $R^{6e}$ is any of the substituents shown in Table 1A to Table 3A (hereinafter, referred to as compound group SX629).

A compound (L-43) in which $R^{2a}$ is a propyl group, $R^{2b}$ is a hydrogen atom, $R^{6c}$ is a fluorine atom, and $R^{6e}$ is any of the substituents shown in Table 1A to Table 3A (hereinafter, referred to as compound group SX630).

A compound (L-43) in which $R^{2a}$ and $R^{2b}$ are propyl groups, $R^{6c}$ is a fluorine atom, and $R^{6e}$ is any of the substituents shown in Table 1A to Table 3A (hereinafter, referred to as compound group SX631).

A compound (L-43) in which $R^{2a}$ is a methyl group, $R^{2b}$ is an ethyl group, $R^{6c}$ is a fluorine atom, and $R^{6e}$ is any of the substituents shown in Table 1A to Table 3A (hereinafter, referred to as compound group SX632).

A compound (L-43) in which $R^{2a}$ and $R^{2b}$ are hydrogen atoms, $R^{6c}$ is a chlorine atom, and $R^{6e}$ is any of the substituents shown in Table 1A to Table 3A (hereinafter, referred to as compound group SX633).

A compound (L-43) in which $R^{2a}$ is a methyl group, $R^{2b}$ is a hydrogen atom, $R^{6c}$ is a chlorine atom, and $R^{6e}$ is any of the substituents shown in Table 1A to Table 3A (hereinafter, referred to as compound group SX634).

A compound (L-43) in which $R^{2a}$ and $R^{2b}$ are methyl groups, $R^{6c}$ is a chlorine atom, and $R^{6e}$ is any of the substituents shown in Table 1A to Table 3A (hereinafter, referred to as compound group SX635).

A compound (L-43) in which $R^{2a}$ is an ethyl group, $R^{2b}$ is a hydrogen atom, $R^{6c}$ is a chlorine atom, and $R^{6e}$ is any of the substituents shown in Table 1A to Table 3A (hereinafter, referred to as compound group SX636).

A compound (L-43) in which $R^{2a}$ and $R^{2b}$ are ethyl groups, $R^{6c}$ is a chlorine atom, and $R^{6e}$ is any of the substituents shown in Table 1A to Table 3A (hereinafter, referred to as compound group SX637).

A compound (L-43) in which $R^{2a}$ is a propyl group, $R^{2b}$ is a hydrogen atom, $R^{6c}$ is a chlorine atom, and $R^{6e}$ is any of the substituents shown in Table 1A to Table 3A (hereinafter, referred to as compound group SX638).

A compound (L-43) in which $R^{2a}$ and $R^{2b}$ are propyl groups, $R^{6c}$ is a chlorine atom, and $R^{6e}$ is any of the substituents shown in Table 1A to Table 3A (hereinafter, referred to as compound group SX639).

A compound (L-43) in which $R^{2a}$ is a methyl group, $R^{2b}$ is an ethyl group, $R^{6c}$ is a chlorine atom, and $R^{6e}$ is any of the substituents shown in Table 1A to Table 3A (hereinafter, referred to as compound group SX640).

A compound (L-43) in which $R^{2a}$ and $R^{2b}$ are hydrogen atoms, $R^{6c}$ is a bromine atom, and $R^{6e}$ is any of the substituents shown in Table 1A to Table 3A (hereinafter, referred to as compound group SX641).

A compound (L-43) in which $R^{2a}$ is a methyl group, $R^{2b}$ is a hydrogen atom, $R^{6c}$ is a bromine atom, and $R^{6e}$ is any of the substituents shown in Table 1A to Table 3A (hereinafter, referred to as compound group SX642).

A compound (L-43) in which $R^{2a}$ and $R^{2b}$ are methyl groups, $R^{6c}$ is a bromine atom, and $R^{6e}$ is any of the substituents shown in Table 1A to Table 3A (hereinafter, referred to as compound group SX643).

A compound (L-43) in which $R^{2a}$ is an ethyl group, $R^{2b}$ is a hydrogen atom, $R^{6c}$ is a bromine atom, and $R^{6e}$ is any of the substituents shown in Table 1A to Table 3A (hereinafter, referred to as compound group SX644).

A compound (L-43) in which $R^{2a}$ and $R^{2b}$ are ethyl groups, $R^{6c}$ is a bromine atom, and $R^{6e}$ is any of the substituents shown in Table 1A to Table 3A (hereinafter, referred to as compound group SX645).

A compound (L-43) in which $R^{2a}$ is a propyl group, $R^{2b}$ is a hydrogen atom, $R^{6c}$ is a bromine atom, and $R^{6e}$ is any of the substituents shown in Table 1A to Table 3A (hereinafter, referred to as compound group SX646).

A compound (L-43) in which $R^{2a}$ and $R^{2b}$ are propyl groups, $R^{6c}$ is a bromine atom, and $R^{6e}$ is any of the substituents shown in Table 1A to Table 3A (hereinafter, referred to as compound group SX647).

A compound (L-43) in which $R^{2a}$ is a methyl group, $R^{2b}$ is an ethyl group, $R^{6c}$ is a bromine atom, and $R^{6e}$ is any of the substituents shown in Table 1A to Table 3A (hereinafter, referred to as compound group SX648).

A compound (L-43) in which $R^{2a}$ and $R^{2b}$ are hydrogen atoms, $R^{6c}$ is an iodine atom, and $R^{6e}$ is any of the substituents shown in Table 1A to Table 3A (hereinafter, referred to as compound group SX649).

A compound (L-43) in which $R^{2a}$ is a methyl group, $R^{2b}$ is a hydrogen atom, $R^{6c}$ is an iodine atom, and $R^{6e}$ is any of the substituents shown in Table 1A to Table 3A (hereinafter, referred to as compound group SX650).

A compound (L-43) in which $R^{2a}$ and $R^{2b}$ are methyl groups, $R^{6c}$ is an iodine atom, and $R^{6e}$ is any of the substituents shown in Table 1A to Table 3A (hereinafter, referred to as compound group SX651).

A compound (L-43) in which $R^{2a}$ is an ethyl group, $R^{2b}$ is a hydrogen atom, $R^{6c}$ is an iodine atom, and $R^{6e}$ is any of the substituents shown in Table 1A to Table 3A (hereinafter, referred to as compound group SX652).

A compound (L-43) in which $R^{2a}$ and $R^{2b}$ are ethyl groups, $R^{6c}$ is an iodine atom, and $R^{6e}$ is any of the substituents shown in Table 1A to Table 3A (hereinafter, referred to as compound group SX653).

A compound (L-43) in which $R^{2a}$ is a propyl group, $R^{2b}$ is a hydrogen atom, $R^{6c}$ is an iodine atom, and $R^{6e}$ is any of the substituents shown in Table 1A to Table 3A (hereinafter, referred to as compound group SX654).

A compound (L-43) in which $R^{2a}$ and $R^{2b}$ are propyl groups, $R^{6c}$ is an iodine atom, and $R^{6e}$ is any of the substituents shown in Table 1A to Table 3A (hereinafter, referred to as compound group SX655).

A compound (L-43) in which $R^{2a}$ is a methyl group, $R^{2b}$ is an ethyl group, $R^{6c}$ is an iodine atom, and $R^{6e}$ is any of the substituents shown in Table 1A to Table 3A (hereinafter, referred to as compound group SX656).

A compound represented by formula (L-44):

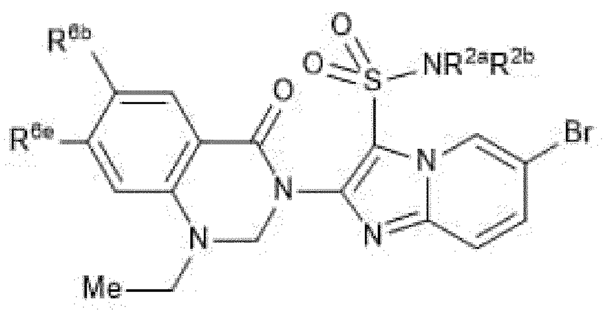

(L-44)

(hereinafter, referred to as compound (L-44)) in which $R^{2a}$ and $R^{2b}$ are hydrogen atoms, $R^{6b}$ is a fluorine atom, and $R^{6e}$ is any of the substituents shown in Table 1A to Table 3A (hereinafter, referred to as compound group SX657).

A compound (L-44) in which $R^{2a}$ is a methyl group, $R^{2b}$ is a hydrogen atom, $R^{6b}$ is a fluorine atom, and $R^{6e}$ is any of

the substituents shown in Table 1A to Table 3A (hereinafter, referred to as compound group SX658).

A compound (L-44) in which $R^{2a}$ and $R^{2b}$ are methyl groups, $R^{6b}$ is a fluorine atom, and $R^{6e}$ is any of the substituents shown in Table 1A to Table 3A (hereinafter, referred to as compound group SX659).

A compound (L-44) in which $R^{2a}$ is an ethyl group, $R^{2b}$ is a hydrogen atom, $R^{6b}$ is a fluorine atom, and $R^{6e}$ is any of the substituents shown in Table 1A to Table 3A (hereinafter, referred to as compound group SX660).

A compound (L-44) in which $R^{2a}$ and $R^{2b}$ are ethyl groups, $R^{6b}$ is a fluorine atom, and $R^{6e}$ is any of the substituents shown in Table 1A to Table 3A (hereinafter, referred to as compound group SX661).

A compound (L-44) in which $R^{2a}$ is a propyl group, $R^{2b}$ is a hydrogen atom, $R^{6b}$ is a fluorine atom, and $R^{6e}$ is any of the substituents shown in Table 1A to Table 3A (hereinafter, referred to as compound group SX662).

A compound (L-44) in which $R^{2a}$ and $R^{2b}$ are propyl groups, $R^{6b}$ is a fluorine atom, and $R^{6e}$ is any of the substituents shown in Table 1A to Table 3A (hereinafter, referred to as compound group SX663).

A compound (L-44) in which $R^{2a}$ is a methyl group, $R^{2b}$ is an ethyl group, $R^{6b}$ is a fluorine atom, and $R^{6e}$ is any of the substituents shown in Table 1A to Table 3A (hereinafter, referred to as compound group SX664).

A compound (L-44) in which $R^{2a}$ and $R^{2b}$ are hydrogen atoms, $R^{6b}$ is a chlorine atom, and $R^{6e}$ is any of the substituents shown in Table 1A to Table 3A (hereinafter, referred to as compound group SX665).

A compound (L-44) in which $R^{2a}$ is a methyl group, $R^{2b}$ is a hydrogen atom, $R^{6b}$ is a chlorine atom, and $R^{6e}$ is any of the substituents shown in Table 1A to Table 3A (hereinafter, referred to as compound group SX666).

A compound (L-44) in which $R^{2a}$ and $R^{2b}$ are methyl groups, $R^{6b}$ is a chlorine atom, and $R^{6e}$ is any of the substituents shown in Table 1A to Table 3A (hereinafter, referred to as compound group SX667).

A compound (L-44) in which $R^{2a}$ is an ethyl group, $R^{2b}$ is a hydrogen atom, $R^{6b}$ is a chlorine atom, and $R^{6e}$ is any of the substituents shown in Table 1A to Table 3A (hereinafter, referred to as compound group SX668).

A compound (L-44) in which $R^{2a}$ and $R^{2b}$ are ethyl groups, $R^{6b}$ is a chlorine atom, and $R^{6e}$ is any of the substituents shown in Table 1A to Table 3A (hereinafter, referred to as compound group SX669).

A compound (L-44) in which $R^{2a}$ is a propyl group, $R^{2b}$ is a hydrogen atom, $R^{6b}$ is a chlorine atom, and $R^{6e}$ is any of the substituents shown in Table 1A to Table 3A (hereinafter, referred to as compound group SX670).

A compound (L-44) in which $R^{2a}$ and $R^{2b}$ are propyl groups, $R^{6b}$ is a chlorine atom, and $R^{6e}$ is any of the substituents shown in Table 1A to Table 3A (hereinafter, referred to as compound group SX671).

A compound (L-44) in which $R^{2a}$ is a methyl group, $R^{2b}$ is an ethyl group, $R^{6b}$ is a chlorine atom, and $R^{6e}$ is any of the substituents shown in Table 1A to Table 3A (hereinafter, referred to as compound group SX672).

A compound (L-44) in which $R^{2a}$ and $R^{2b}$ are hydrogen atoms, $R^{6b}$ is a bromine atom, and $R^{6e}$ is any of the substituents shown in Table 1A to Table 3A (hereinafter, referred to as compound group SX673).

A compound (L-44) in which $R^{2a}$ is a methyl group, $R^{2b}$ is a hydrogen atom, $R^{6b}$ is a bromine atom, and $R^{6e}$ is any of the substituents shown in Table 1A to Table 3A (hereinafter, referred to as compound group SX674).

A compound (L-44) in which $R^{2a}$ and $R^{2b}$ are methyl groups, $R^{6b}$ is a bromine atom, and $R^{6e}$ is any of the substituents shown in Table 1A to Table 3A (hereinafter, referred to as compound group SX675).

A compound (L-44) in which $R^{2a}$ is an ethyl group, $R^{2b}$ is a hydrogen atom, $R^{6b}$ is a bromine atom, and $R^{6e}$ is any of the substituents shown in Table 1A to Table 3A (hereinafter, referred to as compound group SX676).

A compound (L-44) in which $R^{2a}$ and $R^{2b}$ are ethyl groups, $R^{6b}$ is a bromine atom, and $R^{6e}$ is any of the substituents shown in Table 1A to Table 3A (hereinafter, referred to as compound group SX677).

A compound (L-44) in which $R^{2a}$ is a propyl group, $R^{2b}$ is a hydrogen atom, $R^{6b}$ is a bromine atom, and $R^{6e}$ is any of the substituents shown in Table 1A to Table 3A (hereinafter, referred to as compound group SX678).

A compound (L-44) in which $R^{2a}$ and $R^{2b}$ are propyl groups, $R^{6b}$ is a bromine atom, and $R^{6e}$ is any of the substituents shown in Table 1A to Table 3A (hereinafter, referred to as compound group SX679).

A compound (L-44) in which $R^{2a}$ is a methyl group, $R^{2b}$ is an ethyl group, $R^{6b}$ is a bromine atom, and $R^{6e}$ is any of the substituents shown in Table 1A to Table 3A (hereinafter, referred to as compound group SX680).

A compound (L-44) in which $R^{2a}$ and $R^{2b}$ are hydrogen atoms, $R^{6b}$ is an iodine atom, and $R^{6e}$ is any of the substituents shown in Table 1A to Table 3A (hereinafter, referred to as compound group SX681).

A compound (L-44) in which $R^{2a}$ is a methyl group, $R^{2b}$ is a hydrogen atom, $R^{6b}$ is an iodine atom, and $R^{6e}$ is any of the substituents shown in Table 1A to Table 3A (hereinafter, referred to as compound group SX682).

A compound (L-44) in which $R^{2a}$ and $R^{2b}$ are methyl groups, $R^{6b}$ is an iodine atom, and $R^{6e}$ is any of the substituents shown in Table 1A to Table 3A (hereinafter, referred to as compound group SX683).

A compound (L-44) in which $R^{2a}$ is an ethyl group, $R^{2b}$ is a hydrogen atom, $R^{6b}$ is an iodine atom, and $R^{6e}$ is any of the substituents shown in Table 1A to Table 3A (hereinafter, referred to as compound group SX684).

A compound (L-44) in which $R^{2a}$ and $R^{2b}$ are ethyl groups, $R^{6b}$ is an iodine atom, and $R^{6e}$ is any of the substituents shown in Table 1A to Table 3A (hereinafter, referred to as compound group SX685).

A compound (L-44) in which $R^{2a}$ is a propyl group, $R^{2b}$ is a hydrogen atom, $R^{6b}$ is an iodine atom, and $R^{6e}$ is any of the substituents shown in Table 1A to Table 3A (hereinafter, referred to as compound group SX686).

A compound (L-44) in which $R^{2a}$ and $R^{2b}$ are propyl groups, $R^{6b}$ is an iodine atom, and $R^{6e}$ is any of the

substituents shown in Table 1A to Table 3A (hereinafter, referred to as compound group SX687).

A compound (L-44) in which $R^{2a}$ is a methyl group, $R^{2b}$ is an ethyl group, $R^{6b}$ is an iodine atom, and $R^{6e}$ is any of the substituents shown in Table 1A to Table 3A (hereinafter, referred to as compound group SX688).

A compound represented by formula (L-45):

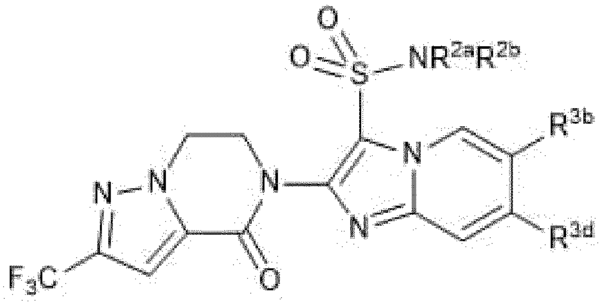

(L-45)

(hereinafter, referred to as compound (L-45)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX689) .

A compound (L-45) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX690).

A compound (L-45) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX691).

A compound (L-45) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX692).

A compound (L-45) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX693).

A compound (L-45) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX694).

A compound (L-45) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX695).

A compound (L-45) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX696).

A compound represented by formula (L-46):

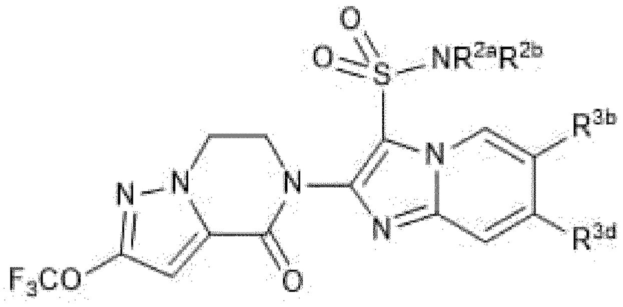

(L-46)

(hereinafter, referred to as compound (L-46)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX697).

A compound (L-46) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX698).

A compound (L-46) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX699).

A compound (L-46) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX700).

A compound (L-46) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX701).

A compound (L-46) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX702).

A compound (L-46) in which $R^{2a}$ and $R^{2b}$ are methyl groups, $R^{3b}$ is a hydrogen atom, and $R^{3d}$ is any of the

substituents shown in Table 7A to Table 15A (hereinafter, referred to as compound group SX703).

A compound (L-46) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX704).

A compound represented by formula (L-47):

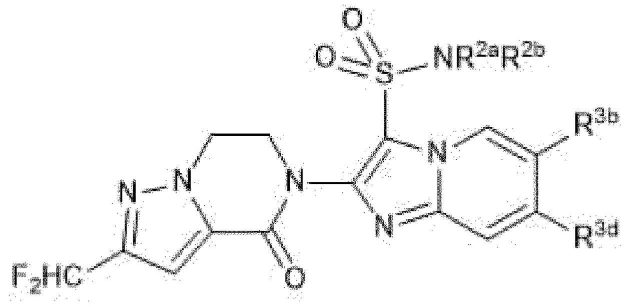

(L-47)

(hereinafter, referred to as compound (L-47)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX705).

A compound (L-47) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX706).

A compound (L-47) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX707).

A compound (L-47) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX708).

A compound (L-47) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX709).

A compound (L-47) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX710).

A compound (L-47) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX711).

A compound (L-47) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX712).

A compound represented by formula (L-48):

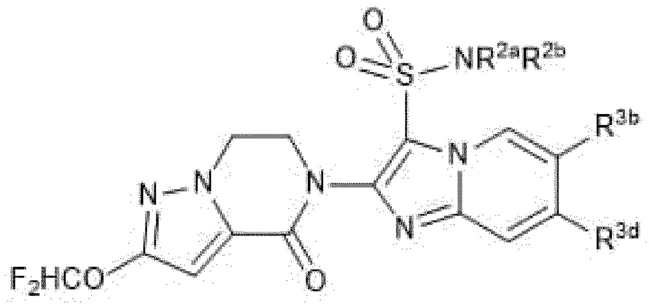

(L-48)

(hereinafter, referred to as compound (L-48)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX713).

A compound (L-48) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX714).

A compound (L-48) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX715).

A compound (L-48) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX716).

A compound (L-48) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX717).

A compound (L-48) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX718).

A compound (L-48) in which $R^{2a}$ and $R^{2b}$ are methyl groups, $R^{3b}$ is a hydrogen atom, and $R^{3d}$ is any of the

substituents shown in Table 7A to Table 15A (hereinafter, referred to as compound group SX719).

A compound (L-48) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX720).

A compound represented by formula (L-49):

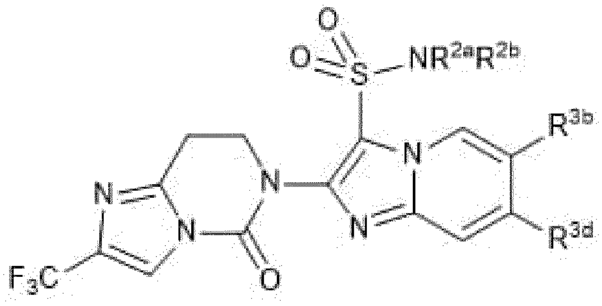

(L-49)

(hereinafter, referred to as compound (L-49)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX721).

A compound (L-49) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX722).

A compound (L-49) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX723).

A compound (L-49) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX724).

A compound (L-49) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX725).

A compound (L-49) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX726).

A compound (L-49) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX727).

A compound (L-49) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX728).

A compound represented by formula (L-50):

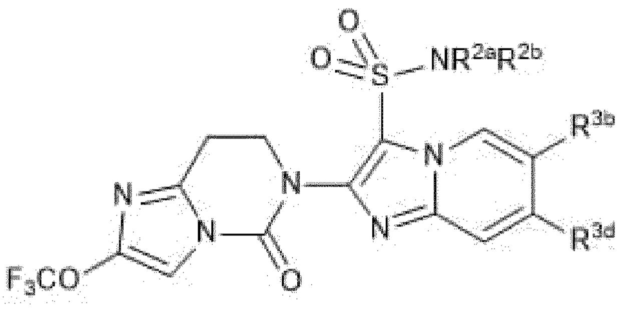

(L-50)

(hereinafter, referred to as compound (L-50)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX729).

A compound (L-50) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX730).

A compound (L-50) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX731).

A compound (L-50) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX732).

A compound (L-50) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX733).

A compound (L-50) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX734).

A compound (L-50) in which $R^{2a}$ and $R^{2b}$ are methyl groups, $R^{3b}$ is a hydrogen atom, and $R^{3d}$ is any of the

substituents shown in Table 7A to Table 15A (hereinafter, referred to as compound group SX735).

A compound (L-50) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX736).

A compound represented by formula (L-51):

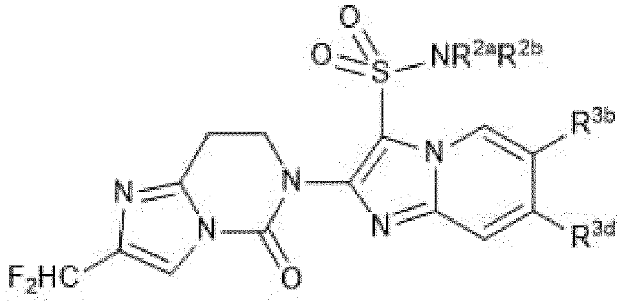

(L-51)

(hereinafter, referred to as compound (L-51)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX737).

A compound (L-51) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX738).

A compound (L-51) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX739).

A compound (L-51) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX740).

A compound (L-51) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX741).

A compound (L-51) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX742).

A compound (L-51) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX743).

A compound (L-51) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX744).

A compound represented by formula (L-52):

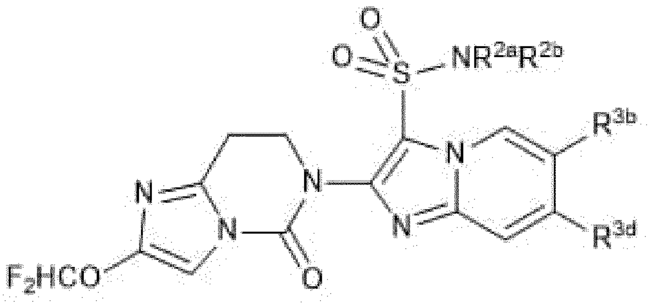

(L-52)

(hereinafter, referred to as compound (L-52)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX745).

A compound (L-52) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX746).

A compound (L-52) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX747).

A compound (L-52) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX748).

A compound (L-52) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX749).

A compound (L-52) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX750).

A compound (L-52) in which $R^{2a}$ and $R^{2b}$ are methyl groups, $R^{3b}$ is a hydrogen atom, and $R^{3d}$ is any of the

substituents shown in Table 7A to Table 15A (hereinafter, referred to as compound group SX751).

A compound (L-52) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX752).

A compound represented by formula (L-53):

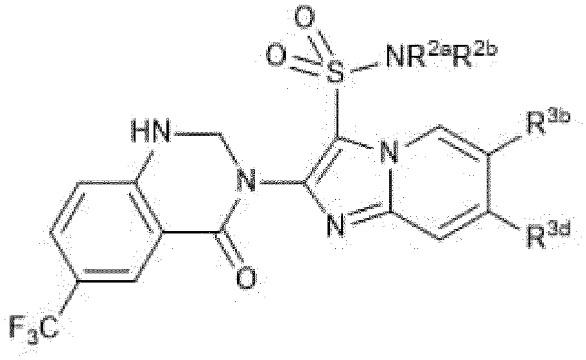

(L-53)

(hereinafter, referred to as compound (L-53)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX753).

A compound (L-53) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX754).

A compound (L-53) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX755).

A compound (L-53) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX756).

A compound (L-53) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX757).

A compound (L-53) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX758).

A compound (L-53) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX759).

A compound (L-53) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX760).

A compound represented by formula (L-54):

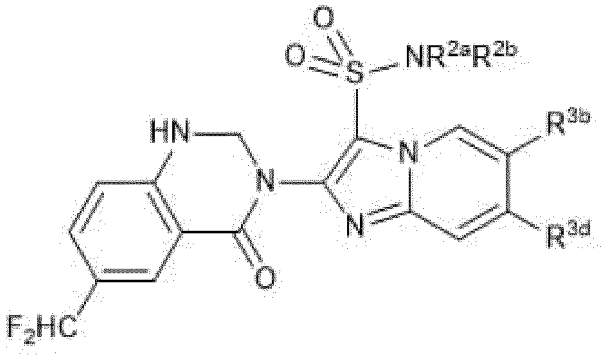

(L-54)

(hereinafter, referred to as compound (L-54)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX761).

A compound (L-54) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX762).

A compound (L-54) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX763).

A compound (L-54) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX764).

A compound (L-54) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX765).

A compound (L-54) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX766).
A compound (L-54) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX767).
A compound (L-54) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX768).
A compound represented by formula (L-55):

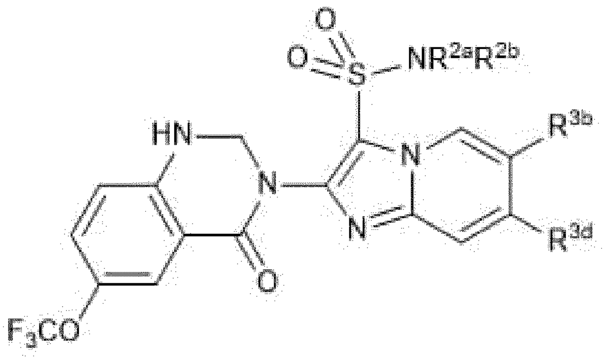

(L-55)

(hereinafter, referred to as compound (L-55)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX769).
A compound (L-55) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX770).
A compound (L-55) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX771).
A compound (L-55) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX772).
A compound (L-55) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX773).
A compound (L-55) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX774).
A compound (L-55) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX775).
A compound (L-55) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX776).
A compound represented by formula (L-56):

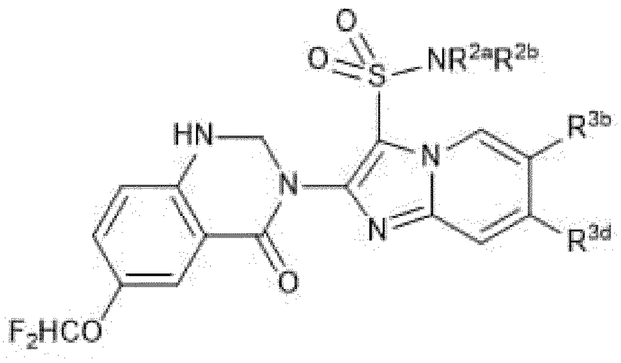

(L-56)

(hereinafter, referred to as compound (L-56)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX777).
A compound (L-56) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX778).
A compound (L-56) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX779).
A compound (L-56) in which $R^{2a}$ is a methyl group, $R^{2b}$ is a hydrogen atom, $R^{3d}$ is a hydrogen atom, and $R^{3b}$ is any

of the substituents shown in Table 7A to Table 15A (hereinafter, referred to as compound group SX780).

A compound (L-56) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX781).

A compound (L-56) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX782).

A compound (L-56) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX783).

A compound (L-56) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX784).

A compound represented by formula (L-57):

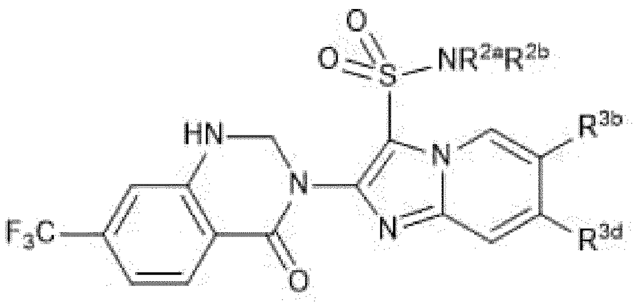

(L-57)

(hereinafter, referred to as compound (L-57)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX785) .

A compound (L-57) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX786).

A compound (L-57) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX787).

A compound (L-57) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX788).

A compound (L-57) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX789).

A compound (L-57) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX790).

A compound (L-57) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX791).

A compound (L-57) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX792).

A compound represented by formula (L-58):

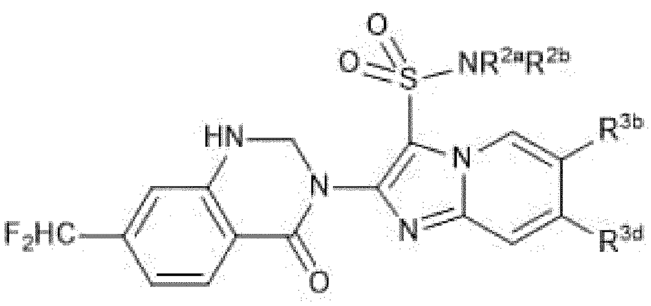

(L-58)

(hereinafter, referred to as compound (L-58)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX793).

A compound (L-58) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX794).

A compound (L-58) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX795).

A compound (L-58) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX796).

A compound (L-58) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX797).
A compound (L-58) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX798).
A compound (L-58) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX799).
A compound (L-58) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX800).
A compound represented by formula (L-59):

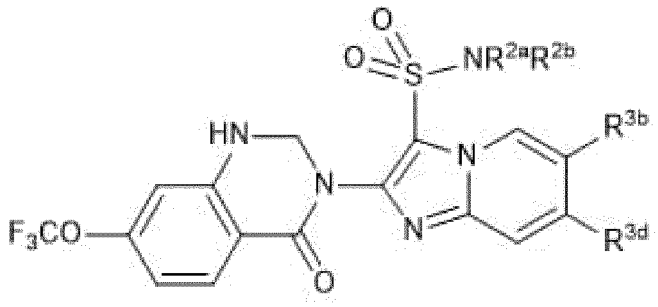

(L-59)

(hereinafter, referred to as compound (L-59)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX801).
A compound (L-59) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX802).
A compound (L-59) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX803).
A compound (L-59) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX804).
A compound (L-59) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX805).
A compound (L-59) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX806).
A compound (L-59) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX807).
A compound (L-59) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX808).
A compound represented by formula (L-60):

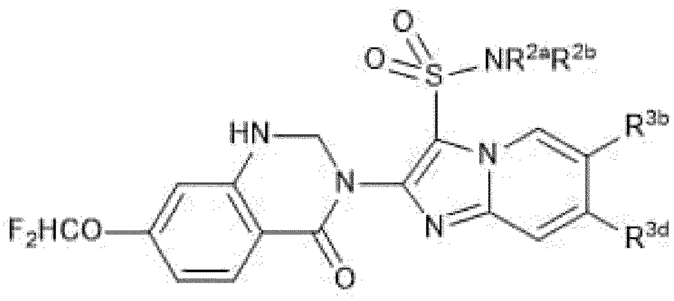

(L-60)

(hereinafter, referred to as compound (L-60)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX809).
A compound (L-60) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX810).
A compound (L-60) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX811).
A compound (L-60) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX812).

A compound (L-60) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX813).

A compound (L-60) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX814).

A compound (L-60) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX815).

A compound (L-60) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX816).

A compound represented by formula (L-61):

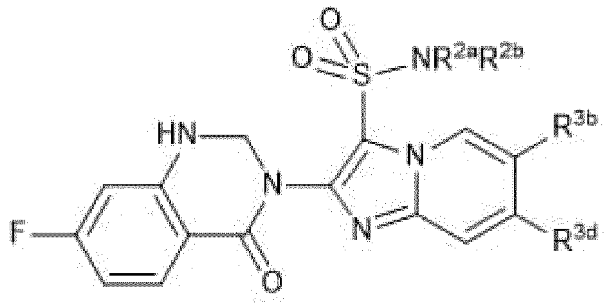

( L-61 )

(hereinafter, referred to as compound (L-61)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX817).

A compound (L-61) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX818).

A compound (L-61) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX819).

A compound (L-61) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX820).

A compound (L-61) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX821).

A compound (L-61) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX822).

A compound (L-61) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX823).

A compound (L-61) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX824).

A compound represented by formula (L-62):

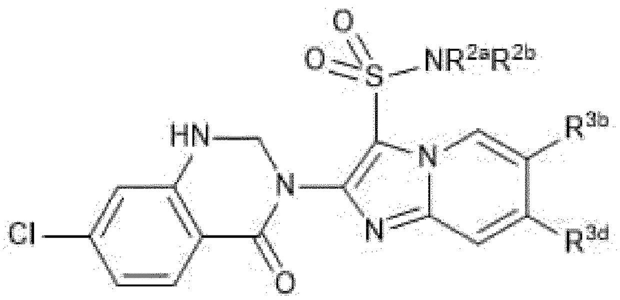

( L-62 )

(hereinafter, referred to as compound (L-62)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX825).

A compound (L-62) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX826).

A compound (L-62) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX827).

A compound (L-62) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX828).

A compound (L-62) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX829).

A compound (L-62) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX830).

A compound (L-62) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX831).

A compound (L-62) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX832).

A compound represented by formula (L-63):

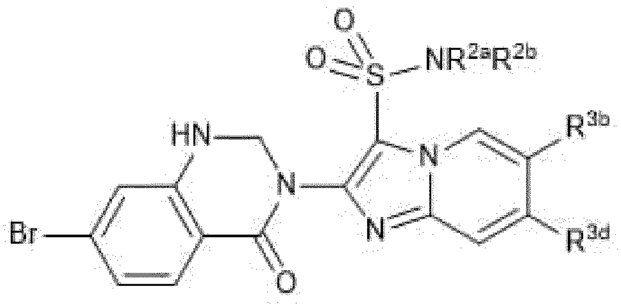

( L-63 )

(hereinafter, referred to as compound (L-63)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX833).

A compound (L-63) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX834).

A compound (L-63) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX835).

A compound (L-63) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX836).

A compound (L-63) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX837).

A compound (L-63) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX838).

A compound (L-63) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX839).

A compound (L-63) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX840).

A compound represented by formula (L-64):

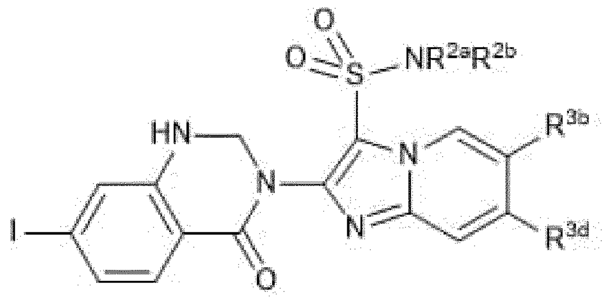

( L-64 )

(hereinafter, referred to as compound (L-64)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX841).

A compound (L-64) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX842).

A compound (L-64) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX843).

A compound (L-64) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX844).

A compound (L-64) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX845).

A compound (L-64) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX846).

A compound (L-64) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX847).

A compound (L-64) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX848).

A compound represented by formula (L-65):

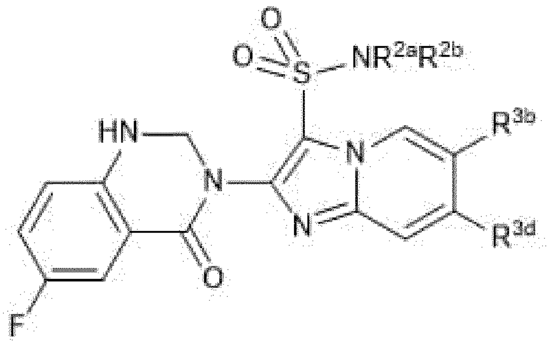

( L-65 )

(hereinafter, referred to as compound (L-65)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX849) .

A compound (L-65) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX850).

A compound (L-65) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX851).

A compound (L-65) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX852).

A compound (L-65) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX853).

A compound (L-65) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX854).

A compound (L-65) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX855).

A compound (L-65) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX856).

A compound represented by formula (L-66):

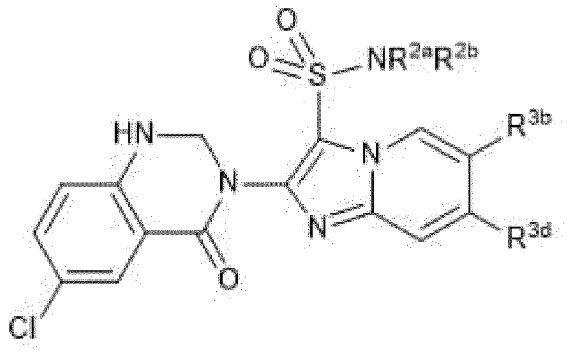

( L-66 )

(hereinafter, referred to as compound (L-66)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX857).

A compound (L-66) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX858).

A compound (L-66) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX859).

A compound (L-66) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX860).
A compound (L-66) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX861).
A compound (L-66) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX862).
A compound (L-66) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX863).
A compound (L-66) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX864).
A compound represented by formula (L-67):

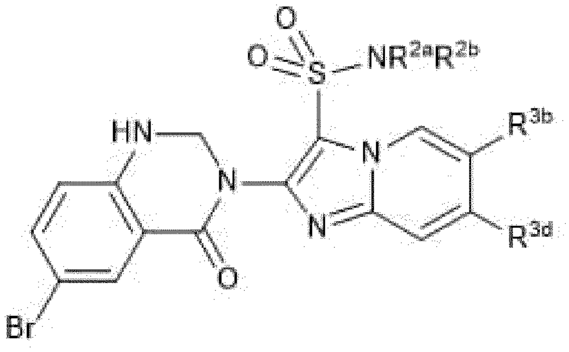

(L-67)

(hereinafter, referred to as compound (L-67)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX865).
A compound (L-67) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX866).
A compound (L-67) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX867).
A compound (L-67) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX868).
A compound (L-67) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX869).
A compound (L-67) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX870).
A compound (L-67) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX871).
A compound (L-67) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX872).
A compound represented by formula (L-68):

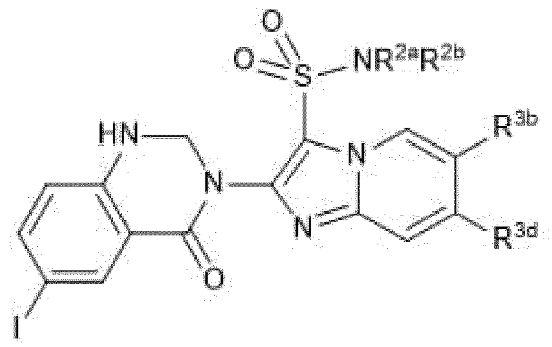

(L-68)

(hereinafter, referred to as compound (L-68)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX873).
A compound (L-68) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX874).

A compound (L-68) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX875).

A compound (L-68) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX876).

A compound (L-68) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX877).

A compound (L-68) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX878).

A compound (L-68) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX879).

A compound (L-68) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX880).

A compound represented by formula (L-69):

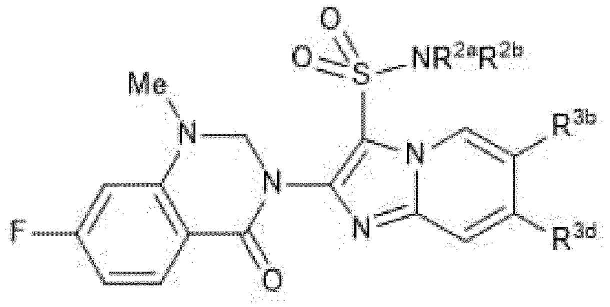

( L-69 )

(hereinafter, referred to as compound (L-69)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX881).

A compound (L-69) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX882).

A compound (L-69) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX883).

A compound (L-69) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX884).

A compound (L-69) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX885).

A compound (L-69) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX886).

A compound (L-69) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX887).

A compound (L-69) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX888).

A compound represented by formula (L-70):

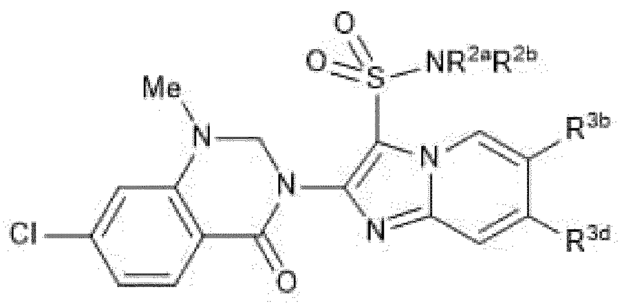

( L-70 )

(hereinafter, referred to as compound (L-70)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX889).

A compound (L-70) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX890).

A compound (L-70) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX891).

A compound (L-70) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX892).

A compound (L-70) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX893).

A compound (L-70) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX894).

A compound (L-70) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX895).

A compound (L-70) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX896).

A compound represented by formula (L-71):

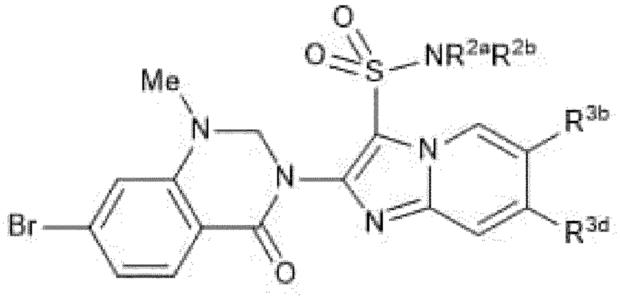

(L-71)

(hereinafter, referred to as compound (L-71)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX897).

A compound (L-71) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX898).

A compound (L-71) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX899).

A compound (L-71) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX900).

A compound (L-71) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX901).

A compound (L-71) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX902).

A compound (L-71) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX903).

A compound (L-71) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX904).

A compound represented by formula (L-72):

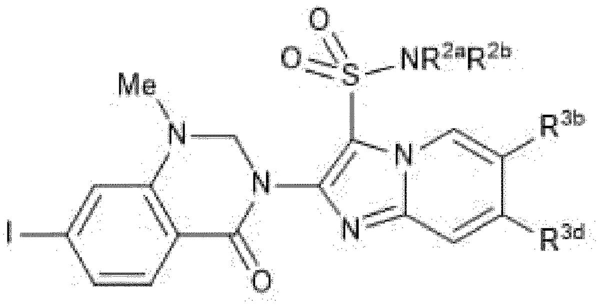

(L-72)

(hereinafter, referred to as compound (L-72)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX905).

A compound (L-72) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX906).

A compound (L-72) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX907).
A compound (L-72) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX908).
A compound (L-72) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX909).
A compound (L-72) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX910).
A compound (L-72) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX911).
A compound (L-72) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX912).
A compound represented by formula (L-73):

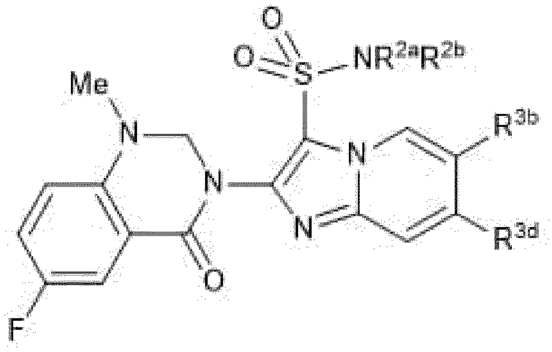

(L-73)

(hereinafter, referred to as compound (L-73)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX913).
A compound (L-73) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX914).
A compound (L-73) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX915).
A compound (L-73) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX916).
A compound (L-73) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX917).
A compound (L-73) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX918).
A compound (L-73) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX919).
A compound (L-73) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX920).
A compound represented by formula (L-74):

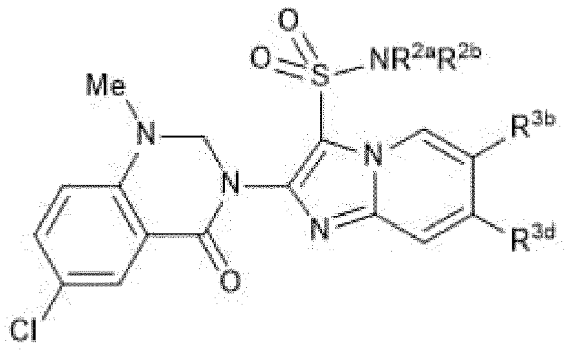

(L-74)

(hereinafter, referred to as compound (L-74)) in which $R^{2a}$ and $R^{2b}$ are hydrogen atoms, $R^{3b}$ is a hydrogen atom, and $R^{3d}$ is

any of the substituents shown in Table 7A to Table 15A (hereinafter, referred to as compound group SX921).

A compound (L-74) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX922).

A compound (L-74) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX923).

A compound (L-74) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX924).

A compound (L-74) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX925).

A compound (L-74) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX926).

A compound (L-74) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX927).

A compound (L-74) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX928).

A compound represented by formula (L-75):

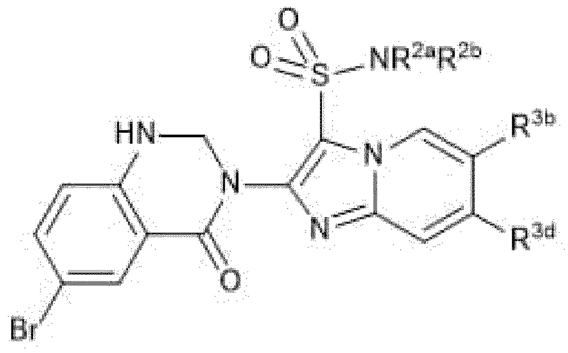

(L-75)

(hereinafter, referred to as compound (L-75)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX929).

A compound (L-75) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX930).

A compound (L-75) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX931).

A compound (L-75) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX932).

A compound (L-75) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX933).

A compound (L-75) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX934).

A compound (L-75) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX935).

A compound (L-75) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX936).

A compound represented by formula (L-76):

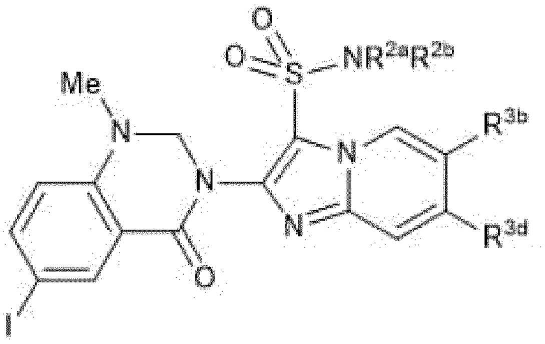

( L-76 )

(hereinafter, referred to as compound (L-76)) in which $R^{2a}$ and $R^{2b}$ are hydrogen atoms, $R^{3b}$ is a hydrogen atom, and $R^{3d}$ is any of the substituents shown in Table 7A to Table 15A (hereinafter, referred to as compound group SX937).

A compound (L-76) in which $R^{2a}$ and $R^{2b}$ are hydrogen atoms, $R^{3d}$ is a hydrogen atom, and $R^{3b}$ is any of the substituents shown in Table 7A to Table 15A (hereinafter, referred to as compound group SX938).

A compound (L-76) in which $R^{2a}$ is a methyl group, $R^{2b}$ is a hydrogen atom, $R^{3b}$ is a hydrogen atom, and $R^{3d}$ is any of the substituents shown in Table 7A to Table 15A (hereinafter, referred to as compound group SX939).

A compound (L-76) in which $R^{2a}$ is a methyl group, $R^{2b}$ is a hydrogen atom, $R^{3d}$ is a hydrogen atom, and $R^{3b}$ is any of the substituents shown in Table 7A to Table 15A (hereinafter, referred to as compound group SX940).

A compound (L-76) in which $R^{2a}$ is an ethyl group, $R^{2b}$ is a hydrogen atom, $R^{3b}$ is a hydrogen atom, and $R^{3d}$ is any of the substituents shown in Table 7A to Table 15A (hereinafter, referred to as compound group SX941).

A compound (L-76) in which $R^{2a}$ is an ethyl group, $R^{2b}$ is a hydrogen atom, $R^{3d}$ is a hydrogen atom, and $R^{3b}$ is any of the substituents shown in Table 7A to Table 15A (hereinafter, referred to as compound group SX942).

A compound (L-76) in which $R^{2a}$ and $R^{2b}$ are methyl groups, $R^{3b}$ is a hydrogen atom, and $R^{3d}$ is any of the substituents shown in Table 7A to Table 15A (hereinafter, referred to as compound group SX943).

A compound (L-76) in which $R^{2a}$ and $R^{2b}$ are methyl groups, $R^{3d}$ is a hydrogen atom, and $R^{3b}$ is any of the substituents shown in Table 7A to Table 15A (hereinafter, referred to as compound group SX944).

Next, formulation examples of the Present compound will be described. Note that "part (s)" represents part (s) by weight. In addition, the Present compound S represents compounds described in the compound groups SX1 to SX944.

Formulation Example 1

A formulation is obtained by mixing 35 parts of a mixture of polyoxyethylene alkyl ether sulfate ammonium salt and silica (weight ratio 1 : 1), 10 parts of any one of the Present compounds S, and 55 parts of water, and then finely pulverizing the mixture through a wet grinding method.

Formulation Example 2

A formulation is obtained by pulverizing and mixing 50 parts of any one of the Present compounds S, 3 parts of calcium lignosulfonate, 2 parts of sodium lauryl sulfate, and 45 parts of silica.

Formulation Example 3

A formulation is obtained by mixing 5 parts of any one of the Present compounds S, 9 parts of polyoxyethylene styrylphenyl ether, 5 parts of polyoxyethylene decyl ether (addition number of ethylene oxide: 5), 6 parts of calcium dodecylbenzene sulfonate, and 75 parts of xylene.

Formulation Example 4

A formulation is obtained by pulverizing and mixing 2 parts of any one of the Present compounds S, 1 part of silica, 2 parts of calcium lignosulfonate, 30 parts of bentonite, and 65 parts of kaolin clay, adding an appropriate amount of water and kneading the mixture, granulating the mixture with a granulator, and then drying the granules.

Formulation Example 5

A formulation is obtained by mixing 10 parts of any one of the Present compounds S with a mixture of 18 parts of benzyl alcohol and 9 parts of DMSO, adding thereto 6.3 parts of GERONOL (registered trademark) TE250, 2.7 parts of

Ethylan (registered trademark) NS-500LQ, and 54 parts of solvent naphtha, and mixing the same.

Formulation Example 6

A formulation is obtained by mixing and dissolving 0.1 parts of any one of the Present compounds S and 39.9 parts of kerosene, placing the mixture in an aerosol container, and filling the container with 60 parts of liquefied petroleum gas (mixture of propane, butane, and isobutane; saturated vapor pressure: 0.47 MPa (25°C)).

Formulation Example 7

A formulation is obtained by mixing 0.2 parts of any one of the Present compounds S, 50 parts of pyrethrum extract cake powder, 30 parts of tab powder, and 19.8 parts of wood powder, adding an appropriate amount of water and kneading the mixture, subjecting the mixture to an extruder to form a plate-like sheet, and forming the sheet into a spiral shape with a punching machine.

Next, the efficacy of the Present compound against harmful arthropods is shown by test examples. In the following test examples, the test was performed at 25°C.

Test Method 1

The test compound is formed into a formulation according to the method described in Formulation Example 1, and water containing 0.03 vol% of Shindain (registered trademark) is added thereto to prepare a diluted solution containing the test compound at a predetermined concentration.

A cucumber (Cucumis sativus) seedling (second true leaf stage) planted in a container is inoculated with about 30 cotton aphids (Aphis gossypii) (all developmental stages of life). One day later, the diluted solution is sprayed onto the seedling at a rate of 10 mL/seedling. After 5 days, the number of surviving insects is examined, and the control value is determined by the following formula.

$$\text{Control value (\%)} = \{1 - (Cb \times Tai)/(Cai \times Tb)\} \times 100$$

Note that characters in the formula represent the following meanings.

Cb: Number of tested insects in non-treated section
Cai: Number of surviving insects in non-treated section at the time of investigation
Tb: Number of tested insects in treated section
Tai: Number of surviving insects in treated section at the time of investigation

Here, the non-treated section means a section in which the same operation as in the treated section is performed except that the test compound is not used.

Test Example 1-1

At a predetermined concentration of 500 ppm, the Present compound is used as a test compound to perform a test in accordance with Test Method 1. Thereby, the control effect on cotton aphid (Aphis gossypii) can be checked.

Test Method 2

The test compound is formed into a formulation according to the method described in Formulation Example 5, and water is added thereto to prepare a diluted solution containing the test compound at a predetermined concentration.

A cucumber (Cucumis sativus) seedling (second true leaf stage) planted in a container is irrigated with the diluted solution at a rate of 5 mL/seedling. After 7 days, the leaf surface of this seedling is inoculated with about 30 cotton aphid (Aphis gossypii) (all developmental stages of life). After 6 days, the number of surviving insects is examined, and the control value is determined by the following formula.

$$\text{Control value (\%)} = \{1 - (Cb \times Tai)/(Cai \times Tb)\} \times 100$$

Note that characters in the formula represent the following meanings.

Cb: Number of tested insects in non-treated section
Cai: Number of surviving insects in non-treated section at the time of investigation
Tb: Number of tested insects in treated section
Tai: Number of surviving insects in treated section at the time of investigation

Here, the non-treated section means a section in which the same operation as in the treated section is performed except that the test compound is not used.

Test Example 2-1

At a predetermined concentration of 1000 ppm, the Present compound is used as a test compound to perform a test in accordance with Test Method 2. Thereby, the control effect on cotton aphid (Aphis gossypii) can be checked.

Test Method 3

The test compound is formed into a formulation according to the method described in Formulation Example 1, and water containing 0.03 vol% of Shindain (registered trademark) is added thereto to prepare a diluted solution containing the test compound at a predetermined concentration.
A cabbage (Brassicae oleracea) seedling (second to third true leaf stage) planted in a container is sprayed with the diluted solution at a rate of 20 mL/seedling. Thereafter, the leaves and stems of the seedling are cut out and placed in a container in which filter paper is laid. Thereafter, five second instar larvae of common cutworm (Spodoptera litura) are released. After 5 days, the number of surviving insects is counted, and the mortality is determined from the following formula.

Mortality (%) = (1 - number of surviving insects/5) $\times$ 100

Test Example 3-1

At a predetermined concentration of 500 ppm, the Present compound is used as a test compound to perform a test in accordance with Test Method 3. Thereby, the control effect on common cutworm (Spodoptera litura) can be checked.

Test Method 4

The test compound is formed into a formulation according to the method described in Formulation Example 1, and water containing 0.03 vol% of Shindain (registered trademark) is added thereto to prepare a diluted solution containing the test compound at a predetermined concentration.
A cabbage (Brassicae oleracea) seedling (second to third true leaf stage) planted in a container is sprayed with the diluted solution at a rate of 20 mL/seedling. Thereafter, the leaves and stems of the seedling are cut out and placed in a container in which filter paper is laid. Thereafter, five second instar larvae of diamondback moth (Plutella xylostella) are released. After 5 days, the number of surviving insects is counted, and the mortality is determined from the following formula.

Mortality (%) = (1 - number of surviving insects/5) $\times$ 100

Test Example 4-1

At a predetermined concentration of 500 ppm, the Present compound below was used as a test compound to perform a test in accordance with Test Method 4. As a result, the Present compound below showed the mortality of 100%.

Present compound: 4

Test Method 5

In 50 $\mu$L of a mixed solution of polyoxyethylene sorbitan monococoate : acetone = 5 : 95 (volume ratio), 1 mg of the test compound is dissolved. Water containing 0.03 vol% of Shindain (registered trademark) is added thereto to prepare a diluted solution containing the test compound at a predetermined concentration.
A young seedling of maize (Zea mays) is immersed in the diluted solution for 30 seconds. Thereafter, the two

seedlings are placed in a petri dish (90 mm diameter), and 10 second instar larvae of Western corn rootworm (Diabrotica virgifera virgifera) are released. After 5 days, the number of dead insects is counted, and the mortality is determined from the following formula.

$$\text{Mortality (\%)} = (\text{Number of dead insects}/10) \times 100$$

Test Example 5-1

At a predetermined concentration of 200 ppm, the Present compound is used as a test compound to perform a test in accordance with Test Method 5. Thereby, the control effect on Western corn rootworm (Diabrotica virgifera virgifera) can be checked.

Test Method 6

An acetone solution prepared so that the amount of the test compound is 800 ppm is poured into a container having an internal volume of 50 mL, and the inner surface of the container is uniformly coated so that the amount of the test compound is 40 mg/$m^2$, and then dried.

Five male imagoes of German cockroach (Blattella germanica) are placed in the container, and the lid is closed. After a lapse of a predetermined time, the state of German cockroach (Blattella germanica) is examined, and the mortality is determined. The mortality is calculated by the following formula.

Mortality (%) = (number of dead insects/number of tested insects) × 100

Test Example 6-1

At a predetermined time of 3 days, the Present compound is used as a test compound to perform a test in accordance with Test Method 6. Thereby, the control effect on German cockroach (Blattella germanica) can be checked.

Test Method 7

An acetone solution prepared so that the amount of the test compound is 2000 ppm is poured into a container having an internal volume of 20 mL, and the inner surface of the container is uniformly coated so that the amount of the test compound is 100 mg/$m^2$, and then dried.

Five nymphs of Asian longhorned tick (Haemaphysalis longicornis) are placed in the container, and the lid is closed. After a lapse of a predetermined time, the state of Asian longhorned tick (Haemaphysalis longicornis) is examined, and the mortality is determined. The mortality is calculated by the following formula.

Mortality (%) = (number of dead insects/number of tested insects) × 100

Test Example 7-1

At a predetermined time of 2 days, the Present compound is used as a test compound to perform a test in accordance with Test Method 7. Thereby, the control effect on Asian longhorned tick (Haemaphysalis longicornis) can be checked.

Test Method 8

An acetone solution prepared so that the amount of the test compound is 800 ppm is poured into a container having an internal volume of 20 mL, and the inner surface of the container is uniformly coated so that the amount of the test compound is 40 mg/$m^2$, and then dried.

Five adult female housefly (Musca domestica) are placed in the container, and the lid is closed. After a lapse of a predetermined time, the state of housefly (Musca domestica) is examined, and the mortality is determined. The mortality is calculated by the following formula.

Mortality (%) = (number of dead insects/number of tested insects) × 100

Test Example 8-1

At a predetermined time of 1 day, the Present compound is used as a test compound to perform a test in accordance with Test Method 8. Thereby, the control effect on housefly (Musca domestica) can be checked.

Test Method 9

The test compound is formed into a formulation according to the method described in Formulation Example 5, and water containing 0.03 vol% of Shindain (registered trademark) is added thereto to prepare a diluted solution containing the test compound at a predetermined concentration.

A cabbage (Brassicae oleracea) seedling (third to fourth true leaf stage) planted in a container is sprayed with the diluted solution at a rate of 20 mL/seedling. Thereafter, ten third instar larvae of common cutworm (Spodoptera litura) are released. After 6 days, the number of surviving insects is counted, and the mortality is determined from the following formula.

Mortality (%) = (1 - number of surviving insects/10) × 100

Test Example 9-1

At a predetermined concentration of 200 ppm, the Present compound is used as a test compound to perform a test in accordance with Test Method 9. Thereby, the control effect on common cutworm (Spodoptera litura) can be checked.

Test Method 10

The test compound is formed into a formulation according to the method described in Formulation Example 5, and water containing 0.03 vol% of Shindain (registered trademark) is added thereto to prepare a diluted solution containing the test compound at a predetermined concentration.

A cabbage (Brassicae oleracea) seedling (third to fourth true leaf stage) planted in a container is sprayed with the diluted solution at a rate of 20 mL/seedling. Thereafter, ten third instar larvae of diamondback moth (Plutella xylostella) are released. After 5 days, the number of surviving insects is counted, and the mortality is determined from the following formula.

Mortality (%) = (1 - number of surviving insects/10) × 100

Test Example 10-1

At a predetermined concentration of 200 ppm, the Present compound is used as a test compound to perform a test in accordance with Test Method 10. Thereby, the control effect on diamondback moth (Plutella xylostella) can be checked.

Test Method 11

The test compound is formed into a formulation according to the method described in Formulation Example 5, and water containing 0.03 vol% of Shindain (registered trademark) is added thereto to prepare a diluted solution containing the test compound at a predetermined concentration.

A cucumber (Cucumis sativus) seedling (second true leaf stage) planted in a container is inoculated with about 30 cotton aphid (Aphis gossypii) (all developmental stages of life). One day later, the diluted solution is sprayed onto the seedling at a rate of 10 mL/seedling. After 5 days, the number of surviving insects is examined, and the control value is determined by the following formula.

$$\text{Control value (\%)} = \{1 - (Cb \times Tai)/(Cai \times Tb)\} \times 100$$

Note that characters in the formula represent the following meanings.

Cb: Number of tested insects in non-treated section
Cai: Number of surviving insects in non-treated section at the time of investigation
Tb: Number of tested insects in treated section

Tai: Number of surviving insects in treated section at the time of investigation

Here, the non-treated section means a section in which the same operation as in the treated section is performed except that the test compound is not used.

Test Example 11-1

At a predetermined concentration of 200 ppm, the Present compound is used as a test compound to perform a test in accordance with Test Method 11. Thereby, the control effect on cotton aphid (Aphis gossypii) can be checked.

Test Method 12

The test compound is formed into a formulation according to the method described in Formulation Example 1, and water is added thereto to prepare a diluted solution containing the test compound at a predetermined concentration.
Into the diluted solution, 30 last instar larvae of house mosquito (Culex pipiens) are released, and the state of the larvae of house mosquito (Culex pipiens) is examined one day later to determine the mortality. The mortality is calculated by the following formula.

Mortality (%) = (number of dead insects/number of tested insects) × 100

Test Example 12-1

The Present compound is used as a test compound to perform a test in accordance with Test Method 12. Thereby, the control effect on house mosquito (Culex pipiens) can be checked.

Test Method 13

One milligram of the Present compound is dissolved in 10 μL of a mixed solution of xylene : DMF : surfactant = 4 : 4 : 1 (volume ratio), and diluted with water containing 0.02 vol% of a spreader to prepare a diluted solution A containing the Present compound at a predetermined concentration.
One milligram of the component is dissolved in 10 μL of a mixed solution of xylene : DMF : surfactant = 4 : 4 : 1 (volume ratio), and diluted with water containing 0.02 vol% of a spreader to prepare a diluted solution B containing the component at a predetermined concentration.
The diluted solution A and the diluted solution B are mixed to obtain a diluted solution C.
Leaf pieces of cucumber cotyledons (length 1.5 cm) are accommodated in each well of a 24 well microplate, 2 wingless imagos and 8 larvae of cotton aphid (Aphis gossypii) are released per well, and 20 μL of the diluted solution C is sprayed per well. This is defined as a treated section.
A well to which 20 μL of water containing 0.02 vol% of a spreader is sprayed instead of the diluted solution C is defined as a non-treated section.
After the diluted solution C is dried, the upper portion of the microplate is covered with a film sheet. After 5 days, the number of surviving insects in each well is examined.
The control value is calculated from the following formula.

$$\text{Control value (\%)} = \{1 - (\text{Tai})/(\text{Cai})\} \times 100$$

Note that symbols in the formula represent the following meanings.

Cai: Number of surviving insects in non-treated section at the time of investigation
Tai: Number of surviving insects in treated section at the time of investigation

Specific examples of the diluted solution C whose effect can be confirmed by Test Method 13 are shown in the following 1) to 5).
1) A diluted solution C having a concentration of the Present compound of 200 ppm and a concentration of the component of 2000 ppm among the combinations shown in the list A. In the list A, Comp X means any one compound selected from the compound groups SX1 to SX944.
List A:

Comp X + clothianidin; Comp X + thiamethoxam; Comp X + imidacloprid; Comp X + thiacloprid; Comp X + flupyradifurone; Comp X + sulfoxaflor; Comp X + triflumezopyrim; Comp X + dicloromezotiaz; Comp X + beta-cyfluthrin; Comp X + tefluthrin; Comp X + fipronil; Comp X + chlorantraniliprole; Comp X + cyantraniliprole; Comp X + tetraniliprole; Comp X + thiodicarb; Comp X + carbofuran; Comp X + fluxametamide; Comp X + afoxolaner; Comp X + fluralaner; Comp X + broflanilide; Comp X + abamectin; Comp X + fluopyram; Comp X + fluensulfone; Comp X + fluazaindolizine; Comp X + tioxazafen; Comp X + flupyrimin; Comp X + mycorrhizal fungi; Comp X + bradyrhizobium japonicum TA-11 strain; Comp X + bacillus firmus; Comp X + bacillus firmus I-1582 strain; Comp X + bacillus amyloliquefaciens; Comp X + bacillus amyloliquefaciens FZB42 strain; Comp X + pasteuria nishizawae; Comp X + pasteuria nishizawae Pn1 strain; Comp X + pasteuria penetrans; Comp X + tebuconazole; Comp X + prothioconazole; Comp X + metconazole; Comp X + ipconazole; Comp X + triticonazole; Comp X + difenoconazole; Comp X + imazaril; Comp X + triadimenol; Comp X + tetraconazole; Comp X + flutriafol; Comp X + mandestrobin; Comp X + azoxystrobin; Comp X + pyraclostrobin; Comp X + trifloxystrobin; Comp X + fluoxastrobin; Comp X + picoxystrobin; Comp X + fenamidone; Comp X + metalaxyl; Comp X + metalaxyl M; Comp X + fludioxonil; Comp X + sedaxane; Comp X + penflufen; Comp X + fluxapyroxad; Comp X + benzovindiflupyr; Comp X + boscalid; Comp X + carboxin; Comp X + penthiopyrad; Comp X + flutolanil; Comp X + captan; Comp X + thiuram; Comp X + tolclofos-methyl; Comp X + thiabendazole; Comp X + ethaboxam; Comp X + mancozeb; Comp X + picarbutrazox; Comp X + oxathiapiprolin; Comp X + silthiofam; Comp X + inpyrfluxam.

2) A diluted solution C having a concentration of the Present compound of 200 ppm and a concentration of the component of 200 ppm among the combinations shown in the list A.

3) A diluted solution C having a concentration of the Present compound of 500 ppm and a concentration of the component of 50 ppm among the combinations shown in the list A.

4) A diluted solution C having a concentration of the Present compound of 500 ppm and a concentration of the component of 5 ppm among the combinations shown in the list A.

5) A diluted solution C having a concentration of the Present compound of 500 ppm and a concentration of the component of 0.5 ppm among the combinations shown in the list A.

INDUSTRIAL APPLICABILITY

The Present compound exhibits an excellent control effect on harmful arthropods.

## Claims

**1.** A compound represented by formula (I) or an N oxide thereof:

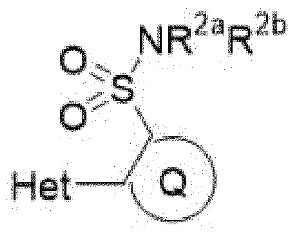

(I)

wherein, in the formula,
Q represented by the following formula

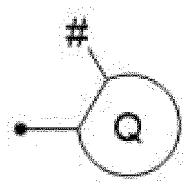

represents a group represented by formula Q1, a group represented by formula Q2, a group represented by formula Q3, a group represented by formula Q4, a group represented by formula Q5, a group represented by formula Q6, or a group represented by formula Q7, where # represents a binding site to the sulfur atom, and • represents a binding site to Het:

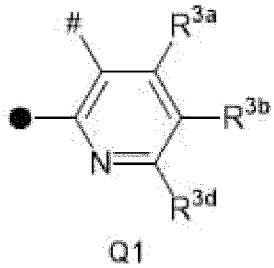

Q1

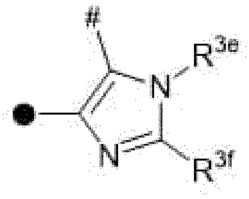

Q2

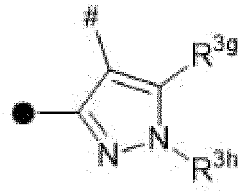

Q3

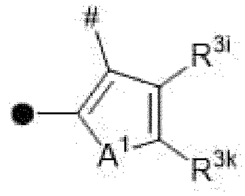

Q4

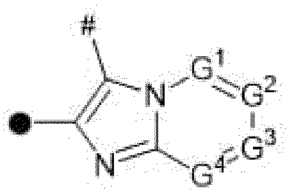

Q5

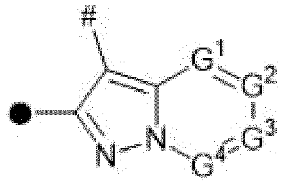

Q6

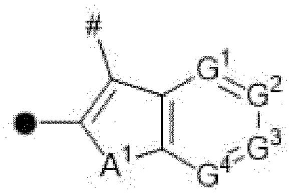

Q7

;

$A^1$ represents $NR^5$, an oxygen atom, or a sulfur atom;
the combination of $G^1$, $G^2$, $G^3$, and $G^4$ represents:
a combination in which $G^1$ is a nitrogen atom or $CR^{3a}$, $G^2$ is $CR^{3b}$, $G^3$ is $CR^{3d}$, and $G^4$ is $CR^{3c}$;
a combination in which $G^1$ is $CR^{3a}$, $G^2$ is a nitrogen atom, $G^3$ is $CR^{3d}$, and $G^4$ is $CR^{3c}$;
a combination in which $G^1$ is $CR^{3a}$, $G^2$ is $CR^{3b}$, $G^3$ is a nitrogen atom, and $G^4$ is $CR^{3c}$; or
a combination in which $G^1$ is $CR^{3a}$, $G^2$ is $CR^{3b}$, $G^3$ is $CR^{3d}$, and $G^4$ is a nitrogen atom;
$R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom; or
$R^{2a}$ and $R^{2b}$ may be combined with the nitrogen atom to which they are attached to form an aziridinyl group, an azetidinyl group, a pyrrolidinyl group, or a piperidyl group;
$R^{3a}$, $R^{3b}$, $R^{3c}$, $R^{3d}$, $R^{3f}$, $R^{3g}$, $R^{3i}$, and $R^{3k}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5- or 6-membered aromatic heterocyclic group optionally substituted with one or more substituents selected from Group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N{=}CHNR^{31}R^{32}$, $N{=}S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}{=}NOR^{17}$, $NR^{11}CR^{24}{=}NOR^{17}$, $S(O)_qR^{23}$, a cyano group, a nitro group, a hydrogen atom, or a halogen atom;
$R^{3e}$ and $R^{3h}$ are identical to or different from each other, and each represents a C1-C6 alkyl group optionally substituted with one or more halogen atoms, or a phenyl group optionally substituted with one or more substituents selected from Group H;
when Q is a group represented by formula Q1, two adjacent substituents among $R^{3a}$, $R^{3b}$, and $R^{3d}$ may be combined with the two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring {the benzene ring, the pyrrole ring, the furan ring, the thiophene ring, the pyrazole ring, the imidazole ring, the oxazole ring, the isoxazole ring, the thiazole ring, the isothiazole ring, the pyridine ring, the pyridazine ring, the pyrimidine ring, and the pyrazine ring may be substituted with one or more substituents selected from Group H}, or a triazole ring optionally substituted with one or more substituents selected from Group I;
p represents 0 or 1;
q represents 0, 1, or 2;
$R^{30}$ represents a C1-C6 chain hydrocarbon group optionally substituted with one or more halogen atoms, a halogen atom, $OR^{35}$, $NR^{36}R^{37}$, or a hydrogen atom;
$R^{17}$ represents a C1-C6 chain hydrocarbon group optionally substituted with one or more halogen atoms, a

phenyl group optionally substituted with one or more substituents selected from Group D, or a hydrogen atom; $R^{12}$ represents a C1-C6 chain hydrocarbon group optionally substituted with one or more substituents selected from Group F, a C3-C7 cycloalkyl group optionally substituted with one or more substituents selected from Group J, a C3-C7 cycloalkenyl group optionally substituted with one or more substituents selected from Group J, a phenyl group optionally substituted with one or more substituents selected from Group D, a 6-membered aromatic heterocyclic group optionally substituted with one or more substituents selected from Group D, a hydrogen atom, or $S(O)_2R^{23}$;

$R^{23}$ represents a C1-C6 chain hydrocarbon group optionally substituted with one or more halogen atoms, or a phenyl group optionally substituted with one or more substituents selected from Group D;

$R^{11a}$ and $R^{12a}$ are combined with the nitrogen atom to which they are attached to form a 3- to 7-membered non-aromatic heterocyclic group optionally substituted with one or more substituents selected from Group E;

$R^{13}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, a phenyl group optionally substituted with one or more substituents selected from Group D, a 5- or 6-membered aromatic heterocyclic group optionally substituted with one or more substituents selected from Group D, or a hydrogen atom;

$R^{14}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, or a phenyl C1-C3 alkyl group {a phenyl moiety in the phenyl C1-C3 alkyl group may be substituted with one or more substituents selected from Group D};

$R^{15}$ and $R^{16}$ are identical to or different from each other, and each represents a C1-C6 alkyl group optionally substituted with one or more halogen atoms;

$R^{31}$ represents a C1-C6 alkyl group optionally substituted with one or more halogen atoms, or a hydrogen atom;

$R^{32}$ represents a C1-C6 chain hydrocarbon group optionally substituted with one or more substituents selected from Group F, a C3-C7 cycloalkyl group optionally substituted with one or more substituents selected from Group J, or a hydrogen atom;

Het represents a group represented by formula Het 5, a group represented by formula Het 6, or a group represented by formula Het 7:

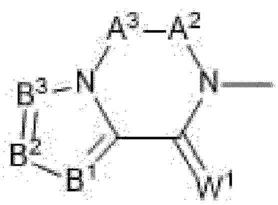
Het5

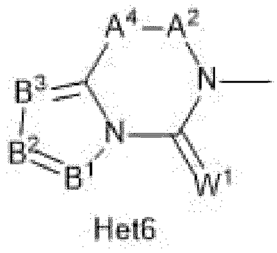
Het6

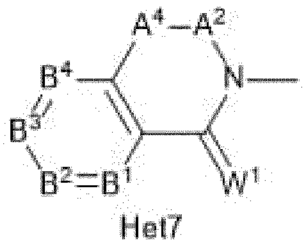
Het7

;

$A^2$ represents $NR^{5a}$ or $CR^{4a}R^{4d}$;

$A^3$ represents $CR^{4b}R^{4e}$;

$A^4$ represents $NR^{5b}$ or $CR^{4c}R^{4f}$;

$W^1$ represents an oxygen atom or a sulfur atom;

when Het is a group represented by formula Het 5,

the combination of $B^1$, $B^2$, and $B^3$ represents:

a combination in which $B^1$ is $CR^{6e}$, $B^2$ is a nitrogen atom or $CR^{6b}$, and $B^3$ is a nitrogen atom or $CR^{6c}$;

a combination in which $B^1$ is a nitrogen atom or $CR^{6a}$, $B^2$ is $CR^{6e}$, and $B^3$ is a nitrogen atom or $CR^{6c}$; or

a combination in which $B^1$ is a nitrogen atom or $CR^{6a}$, $B^2$ is a nitrogen atom or $CR^{6b}$, and $B^3$ is $CR^{6e}$;

when Het is a group represented by formula Het 6,

the combination of $A^2$ and $A^4$ represents:

a combination in which $A^2$ is $CR^{4a}R^{4d}$, and $A^4$ is $NR^{5b}$ or $CR^{4c}R^{4f}$; or

a combination in which $A^2$ is $NR^{5a}$, and $A^4$ is $CR^{4c}R^{4f}$;

the combination of $B^1$, $B^2$, and $B^3$ represents:

a combination in which $B^1$ is $CR^{6e}$, $B^2$ is a nitrogen atom or $CR^{6b}$, and $B^3$ is a nitrogen atom or $CR^{6c}$;

a combination in which $B^1$ is a nitrogen atom or $CR^{6a}$, $B^2$ is $CR^{6e}$, and $B^3$ is a nitrogen atom or $CR^{6c}$; or

a combination in which $B^1$ is a nitrogen atom or $CR^{6a}$, $B^2$ is a nitrogen atom or $CR^{6b}$, and $B^3$ is $CR^{6e}$;

when Het is a group represented by formula Het 7,
the combination of $A^2$ and $A^4$ represents:

a combination in which $A^2$ is $CR^{4a}R^{4d}$, and $A^4$ is $NR^{5b}$ or $CR^{4c}R^{4f}$; or
a combination in which $A^2$ is $NR^{5a}$, and $A^4$ is $CR^{4c}R^{4f}$;
the combination of $B^1$, $B^2$, $B^3$, and $B^4$ represents:

a combination in which $B^1$ is a nitrogen atom or $CR^{6a}$, $B^2$ is $CR^{6e}$, $B^3$ is a nitrogen atom or $CR^{6c}$, and $B^4$ is a nitrogen atom or $CR^{6d}$;
a combination in which $B^1$ is a nitrogen atom or $CR^{6a}$, $B^2$ is a nitrogen atom or $CR^{6b}$, $B^3$ is $CR^{6e}$, and $B^4$ is a nitrogen atom or $CR^{6d}$;
a combination in which $B^1$ is a nitrogen atom or $CR^{6a}$, $B^2$ is a nitrogen atom or $CR^{6b}$, $B^3$ is $CR^{6c}$, and $B^4$ is $CR^{6e}$;
a combination in which $B^1$ is a nitrogen atom or $CR^{6a}$, $B^2$ is $CR^{6b}$, $B^3$ is a nitrogen atom, and $B^4$ is $CR^{6e}$; or
a combination in which $B^1$ is $CR^{6a}$, $B^2$ is a nitrogen atom, $B^3$ is a nitrogen atom, and $B^4$ is $CR^{6e}$;
$R^{4a}$, $R^{4b}$, $R^{4c}$, $R^{6a}$, $R^{6b}$, $R^{6c}$, and $R^{6d}$ are identical to or different from each other, and each represents a C1-C6 chain hydrocarbon group optionally substituted with one or more halogen atoms, a nitro group, $OR^{18}$, $NR^{18}R^{19}$, a cyano group, an amino group, a halogen atom, or a hydrogen atom;
$R^{4d}$, $R^{4e}$, and $R^{4f}$ are identical to or different from each other, and each represents a C1-C6 chain hydrocarbon group optionally substituted with one or more halogen atoms, a cyano group, a halogen atom, or a hydrogen atom;
$R^{6e}$ represents a C1-C6 chain hydrocarbon group substituted with one or more substituents selected from a group consisting of a cyano group and a halogen atom, a C3-C4 cycloalkyl group optionally substituted with one or more substituents selected from a group consisting of a cyano group and a halogen atom, $S(O)_mR^7$, $OR^7$, a halogen atom, or $OS(O)_2R^7$;
$R^5$ represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom;
$R^{5a}$ and $R^{5b}$ are identical to or different from each other, and each represents a C1-C6 chain hydrocarbon group optionally substituted with one or more substituents selected from Group K, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom;
$R^7$ represents a C1-C6 chain hydrocarbon group substituted with one or more halogen atoms;
m represents 0, 1, or 2;
$R^{18}$ and $R^{35}$ are identical to or different from each other, and each represents a C1-C6 chain hydrocarbon group optionally substituted with one or more halogen atoms; and
$R^{11}$, $R^{19}$, $R^{24}$, $R^{36}$, and $R^{37}$ are identical to or different from each other, and each represents a C1-C6 chain hydrocarbon group optionally substituted with one or more halogen atoms, or a hydrogen atom;
Group B: a group consisting of a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, a C3-C6 cycloalkyl group optionally substituted with one or more halogen atoms, a cyano group, a hydroxy group, and a halogen atom;
Group C: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, and a halogen atom;
Group D: a group consisting of a C1-C6 chain hydrocarbon group optionally substituted with one

or more halogen atoms, a hydroxy group, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a sulfanyl group, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom,

wherein $R^{21}$ and $R^{22}$ are identical to or different from each other, and each represents a C1-C6 alkyl group optionally substituted with one or more halogen atoms;

Group E: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a halogen atom, an oxo group, a hydroxy group, a cyano group, and a nitro group;

Group F: a group consisting of a C1-C6 alkoxy group optionally substituted with one or more halogen atoms, a phenyl group optionally substituted with one or more substituents selected from Group D, a 5- or 6-membered aromatic heterocyclic group optionally substituted with one or more substituents selected from Group D, a C3-C7 cycloalkyl group optionally substituted with one or more halogen atoms, a 3- to 7-membered non-aromatic heterocyclic group optionally substituted with one or more substituents selected from Group C, an amino group, $NHR^{21}$, $NR^{21}R^{22}$, a halogen atom, and a cyano group;

Group H: a group consisting of a C1-C6 alkyl group optionally substituted with one or more halogen atoms, $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5- or 6-membered aromatic heterocyclic group, wherein

$R^9$ represents a C1-C6 alkyl group optionally substituted with one or more halogen atoms, or a C3-C6 cycloalkyl group optionally substituted with one or more halogen atoms, and

$R^{10}$ represents a C1-C6 alkyl group optionally substituted with one or more halogen atoms, a C3-C6 cycloalkyl group optionally substituted with one or more halogen atoms, or a hydrogen atom;

Group I: a group consisting of a C2-C6 chain hydrocarbon group optionally substituted with one or more halogen atoms, a C3-C6 cycloalkyl group optionally substituted with one or more halogen atoms, a phenyl group optionally substituted with one or more substituents selected from Group D, a 5- or 6-membered aromatic heterocyclic group optionally substituted with one or more substituents selected from Group D, a C2-C6 alkylcarbonyl group optionally substituted with one or more halogen atoms, a C2-C6 alkoxycarbonyl group optionally substituted with one or more halogen atoms, an aminocarbonyl group, a (C1-C6 alkyl) aminocarbonyl group optionally substituted with one or more halogen atoms, a methyl group, and a di (C1-C4 alkyl) aminocarbonyl group optionally substituted with one or more halogen atoms;

Group J: a group consisting of a C1-C6 alkyl group optionally substituted with one or more halogen atoms, a halogen atom, and a cyano group;

Group K: a group consisting of a C1-C6 alkoxy group optionally substituted with one or more halogen atoms, a phenyl group optionally substituted with one or more substituents selected from Group D, a 5- or 6-membered aromatic heterocyclic group optionally substituted with one or more substituents selected from Group D, a C3-C7 cycloalkyl group optionally substituted with one or more halogen atoms, and a 3- to 7-membered non-aromatic heterocyclic group optionally substituted with one or more substituents selected from the group C.

**2.** The compound or the N oxide thereof according to claim 1, wherein Q is a group represented by formula Q2, a group represented by formula Q3, or a group represented by formula Q4.

**3.** The compound or the N oxide thereof according to claim 1, wherein Q is a group represented by formula Q1 or a group represented by formula Q5.

**4.** The compound or the N oxide thereof according to claim 1, wherein Q is a group represented by formula Q5.

**5.** The compound or the N oxide of the compound according to claim 1, wherein

Q is a group represented by formula Q5;
$G^1$ is $CR^{3a}$;
$G^2$ is $CR^{3b}$;
$G^3$ is $CR^{3d}$; and
$G^4$ is $CR^{3c}$.

**6.** The compound or the N oxide thereof according to [1], wherein

Q is a group represented by formula Q5;
$G^1$ is $CR^{3a}$;
$G^2$ is $CR^{3b}$;
$G^3$ is $CR^{3d}$;
$G^4$ is $CR^{3c}$;
$R^{3a}$ is a hydrogen atom;
$R^{3b}$ is a C1-C6 chain hydrocarbon group optionally substituted with one or more substituents selected from Group B, a hydrogen atom, or a halogen atom;
$R^{3c}$ is a hydrogen atom; and
$R^{3d}$ is a C1-C6 chain hydrocarbon group optionally substituted with one or more substituents selected from Group B, a hydrogen atom, or a halogen atom.

**7.** The compound or the N oxide thereof according to any one of claims 1 to 6, wherein Het is a group represented by formula Het 7;

$A^2$ is $CR^{4a}R^{4d}$;
$A^4$ is $NR^{5b}$; and
$W^1$ is an oxygen atom.

**8.** A composition for controlling a harmful arthropod comprising the compound or an N-oxide thereof according to any one of claims 1 to 7; and an inert carrier.

**9.** A composition comprising one or more ingredients selected from the group consisting of Group (a), Group (b), Group (c), and Group (d), and the compound or an N-oxide thereof according to any one of claims 1 to 7:

Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

**10.** A method for controlling a harmful arthropod which comprises applying an effective amount of the compound or an N-oxide thereof according to any one of claims 1 to 7, or an effective amount of the composition according to claim 9, to a harmful arthropod or a habitat where a harmful arthropod lives.

**11.** A seed or a vegetative reproductive organ holding an effective amount of the compound or the N oxide thereof according to any one of claims 1 to 7, or an effective amount of the composition according to claim 9.

## INTERNATIONAL SEARCH REPORT

International application No. **PCT/JP2023/037149**

**A. CLASSIFICATION OF SUBJECT MATTER**

***C07D 401/04***(2006.01)i; ***A01M 1/20***(2006.01)i; ***A01N 43/42***(2006.01)i; ***A01N 43/54***(2006.01)i; ***A01N 43/653***(2006.01)i; ***A01N 43/90***(2006.01)i; ***A01N 47/02***(2006.01)i; ***A01N 53/12***(2006.01)i; ***A01N 53/14***(2006.01)i; ***A01P 7/02***(2006.01)i; ***A01P 7/04***(2006.01)i; ***A61K 31/4725***(2006.01)i; ***A61P 33/14***(2006.01)i
FI: C07D401/04 CSP; A01M1/20; A01N43/42 102; A01N43/54 G; A01N43/653 G; A01N43/90 103; A01N43/90 104; A01N47/02; A01N53/12; A01N53/14; A01P7/02; A01P7/04; A61K31/4725; A61P33/14

According to International Patent Classification (IPC) or to both national classification and IPC

**B. FIELDS SEARCHED**

Minimum documentation searched (classification system followed by classification symbols)

C07D401/04; A01M1/20; A01N43/42; A01N43/54; A01N43/653; A01N43/90; A01N47/02; A01N53/12; A01N53/14; A01P7/02; A01P7/04; A61K31/4725; A61P33/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2022/186133 A1 (NIHON NOHYAKU CO., LTD.) 09 September 2022 (2022-09-09) entire text | 1-11 |
| A | JP 2018-523664 A (BAYER CROPSCIENCE AG) 23 August 2018 (2018-08-23) entire text | 1-11 |
| A | JP 2018-505881 A (BAYER CROPSCIENCE AG) 01 March 2018 (2018-03-01) entire text | 1-11 |
| A | JP 2017-512191 A (BAYER CROPSCIENCE AG) 18 May 2017 (2017-05-18) entire text | 1-11 |
| A | JP 2018-536639 A (BAYER CROPSCIENCE AG) 13 December 2018 (2018-12-13) entire text | 1-11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2023** | **26 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/037149**

| Patent document cited in search report | Publication date (day/month/year) | Patent family member(s) | Publication date (day/month/year) |
|---|---|---|---|
| WO 2022/186133 A1 | 09 September 2022 | (Family: none) | |
| JP 2018-523664 A | 23 August 2018 | US 2018/0271099 A1<br>US 2020/0029567 A1<br>WO 2017/025419 A2<br>EP 3331870 A2<br>EP 3896065 A1<br>EP 3896066 A2<br>KR 10-2018-0032640 A<br>CN 108137548 A | |
| JP 2018-505881 A | 01 March 2018 | US 2018/0002345 A1<br>WO 2016/124563 A1<br>EP 3253210 A1<br>KR 10-2017-0124533 A<br>CN 107428764 A | |
| JP 2017-512191 A | 18 May 2017 | US 2017/0073342 A1<br>US 2019/0241564 A1<br>US 2023/0087882 A1<br>WO 2015/121136 A1<br>EP 3107912 A1<br>KR 10-2016-0122809 A<br>CN 106414441 A | |
| JP 2018-536639 A | 13 December 2018 | US 2018/0305353 A1<br>WO 2017/072039 A1<br>EP 3368521 A1<br>KR 10-2018-0069005 A<br>CN 108430986 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2022165233 A
- WO 2020158889 A
- WO 2020203763 A

### Non-patent literature cited in the description

- *Bioorganic & Medicinal Chemistry Letters*, 2016, vol. 26, 3822-3825
- *Journal of Heterocyclic Chemistry*, 2003, vol. 40, 677-679
- *Bioorg.Med.Chem.Lett.*, 2009, vol. 19, 1773-1778
- *Org.Process Res.Dev.*, 2021, vol. 25, 858-870
- Manual on development and use of FAO and WHO Specifications for pesticides, FAO Plant Production and Protection Papers. 2016, 271-276